# EUROPEAN PATENT APPLICATION

(11) **EP 2 354 222 A1**
(43) Date of publication of application: **10.08.2011**
(21) Application number: 10010133.6
(22) Date of filing: 25.03.2005
(51) Int. Cl.: C12N 9/42, C12N 15/62, C12P 21/02, C12P 21/06

(54) **Cellulase fusion protein and heterologous cellulase fusion construct encoding the same**

(30) Priority: 25.03.2004 US 556711 P
(62) Divisional of application: 05744703.9
(71) Applicant: Genencor International, Inc., Palo Alto, CA 94304-1013 (US)
(72) Inventor: Bower, Benjamin S., Newark CA 94560 (US); Larenas, Edmund A., Moss Beach 5 California 94038 (US); Mitchinson, Colin, Half Moon Bay 5 California 94019 (US)
(74) Representative: Forrest, Graham Robert

(57) **Abstract**

The present invention relates to a heterologous cellulase fusion construct, which encodes a fusion protein having cellulolytic activity comprising a first catalytic domain derived from a fungal exo-cellobiohydrolase and a second catalytic domain derived from a cellulase enzyme. The invention also relate to vectors and fungal host cells comprising the heterologous cellulase fusion construct as well as methods for producing said cellulase fusion protein and enzymatic cellulase compositions.

## Description

### SPONSORED RESEARCH AND DEVELOPMENT

Portions of this work were funded by Subcontract No. ZCO-0-30017-01 with the National Renewable Energy Laboratory under Prime Contract No. DE-AC36-99G010337 with the United States Department of Energy. Accordingly the United States Government may have certain rights in this invention.

### RELATED APPLICATIONS

The present application claims priority to U.S. Provisional Patent Application Serial No. 60/556,711, entitled "Cellulase Fusion Protein and Heterologous Fusion Construct Encoding the Same" and filed on March 25, 2004.

### FIELD OF THE INVENTION

The present invention relates to a heterologous cellulase fusion construct, which encodes a fusion protein having cellulolytic activity comprising a first catalytic domain derived from a fungal exo-cellobiohydrolase and a second catalytic domain derived from a cellulase enzyme. The invention also relates to vectors and fungal host cells comprising the heterologous cellulase fusion construct as well as methods for producing said cellulase fusion protein and enzymatic cellulase compositions.

### BACKGROUND OF THE INVENTION

Cellulose and hemicellulose are the most abundant plant materials produced by photosynthesis. They can be degraded and used as an energy source by numerous microorganisms, including bacteria, yeast and fungi, which produce extracellular enzymes capable of hydrolysis of the polymeric substrates to monomeric sugars (Aro et al., 2001). As the limits of non-renewable resources approach, the potential of cellulose to become a major renewable energy resource is enormous (Krishna *et al.,* 2001). The effective utilization of cellulose through biological processes is one approach to overcoming the shortage of foods, feeds, and fuels (Ohmiya *et al.,* 1997).

Cellulases are enzymes that hydrolyze cellulose (beta-1,4-glucan or beta D-glucosidic linkages) resulting in the formation of glucose, cellobiose, cellooligosaccharides, and the like. Cellulases have been traditionally divided into three major classes: endoglucanases (EC 3.2.1.4) ("EG"), exoglucanases or cellobiohydrolases (EC 3.2.1.91) ("CBH") and beta-glucosidases ([beta] -D-glucoside glucohydrolase; EC 3.2.1.21) ("BG") (Knowles *et al.,* 1987 and Schulein, 1988). Endoglucanases act mainly on the amorphous parts of the cellulose fiber, whereas cellobiohydrolases are also able to degrade crystalline cellulose.

Cellulases are known to be produced by a large number of bacteria, yeast and fungi. Certain fungi produce a complete cellulase system capable of degrading crystalline forms of cellulose, such that the cellulases are readily produced in large quantities via fermentation.

In order to efficiently convert crystalline cellulose to glucose the complete cellulase system comprising components from each of the CBH, EG and BG classifications is required, with isolated components less effective in hydrolyzing crystalline cellulose (Filho *et al.,* 1996). In particular, the combination of EG-type cellulases and CBH- type cellulases interact to more efficiently degrade cellulose than either enzyme used alone (Wood, 1985; Baker *et al.,* 1994; and Nieves *et al.,* 1995).

Additionally, cellulases are known in the art to be useful in the treatment of textiles for the purposes of enhancing the cleaning ability of detergent compositions, for use as a softening agent, for improving the feel and appearance of cotton fabrics, and the like (Kumar *et al.,* 1997). Cellulase-containing detergent compositions with improved cleaning performance (US Pat. No. 4,435,307; GB App. Nos. 2,095,275 and 2,094,826) and for use in the treatment of fabric to improve the feel and appearance of the textile (US Pat. Nos. 5,648,263, 5,691,178, and 5,776,757, and GB App. No. 1,358,599), have been described in the literature.

Hence, cellulases produced in fungi and bacteria have received significant attention. In particular, fermentation of *Trichoderma spp.* (*e.g., Trichoderma longibrachiatum* or *Trichoderma reesei*) has been shown to produce a complete cellulase system capable of degrading crystalline forms of cellulose. Over the years, *Trichoderma* cellulase production has been improved by classical mutagenesis, screening, selection and development of highly refined, large scale inexpensive fermentation conditions. While the multi-component cellulase system of *Trichoderma spp.* is able to hydrolyze cellulose to glucose, there are cellulases from other microorganisms, particularly bacterial strains, with different properties for efficient cellulose hydrolysis, and it would be advantageous to express these proteins in a filamentous fungus for industrial scale cellulase production. However, the results of many studies demonstrate that the yield of bacterial enzymes from filamentous fungi is low (Jeeves *et al.,* 1991).

In this invention, a heterologous cellulase fusion construct, which includes the coding region of a fungal exo-cellobiohydrolase (CBH) catalytic domain fused to a coding region of a cellulase catalytic domain, has been introduced and expressed in a filamentous fungi host cell to increase the yield and effectiveness of cellulase enzymes.

### SUMMARY OF THE INVENTION

In a first aspect, the invention includes a heterologous cellulase fusion construct comprising in operable linkage from the 5' end of said construct, (a) a DNA molecule encoding a signal sequence, (b) a DNA molecule encoding a first catalytic domain, wherein said first catalytic domain is derived from of a fungal exo-cellobiohydrolase, and (c) a DNA molecule encoding a second catalytic domain, wherein said second catalytic domain is the catalytic domain of a cellulase enzyme.

In a first embodiment of this aspect, the heterologous cellulase fusion construct further comprises a linker sequence located 3' of the first catalytic domain and 5' of the second catalytic domain. In a second embodiment, the heterologous cellulase fusion construct lacks the cellulose binding domain (CBD) of the exo-cellobiohydrolase. In a third embodiment, the heterologous cellulase fusion construct further comprises a kexin site located after the linker sequence and before the second catalytic domain. In a fourth embodiment, the heterologous fusion construct will comprise a promoter of a filamentous fungus secretable protein, said promoter located in operable linkage 5' of the first catalytic domain. In a fifth embodiment, the promoter is a *cbh* promoter and preferably a *cbh*1 promoter derived from *T. reesei.* In a sixth embodiment, the first catalytic domain is derived from a CBH1 exo-cellobiohydrolase and particularly a CBH1 having an amino acid sequence of at least 90% sequence identity with the sequence set forth in SEQ ID NO.: 6. In a seventh embodiment, the second catalytic domain is an endoglucanase catalytic domain. In an eighth embodiment, the second catalytic domain is an exo-cellbiohydrolase catalytic domain. In a ninth embodiment, the second catalytic domain is derived from a bacterial cellulase. In a tenth embodiment, the second catalytic domain is selected from the group consisting of an *Acidothermus cellulolyticus* GH5A endoglucanase I (E1) catalytic domain; an *Acidothermus cellulolyticus* GH48 (GH48) cellulase catalytic domain; an *Acidothermus cellulolyticus* GH74 endoglucanase (GH74-EG) catalytic domain: a *Thermobifida fusca* E3 (Tf-E3) cellulase catalytic domain; and a *Thermobifida fusca* E5 endoglucanase (Tf-E5) catalytic domain. In an eleventh embodiment, the heterologous cellulase fusion construct lacks the cellulose binding domain of the exo-cellbiohydrolase of the first catalytic domain and the cellulose binding
domain of the cellulase of the second catalytic domain. In a twelfth embodiment, the second catalytic domain is an *Acidothermus cellulolyticus* GH5A E1 catalytic domain and particularly the *Acidothermus cellulolyticus* GH5A E1 catalytic domain having an amino acid sequence of at least 90% sequence identity with the sequence set forth in SEQ ID NO. 8. In a thirteenth embodiment, the heterologous cellulase fusion construct comprises a terminator sequence located 3' to the second catalytic domain. In a fourteenth embodiment, the heterologous fusion construct comprises a selectable marker.

In a second aspect, the invention includes a vector comprising in operable linkage from the 5' end, a promoter of a filamentous fungus secretable protein, a DNA. molecule encoding a signal sequence, a DNA molecule encoding a first catalytic domain, wherein said first catalytic domain is derived from a fungal exo-cellobiohydrolase, a DNA molecule encoding a second catalytic domain, wherein said second catalytic domain is the catalytic domain of a cellulase and a terminator. In one embodiment, the vector will further include a selectable marker. In a second embodiment, the vector will comprise a linker located 3' of the first catalytic domain and 5' of the second catalytic domain. In a third embodiment, the vector will lack the DNA sequence encoding cellulose binding domain of the first catalytic domain. In a fourth embodiment, the vector will comprise a kexin site. In a fifth embodiment, the second catalytic domain is derived from a bacterial cellulase. In a sixth embodiment, the vector lacks the DNA sequence encoding cellulose binding domain of the cellulase of the second catalytic domain.

In a third aspect, the invention includes a fungal host cell transformed with a heterologous cellulase fusion construct or a fungal host cell transformed with a vector comprising a heterologous cellulase fusion construct as described herein.

In a fourth aspect, the invention includes a recombinant fungal cell comprising the heterologous cellulase fusion construct or a vector comprising the same.

In a particularly preferred embodiment of the third and fourth aspects, the fungal host cell is a *Trichoderma* host cell and more particularly a strain of *T. reesei.* In another embodiment of these aspects, native cellulase genes, such as *cbh1, cbh2, egl1* and *egl2* have been deleted from the fungal cells. In a third embodiment, the native cellulose binding domain has been deleted from the fungal cells.

In a fifth aspect, the invention includes an isolated cellulase fusion protein having cellulolytic activity which comprises a first catalytic domain, wherein said first catalytic domain is an exo-cellobiohydrolase catalytic domain and a second catalytic domain, wherein said second catalytic domain is derived from a cellulase. In one embodiment of this aspect, the exo-cellobiohydrolase is a CBH1. In a second embodiment, the second catalytic domain is derived from a bacterial cellulase. In a third embodiment, the bacterial cellulase is an endoglucanase and in another embodiment the bacterial cellulase is an exo-cellobiohydrolase. In a fourth embodiment, the bacterial cellulase is derived from a strain of *Acidothermus cellulolyticus.* In a fifth embodiment, the invention concerns a cellulolytic composition comprising the isolated cellulase fusion protein.

In a sixth aspect, the invention includes a method of producing an enzyme having cellulolytic activity comprising, a) stably transforming a filamentous fungal host cell with a heterologous cellulase fusion construct or vector as defined above in the first aspect and second aspect; b) cultivating the transformed fungal host cell under conditions suitable for said fungal host cell to produce an enzyme having cellulolytic activity; and c) recovering said enzyme.

In one embodiment of this aspect, the filamentous fungal host cell is a *Trichoderma* cell, and particularly a *T. reesei* host cell. In a second embodiment, the exo-cellobiohydrolase is a CBH1 and the cellulase is an *Acidothermus cellulolyticus* cellulase or a *Thermobifida fusca* cellulase. In a third embodiment, the recovered enzyme is a cellulase fusion protein, components of the cellulase fusion protein, or a combination of the cellulase fusion protein and the components thereof. In a fourth embodiment, the recovered enzyme(s) is purified.

In an seventh aspect, the invention includes a *Trichoderma* host cell which expresses a cellulase fusion protein, wherein said fusion protein comprises a first catalytic domain, wherein the catalytic domain is derived from an exo-cellobiohydrolase and a second catalytic domain, wherein the second catalytic domain is derived from a cellulase enzyme. In one embodiment, the *Trichoderma* host cell is a *T. reesei* cell. In a second embodiment, the exo-cellobiohydrolase is a CBH1 and the cellulase is a bacterial cellulase. In a third embodiment, the bacterial cellulase is derived from an *Acidothermus cellulolyticus* cellulase and particularly an *Acidothermus cellulolyticus* E1, GH48 or GH74 cellulase. In a fourth embodiment, the fusion protein will lack the CBD of the cellulase and in other embodiments the fusion protein will include the CBD of the cellulase. In a fifth embodiment, the *T. reesei* host cell includes deleted native cellulase genes.

In an eighth aspect, the invention includes a fungal cellulase composition comprising a cellulase fusion protein or components thereof, wherein the fusion protein or components thereof is the product of a recombinant *Trichoderma spp.*

### BRIEF DESCRIPTION OF THE FIGURES

Figure 1 is a representation of a heterologous cellulase fusion construct encompassed by the invention, which includes a *Trichoderma reesei cbh1* promoter, a *cbh1* core (*cbh1* signal sequence and *cbh1* catalytic domain), a *cbh1* linker sequence, a kexin site, an E1 core (an *Acidothermus cellulolyticus* E1 endoglucanase catalytic domain), a *cbh1* terminator and an *A. nidulans amd*S selectable marker.

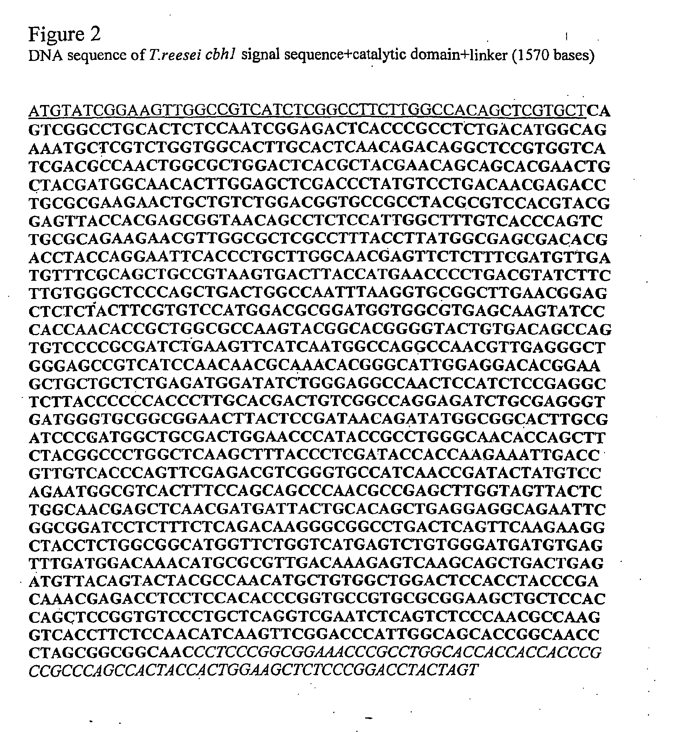

Figure 2 is a DNA sequence (SEQ ID NO: 1) of the *T. reesei cbh1* signal sequence (SEQ ID NO: 2); the *T. reesei cbh1* catalytic domain (SEQ ID NO: 3), and the *T. reesei cbh1* linker (SEQ ID NO: 4). The signal sequence is underlined, the catalytic domain is in bold, and the linker sequence is in italics.
Figure 3 shows the predicted amino acid sequence (SEQ ID NO: 5) based on the nucleotide sequence provided in Figure 2, wherein the signal peptide is underlined, the catalytic domain, represented by (SEQ ID NO: 6), is in bold, and the linker is in italics.

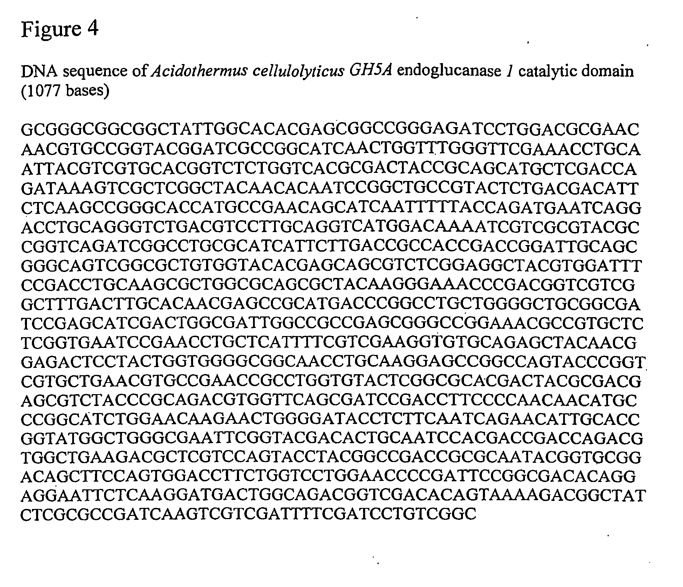

Figures 4 is an illustration of a nucleotide sequence (SEQ ID NO: 7) encoding an *Acidothermus cellulolyticus* GH5A endoglucanase I (E1) catalytic domain.

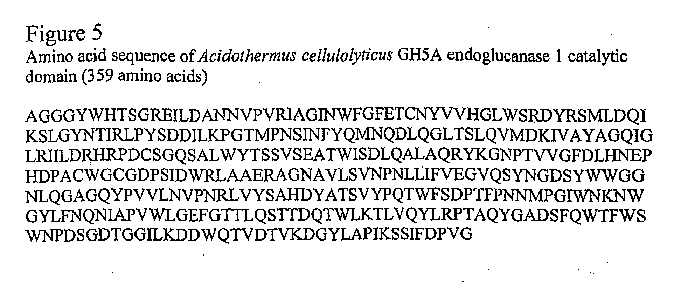

Figure 5 is the predicted amino acid sequence (SEQ ID NO: 8) of the *Acidothermus cellulolyticus* GH5A E1 catalytic domain based on the nucleotide sequence provided in Figure 4.

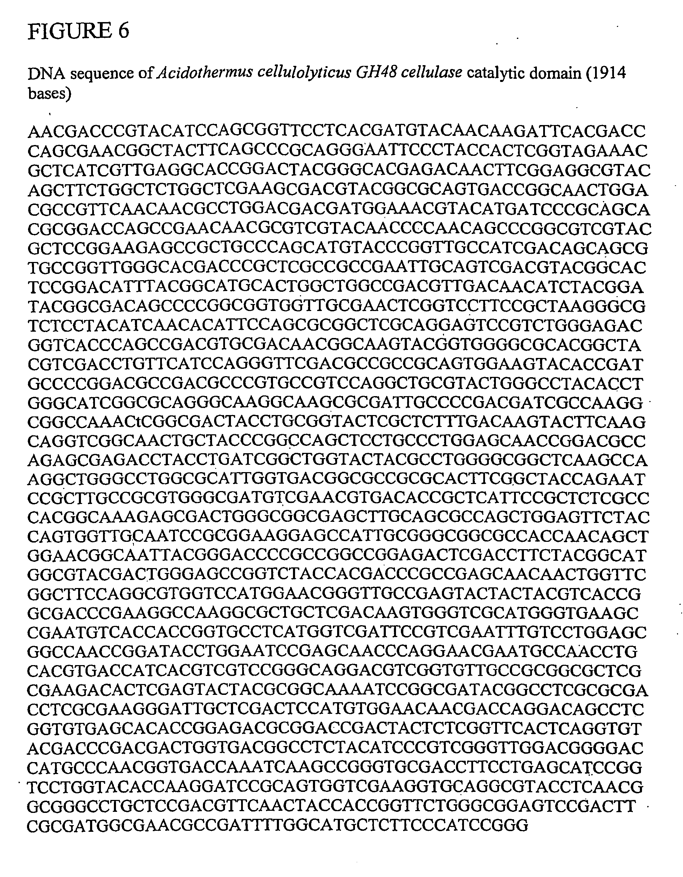

Figure 6 is an illustration of a nucleotide sequence (SEQ ID NO: 9) encoding an *Acidothermus cellulolyticus* GH48 cellulase catalytic domain.

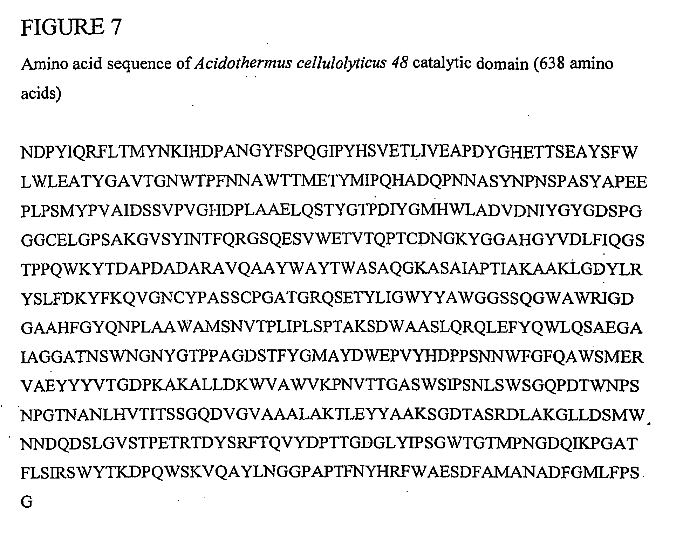

Figure 7 is the predicted amino acid sequence (SEQ ID NO: 10) of the *Acidothermus cellulolyticus* GH48 catalytic domain based on the nucleotide sequence provided in Figure 6.

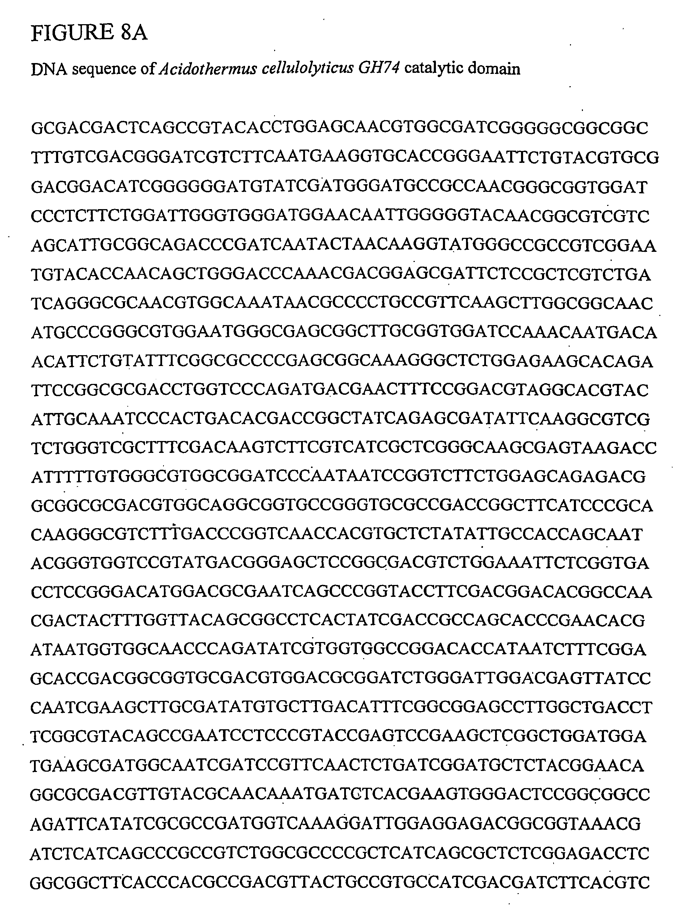

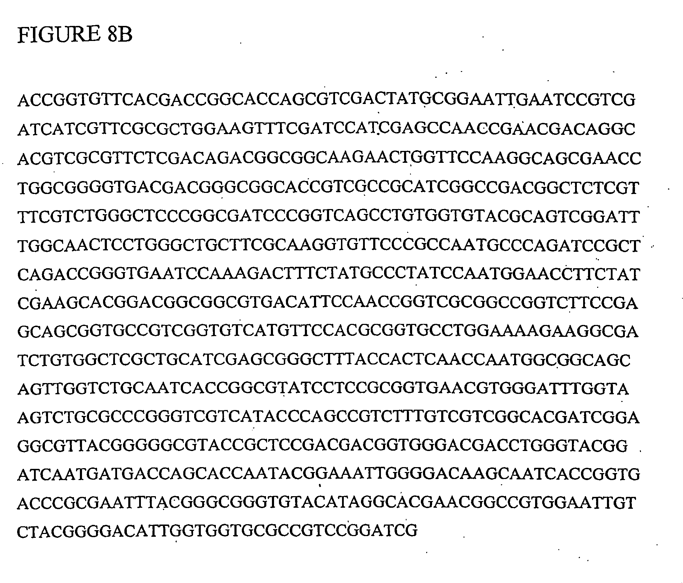

Figures 8A-8B are an illustration of a nucleotide sequence (SEQ ID NO: 11) encoding an *Acidothermus cellulolyticus* GH74- endoglucanase (EG) catalytic domain.

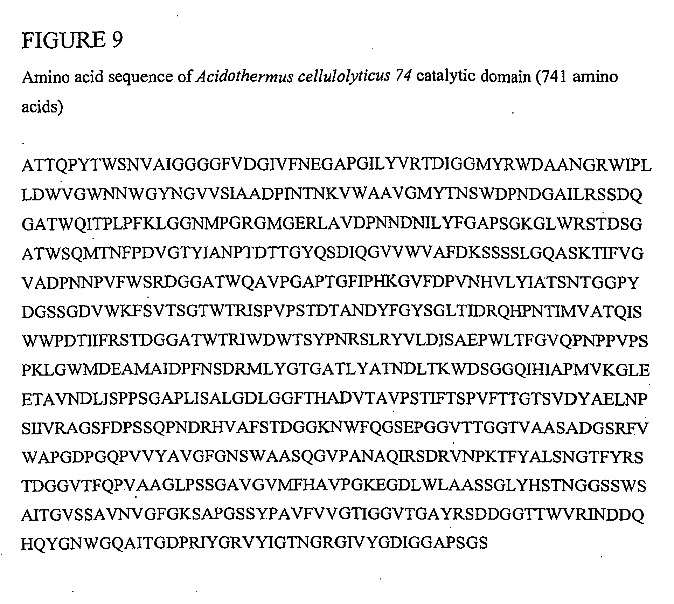

Figure 9 is the predicted amino acid sequence (SEQ ID NO: 12) of the *Acidothermus cellulolyticus* GH74-EG based on the nucleotide sequence provided in Figures 8A and 8B.

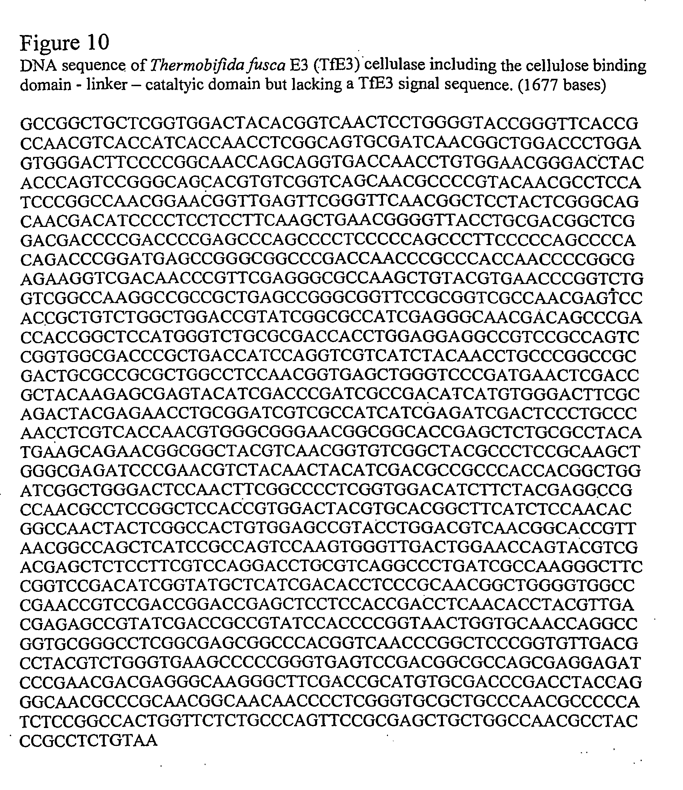

Figure 10 is an illustration of a nucleotide sequence (SEQ ID NO: 13) encoding the CBD, linker and catalytic domain of a *Thermobifida fusca* E-3 (TfE-3) cellulase.

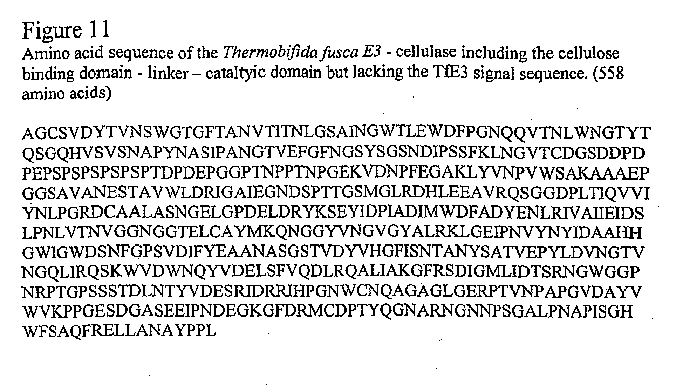

Figure 11 is the predicted amino acid sequence (SEQ ID NO: 14) of the TfE-3 CBD, linker and catalytic domain based on the nucleotide sequence provided in Figure 10.

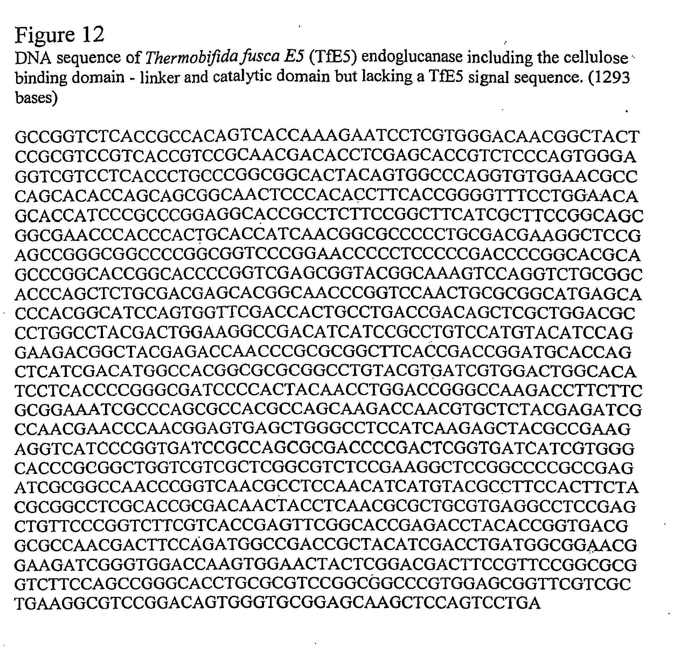

Figure 12 is an illustration of a nucleotide sequence (SEQ ID NO: 15) encoding the CBD, linker and catalytic domain of a *Thermobifida fusca* endoglucanase 5 (TfE5).

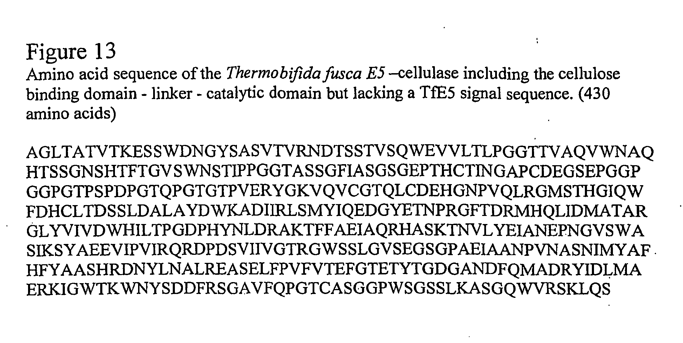

Figure 13 is the predicted amino acid sequence (SEQ ID NO: 16) of the TfE5 CBD, linker and catalytic domain based on the nucleotide sequence provided in Figure 12.
Figure 14 is the nucleotide sequence(2656 bases) (SEQ ID NO: 17) of a heterologous cellulase fusion construct described in example 1 comprising, the *T. reesei CBH1 signal* sequence; the catalytic domain of the *T. reesei* CBH1; the *T. reesei* CBH1 linker sequence; a kexin cleavage site which includes codons for the amino acids, SKR, and the sequence coding for the *Acidothermus cellulolyticus* GH5A-E1 catalytic domain.
Figure 15 is the predicted amino acid sequence (SEQ ID NO: 18) of the cellulase fusion protein based on the nucleic acid sequence in Figure 14.
Figure 16 provides a schematic diagram of the pTrex4 plasmid, which was used for expression of a heterologous fusion cellulase construct (CBH1-endoglucanase) as described in the examples and includes the *Trichoderma reesei cbh1* promoter, the *T. reesei CBH1* signal sequence, catalytic domain, and linker sequences, a kexin cleavage site and a cellulase gene of interest (in this example an endoglucanase) inserted between a Spel and Ascl site, a *cbh*I *Trichoderma reesei* terminator and the *amd*S *Aspergillus nidulans* acetamidase marker gene.
Figures 17A - E provide the nucleotide sequence (SEQ ID NO:19) (10239 bp) of the pTrex4 plasmid of Figure 16 excluding the cellulase gene of interest which includes the cellulose catalytic domain.
Figure 18 illustrates a SDS-PAGE gel of supernate samples of shake flask growth of clones of a *T. reesei* strain deleted for the cellulases, *cbh1, cbh2, egl1* and *egl2 and* transformed with the CBH1-E1 fusion construct. Lanes 1 and 10 represent MARK 12 Protein Standard (Invitrogen, Carlsbad, CA). Lanes 2 - 8 represent various transformants and lane 9 represents the untransformed *T. reesei* strain. The upper arrow indicates the cellulase fusion protein and the lower arrow indicates the cleaved E1 catalytic domain.
Figure 19 illustrates a SDS-PAGE gel of supernate samples of shake flask growth of clones of a *T. reesei* strain deleted for the cellulases, *cbh1, cbh2, egl1* and *egl2* and transformed with the CBH1-GH48 fusion construct. Lane1 represents the untransformed control. Lane 2 represents MARK12 Protein Standard (Invitrogen, Carlsbad, CA). Lanes 3 -12 represent various transformants. The arrow indicates the CBH1-GH48 fusion protein.
Figure 20 illustrates a SDS-PAGE gel of supernate samples of shake flask growth of clones of a *T. reesei* strain deleted for the cellulases, *cbh1, cbh2, egl1* and *eg*/2 and transformed with the CBH1-GH74 fusion construct. Lane 1 represents the untransformed control. Lane 3 represents MARK 12 Protein Standard (Invitrogen, Carlsbad, CA). Lanes 2 and 4 - 12 represent various transformants. The upper arrow around indicates the CBH1-GH74 fusion protein and the lower arrow indicates the cleaved GH74 catalytic domain.
Figure 21 illustrates a SDS-PAGE gel of supernate samples of shake flask growth of clones of a *T. reesei* strain deleted for the cellulases, *cbh1, cbh2, egl1* and egl2 and transformed with the CBH1-TfE3 fusion construct. Lane1 represents MARK 12 Protein Standard (Invitrogen, Carlsbad, CA). Lanes 2 - 12 represent various transformants. The arrow indicates new bands observed in the CBH1-TfE-3 fusion expressing transformants.
Figure 22 illustrates a SDS-PAGE gel of supernate samples of shake flask growth of clones of a *T*. *reesei* strain deleted for the cellulases, *cbh1, cbh2, egl1* and *eg*/*2* and transformed with the CBH1-TfE5 fusion construct. Lane1 represents MARK 12 Protein Standard (Invitrogen). Lane 2 represents the untransformed strain and lanes 3 -12 represent various transformants. Arrows indicate new bands observed in the CBH1-TfE5 fusion expressing transformants.
Figure 23 illustrates the % cellulose conversion to soluble sugars over time for a *T. reesei* parent strain comprising native cellulase genes with a corresponding *T. reesei* strain which expresses the CBH1-E1 fusion protein and reference is made to example 3.

### DETAILED DESCRIPTION OF THE INVENTION

The invention will now be described in detail by way of reference only using the following definitions and examples. All patents and publications, including all sequences disclosed within such patents and publications, referred to herein are expressly incorporated by reference.

Unless defined otherwise herein, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this invention. Practitioners are particularly directed to Sambrook et al., MOLECULAR CLONING: A LABORATORY MANUAL (Second and Third Editions), Cold Spring Harbor Press, Plainview, N.Y., 1989 and 2001, and Ausubel FM et al., Current Protocols in Molecular Biology, John Wiley & Sons, New York, NY, 1993, for definitions and terms of the art.

It is to be understood that this invention is not limited to the particular methodology, protocols, and reagents described, as these may vary. Although any methods and materials similar or equivalent to those described herein can be used in the practice or testing of the present invention, the preferred methods and materials are described.

Numeric ranges are inclusive of the numbers defining the range. Unless otherwise indicated, nucleic acids are written left to right in 5' to 3' orientation; amino acid sequences are written left to right in amino to carboxy orientation, respectively.

Other objects, features and advantages of the present invention will become apparent from the following detailed description. It should be understood, however, that the detailed description and specific examples, while indicating preferred embodiments of the invention, are given by way of illustration only, since various changes and modifications within the scope and spirit of the invention will become apparent to one skilled in the art from this detailed description.

### 1. DEFINITIONS

The term "heterologous cellulase fusion construct" refers to a nucleic acid construct that is composed of parts of different genes in operable linkage. The components include from the 5' end a DNA molecule encoding a first catalytic domain, wherein said first catalytic domain is a fungal exo-cellobiohydrolase catalytic domain and a DNA molecule encoding a second catalytic domain, wherein said second catalytic domain is a cellulase catalytic domain.

The term "cellulase fusion protein" or "fusion protein having cellulolytic activity" refers to an enzyme which exhibits cellulolytic activity and which has both a fungal exo-cellobiohydrolase catalytic domain and a cellulase catalytic domain.

The term "components of a cellulase fusion protein" refers to individual (cleaved) fragments of the cellulase fusion protein, wherein each fragment has cellulolytic activity and includes either the first or the second catalytic domain of the fusion protein.

The term "cellulase" refers to a category of enzymes capable of hydrolyzing cellulose (beta-1,4-glucan or beta D-glucosidic linkages) polymers to shorter cello-oligosaccharide oligomers, cellobiose and/or glucose.

The term "exo-cellobiohydrolase" (CBH) refers to a group of cellulase enzyme classified as EC 3.2.1.91. These enzymes are also known as exoglucanases or cellobiohydrolases. CBH enzymes hydrolyze cellobiose from the reducing or non-reducing end of cellulose. In general a CBH1 type enzyme preferentially hydrolyzes cellobiose from the reducing end of cellulose and a CBH2 type enzyme preferentially hydrolyzes the non-reducing end of cellulose.

The term "endoglucanase" (EG) refers to a group of cellulase enzymes classified as EC 3.2.1.4. An EG enzyme hydrolyzes internal beta-1,4 glucosidic bonds of the cellulose.

The term "beta-glucosidases" refers to a group of cellulase enzymes classified as EC 3.2.1.21.

"Cellulolytic activity" encompasses exoglucanase activity, endoglucanase activity or both types of enzymatic activity.

The term "catalytic domain" refers to a structural portion or region of the amino acid sequence of a cellulase which possess the catalytic activity of the cellulase. The catalytic domain is a structural element of the cellulase tertiary structure that is distinct from the cellulose binding site, which is a structural element which binds the cellulase to a substrate such as cellulose.

The term "cellulose binding domain (CBD)" as used herein refers to a portion of the amino acid sequence of a cellulase or a region of the enzyme that is involved in the cellulose binding activity of a cellulase. Cellulose binding domains generally function by non-covalently binding the cellulase to cellulose, a cellulose derivative or other polysaccharide equivalent thereof. CBDs typically function independent of the catalytic domain.

The term "first catalytic domain" refers to the catalytic domain of a fungal exo-cellobiohydrolase.

The term "second catalytic domain" or "cellulase catalytic domain" refers to the catalytic domain of a cellulase enzyme, wherein the cellulase enzyme may be an exo-cellobiohydrolase or an endoglucanase and wherein the catalytic domain is operably linked to the 3' end of the first catalytic domain.

A nucleic acid is "operably linked" when it is placed into a functional relationship with another nucleic acid sequence. For example, DNA encoding a signal peptide is operably linked to DNA encoding a polypeptide if it is expressed as a preprotein that participates in the secretion of the polypeptide; a promoter is operably linked to a coding sequence if it affects the transcription of the sequence. Generally, "operably linked" means that the DNA sequences being linked are contiguous, and, in the case of the heterologous cellulase fusion construct contiguous and in reading frame.

As used herein, the term "gene" means the segment of DNA involved in producing a polypeptide chain, that may or may not include regions preceding and following the coding region, *e.g*. 5' untranslated (5' UTR) or "leader" sequences and 3' UTR or "trailer" sequences, as well as intervening sequences (introns) between individual coding segments (exons).

The term "polypeptide" as used herein refers to a compound made up of a single chain of amino acid residues linked by peptide bonds. The term "protein" as used herein may be synonymous with the term "polypeptide" or may refer, in addition, to a complex of two or more polypeptides.

The term "nucleic acid molecule", "nucleic acid" or "polynucleotide" includes RNA, DNA and cDNA molecules. It will be understood that, as a result of the degeneracy of the genetic code, a multitude of-nucleotide sequences encoding a given protein such as a cellulase fusion protein, may be produced.

A "heterologous" nucleic acid sequence has a portion of the sequence, which is not native to the cell in which it is expressed. For example, heterologous, with respect to a control sequence refers to a control sequence (*i.e*. promoter or enhancer) that does not function in nature to regulate the same gene the expression of which it is currently regulating. Generally, heterologous nucleic acid sequences are not endogenous to the cell or part of the genome in which they are present, and have been added to the cell, by infection, transfection, transformation, microinjection, electroporation, or the like. A "heterologous" nucleic acid sequence may contain a control sequence/DNA coding sequence combination that is the same as, or different from a control sequence/DNA coding sequence combination found in the native cell. The term heterologous nucleic acid sequence encompasses a heterologous cellulase fusion construct according to the invention.

As used herein, the term "vector" refers to a nucleic acid sequence or construct designed for transfer between different host cells. An "expression vector" refers to a vector that has the ability to incorporate and express heterologous DNA sequences in a foreign cell. An expression vector may be generated recombinantly or synthetically, with a series of specified nucleic acid elements that permit transcription of a particular nucleic acid in a target cell. The recombinant expression cassette can be incorporated into a plasmid, chromosome, mitochondrial DNA, virus, or nucleic acid fragment.

As used herein, the term "plasmid" refers to a circular double-stranded (ds) DNA construct used as a cloning vector, and which forms an extrachromosomal self-replicating genetic element in many bacteria and some eukaryotes.

As used herein, the term "selectable marker" refers to a nucleotide sequence which is capable of expression in cells and where expression of the selectable marker confers to cells containing the expressed gene the ability to grow in the presence of a corresponding selective agent, or under corresponding selective growth conditions.

As used herein, the term "promoter" refers to a nucleic acid sequence that functions to direct transcription of a downstream gene. The promoter will generally be appropriate to the host cell in which the target gene is being expressed. The promoter together with other transcriptional and translational regulatory nucleic acid sequences (also termed "control sequences") are necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

The term "signal sequence" or "signal peptide" refers to a sequence of amino acids at the N-terminal portion of a protein, which facilitates the secretion of the mature form of the protein outside the cell. The mature form of the extracellular protein lacks the signal sequence which is cleaved off during the secretion process.

By the term "host cell" is meant a cell that contains a heterologous cellulase fusion construct encompassed by the invention or a vector including the same and supports the replication, and/or transcription or transcription and translation (expression) of the heterologous cellulase fusion construct. Host cells for use in the present invention can be prokaryotic cells, such as *E*. *coli,* or eukaryotic cells such as yeast, plant, insect, amphibian, or mammalian cells. In general, host cells are filamentous fungi.

The term "filamentous fungi" includes all filamentous fungi recognized by those of skill in the art. A preferred fungus is selected from the subdivision Eumycota and Oomycota and particularly from the group consisting of *Aspergillus, Trichoderma, Fusarium, Ghrysosporium, Penicillium, Humicola, Neurospora,* or alternative sexual forms thereof such as *Emericella* and *Hypocrea* (See, Kuhls et al., 1996).

The filamentous fungi are characterized by vegetative mycelium having a cell wall composed of chitin, glucan, chitosan, mannan, and other complex polysaccharides, with vegetative growth by hyphal elongation and carbon catabolism that is obligately aerobic.

The term "derived" encompasses the terms, originated from, obtained or obtainable from, and isolated from.

An "equivalent" amino acid sequence is an amino acid sequence that is not identical to an original reference amino acid sequence but includes some amino acid changes, which may be substitutions, deletions, additions or the like, wherein the protein exhibits essentially the same qualitative biological activity of the reference protein. An equivalent amino acid sequence will have between 80% - 99% amino acid identity to the original reference sequence. Preferably the equivalent amino acid sequence will have at least 85%, 90%, 93%, 95%, 96%, 98% and 99% identity to the reference sequence.

A "substitution" results from the replacement of one or more nucleotides or amino acid by different nucleotides or amino acids, respectively. Substitutions are usually made in accordance with known conservative substitutions, wherein one class of amino acid is substituted with an amino acid in the same class. A "non-conservative substitution" refers to the substitution of an amino acid in one class with an amino acid from another class.

A "deletion" is a change in a nucleotide or amino acid sequence in which one or more nucleotides or amino acids are absent.

An "addition" is a change in a nucleotide or amino acid sequence that has resulted from the insertion of one or more nucleotides or amino acid as compared to an original reference sequence.

As used herein, "recombinant" includes reference to a cell or vector, that has been modified by the introduction of heterologous nucleic acid sequences or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention.

As used herein, the terms "transformed", "stably transformed" or "transgenic" with reference to a cell means the cell has a heterologous nucleic acid sequence according to the invention integrated into its genome or as an episomal plasmid that is maintained through multiple generations.

The term "introduced" in the context of inserting a heterologous cellulase fusion construct or heterologous nucleic acid sequence into a cell, means "transfection", "transformation" or "transduction" and includes reference to the incorporation of a heterologous nucleic acid sequence or heterologous cellulase fusion construct into a eukaryotic or prokaryotic cell where the heterologous nucleic acid sequence or heterologous cellulase nucleic acid construct may be incorporated into the genome of the cell (for example, chromosome, plasmid, plastid, or mitochondrial DNA), converted into an autonomous replicon, or transiently expressed (for example, transfected mRNA).

As used herein, the term "expression" refers to the process by which a polypeptide is produced based on the nucleic acid sequence of a gene. The process includes both transcription and translation.

It follows that the term "cellulase fusion protein expression" or "fusion expression" refers to transcription and translation of a "heterologous cellulase fusion construct" comprising a first catalytic domain fused to a second cellulase catalytic domain, the products of which include precursor RNA, mRNA, polypeptide, post-translationally processed polypeptides, and derivatives thereof.

As used herein, the term "purifying" generally refers to subjecting recombinant nucleic acid or protein containing cells to biochemical purification and/or column chromatography.

As used herein, the terms "active" and "biologically active" refer to a biological activity associated with a particular protein, such as the enzymatic activity associated with a cellulase. It follows that the biological activity of a given protein refers to any biological activity typically attributed to that protein by those of skill in the art.

As used herein, the term "enriched" means that the concentration of a cellulase enzyme found in a fungal cellulase composition is greater relative to the concentration found in a wild type or naturally occurring fungal cellulase composition. The terms enriched, elevated and enhanced may be used interchangeably herein.

A "wild type fungal cellulase composition" is one produced by a naturally occurring fungal source and which comprises one or more BG, CBH and EG components wherein each of these components is found at the ratio produced by the fungal source.

Thus, to illustrate, a naturally occurring cellulase system may be purified into substantially pure components by recognized separation techniques well published in the literature, including ion exchange chromatography at a suitable pH, affinity chromatography, size exclusion and the like. A purified cellulase fusion protein or components thereof may then be added to the enzymatic solution resulting in an enriched cellulase solution. It is also possible to elevate the amount of EG or CBH produced by a microbe by expressing a cellulase fusion protein encompassed by the invention.

"A", "an" and "the" include plural references unless the context clearly dictates otherwise.

As used herein the term "comprising" and its cognates are used in their inclusive sense: that is equivalent to the term "including" and its corresponding cognates.

"ATCC" refers to American Type Culture Collection located in Manassas VA 20108 (ATCC, www/atcc.org).

"NRRL" refers to the Agricultural Research Service Culture Collection, National Center for Agricultural utilization Research (and previously known as USDA Northern Regional Research Laboratory), Peoria, ILL.

### 2. PREFERRED EMBODIMENTS

### A. Components and Construction of Heterologous Cellulase Fusion Constructs and Expression Vectors.

A heterologous cellulase fusion construct or a vector comprising the heterologous cellulase fusion construct may be introduced into and replicated in a filamentous fungal host cell for protein expression and secretion.

The components of the heterologous cellulase fusion construct comprise in operable linkage from the 5' end of said construct, optionally a signal peptide, a DNA molecule encoding a first catalytic domain, wherein said first catalytic domain is derived from a fungal exo-cellobiohydrolase (CBH) and a DNA molecule encoding a second catalytic domain, wherein the second catalytic domain is derived from a cellulase enzyme.

In another embodiment, the heterologous cellulase fusion comprises in operable linkage from the 5' end of said construct, a signal peptide, a DNA molecule encoding a CBH catalytic domain, a DNA molecule encoding the CBD of a second cellulase catalytic domain, and a DNA molecule encoding the second cellulase catalytic domain, wherein said cellulase second catalytic domain is derived from a bacterial cellulase.

In another embodiment, the construct will comprise in operable linkage from the 5' end of said construct, a signal peptide, a DNA molecule encoding a catalytic domain of a CBH, optionally a DNA molecule encoding the CBD of the CBH, a linker, optionally a DNA molecule encoding a CBD of a second catalytic domain, and a DNA molecule encoding the second catalytic domain.

In a further embodiment, the heterologous cellulase fusion construct or an expression vector comprises in operable linkage from the 5' end a promoter of a filamentous fungus secretable protein; a DNA molecule encoding a signal sequence; a DNA molecule encoding a first catalytic domain, a linker, optionally a DNA encoding the CBD of a second catalytic domain, a DNA encoding the second catalytic domain, and a terminator.

One preferred expression vector will include in operable linkage from the 5' end, a promoter of a filamentous fungus secretable protein, a DNA molecule encoding a fungal exo-cellobiohydrolase signal sequence, a DNA molecule encoding a catalytic domain of an exo-cellobiohydrolase, a linker, a DNA molecule encoding a cellulase catalytic domain, and a terminator.

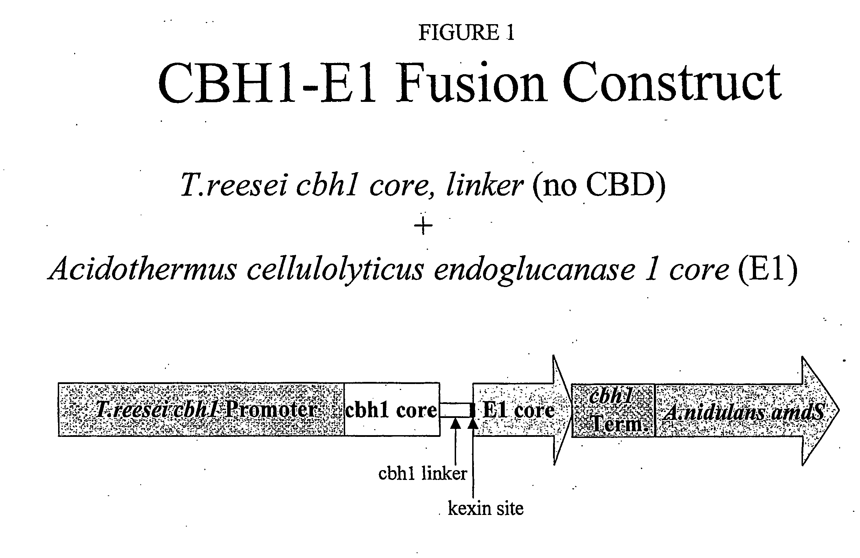

In some embodiments the vector will include the CBD of the exo-cellobiohydrolase and in some embodiments the vector will include the CBD of the cellulase of the second catalytic domain. In a preferred embodiment, the coding sequence for the cellulase catalytic domain (either including the cellulase CBD or lacking the cellulase CBD) will not include the cellulase signal sequence. Reference is made to Figures 1, 14 and 16 as examples of embodiments including an expression vector and heterologous cellulase fusion construct of the invention.

Exemplary promoters include both constitutive promoters and inducible promoters. Examples include the promoters from the *Aspergillus niger, A awamori* or *A.* oryzae glucoamylase, alpha-amylase, or alpha-glucosidase encoding genes; the *A. nidulans gpdA* or *trpC* Genes; the *Neurospora* crassa *cbh1* or *trp1* genes; the *A. niger* or *Rhizomucor miehei* aspartic proteinase encoding genes; the *T. reesei cbh1, cbh2, egl1, egl2,* or other cellulase encoding genes; a CMV promoter, an SV40 early promoter, an RSV promoter, an EF-1α promoter, a promoter containing the tet responsive element (TRE) in the tet-on or tet-off system as described (ClonTech and BASF), the beta actin promoter. In some embodiments the promoter is one that is native to the fungal host cell to be transformed.

In one preferred embodiment, the promoter is an exo-cellobiohydrolase *cbh1* or *cbh2* promoter and particularly a *cbh1* promoter, such as a *T. reesei cbh1* promoter. The T. *reesei cbh1* promoter is an inducible promoter, and reference is made to GenBank Accession No. D86235.

The DNA sequence encoding an exo-cellobiohydrolase catalytic domain is operably linked to a DNA sequence encoding a signal sequence. The signal sequence is preferably that which is naturally associated with the exo-cellobiohydrolase to be expressed. Preferably the signal sequence is encoded by a *Trichoderma* or *Aspergillus* gene which encodes a CBH. More preferably the signal sequence is encoded by a *Trichoderma* gene which encodes a CBH1. In further embodiments, the promoter and signal sequence of the heterologous cellulase fusion construct are derived from the same source. In some embodiments, the signal sequence is a *Trichoderma cbh1* signal sequence that is operably linked to a *Trichoderma cbh1* promoter. In further embodiments, the signal sequence has the amino acid sequence of SEQ ID NO: 2 or a sequence having at least 95% identity thereto.

Most exo-cellobiohydrolases (CBHs) and endoglucanases (EGs) have a multidomain structure consisting of a catalytic domain separated from a cellulose binding domain (CBD) by a linker peptide (Suurnakki *et al.,* 2000). The catalytic domain contains the active site whereas the CBD interacts with cellulose by binding the enzyme to it (van Tilbeurgh *et al.,* 1986 and Tomme *et al.,* 1988).

Numerous cellulases have been described in the scientific literature, examples of which include: from *Trichoderma reesei:* Shoemaker, S. et al., Bio/Technology, 1:691-696, 1983, which discloses CBH1; Teeri, T. et al., Gene, 51:43-52, 1987, which discloses CBH2; Penttila, M. et al., Gene, 45:253-263, 1986, which discloses EG1; Saloheimo, M. et al., Gene, 63:11-22, 1988, which discloses EG2; Okada, M. et al., Appl. Environ. Microbiol., 64:555-563, 1988, which discloses EG3; Saloheimo, M. et al., Eur. J. Biochem., 249:584-591, 1997, which discloses EG4; and Saloheimo, A. et al., Molecular Microbiology, 13:219-228, 1994, which discloses EG5. Exo-cellobiohydrolases and endoglucanases from species other than *Trichoderma* have also been described e.g., Ooi *et al.,* 1990, which discloses the cDNA sequence coding for endoglucanase F1-CMC produced by *Aspergillus aculeatus;* Kawaguchi T et *al.,* 1996, which discloses the cloning and sequencing of the cDNA encoding beta-glucosidase 1 from *Aspergillus aculeatus;* Sakamoto *et al.,* 1995, which discloses the cDNA sequence encoding the endoglucanase CMCase-1 from *Aspergillus kawachii* IFO 4308; and Saarilahti *et al.,* 1990 which discloses an endoglucanase from *Erwinia carotovara.* The sequences encoding these enzymes may be used in the heterologous cellulase fusion construct of the invention.

In some embodiments the first catalytic domain is derived from a fungal CBH. In other embodiments the CBH is a CBH1 type exo-cellobiohydrolase and in other embodiments the catalytic domain is derived from a CBH2 type exo-cellobiohydrolase. In some embodiments, the CBH1 catalytic domain is derived from a *Trichoderma* spp.

In one embodiment, the first catalytic domain is encoded by a nucleic acid sequence of a *Trichoderma reesei cbh1.* In some embodiments the nucleic acid is the sequence of SEQ ID NO:3 and nucleotide sequences homologous thereto.

In other embodiments, the first catalytic domain will have the amino acid sequence of SEQ ID NO: 6 and equivalent amino acid sequences thereto. Further DNA sequences encoding any equivalents of said amino acid sequences of SEQ ID NO: 6, wherein said equivalents have a similar qualitative biological activity to SEQ ID NO: 6 may be incorporated into the heterologous cellulase fusion construct.

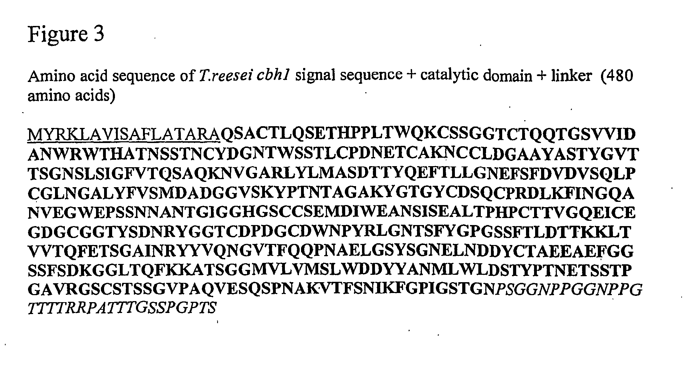

In some embodiments, heterologous cellulase fusion constructs encompassed by the invention will include a linker located 3' to the sequence encoding the exo-cellobiohydrolase catalytic domain and 5' to the sequence encoding the endoglucanase catalytic domain. In some preferred embodiments, the linker is derived from the same source as the catalytic domain of the exo-cellobiohydrolase. Preferably the linker will be derived from a *Trichoderma cbh1* gene. One preferred linker sequence is illustrated in Figure 3. In other embodiments, the heterologous cellulase fusion construct or a an expression vector may include two or more linkers. For example, a linker may be located not only between the coding sequence of the first catalytic domain and the coding sequence of the second catalytic domain, but also between the coding region of the CBD of the second catalytic domain and the coding region of the second catalytic domain. In general a linker may be between about 5 to 60 amino acid residues, between about 15 to 50 amino acid residues, and between about 25 to 45 amino acid residues. Reference is made to Srisodsuk M. et al., 1993 for a discussion of the linker peptide of *T. reesei* CBH1.

In addition to the linker sequence, a heterologous cellulase fusion construct or expression vector including a cellulase fusion construct may include a cleavage site, such as a protease cleavage site. In one preferred embodiment, the cleavage site is a kexin site which encodes the dipeptide Lys-Arg.

The heterologous cellulase fusion constructs include a coding sequence for a second catalytic domain of a cellulase. The cellulase may be from a fungal or bacterial source. Additionally, the cellulase may be an exo-cellobiohyrolase (CBH) or an endoglucanase (EG). In some preferred embodiments, the second catalytic domain will be derived from a bacterial cellulase. Source of both CBH and EG cellulases are mentioned above. Additionally, endoglucanases are found in more than 13 of the Glycosyl Hydrolase families using the classification of Coutinho, P. M. et al. (1999) Carbohydrate-Active Enzymes (CAZy) server at afmb.cnrs-mrs.fr/~cazy/CAZY/index.

Particularly preferred DNA sequences encoding a second catalytic domain of a bacterial cellulase include:
a) the DNA of SEQ ID NO: 7 encoding an *Acidothermus cellulolyticus* GH5A endoglucanase I (E1) catalytic domain having amino acid sequence SEQ ID NO: 8;
b) the DNA sequence of SEQ ID NO: 9 encoding an *Acidothermus cellulolyticus* GH48 cellulase catalytic domain having amino acid sequence SEQ ID NO: 10;
c) the DNA of SEQ ID NO: 11 encoding an *Acidothermus cellulolyticus* GH74 endoglucanase catalytic domain having amino acid sequence SEQ ID NO: 12;
d) the DNA of SEQ ID NO:13 encoding a *Thermobifida fusca* E3 cellulase having amino acid sequence SEQ ID NO: 14;
e) the DNA sequence of SEQ ID NO: 15 encoding a *Thermobifida fusca* E5 endoglucanase having amino acid sequence SEQ ID NO: 16 and
f) DNA sequences or homologous DNA sequences encoding any equivalents of said amino acid sequences of SEQ ID NOs: 8, 10, 12, 14 and 16, wherein said equivalents have a similar qualitative biological activity to said sequences.

In some preferred embodiments the endoglucanase is an *Acidothermus cellulolyticus* E1 and reference is made to the an *Acidothermus cellulolyticus* endoglucanases disclosed in WO 9105039; WO 9315186; USP 5,275,944; WO 9602551; USP 5,536,655 and WO 0070031. Also reference is made to GenBank U33212. In some embodiments, the *Acidothermus cellulolyticus* E1 has an amino acid sequence of a least 90%, 93%, 95% and 98% sequence identity with the sequence set forth in SEQ ID NO: 6.

As stated above homologous nucleic acid sequences to the nucleic acid sequences illustrated in SEQ ID NOs: 1, 3, 7, 9, 11, 13, and 15 may be used in a heterologous cellulase fusion construct or vector according to the invention. Homologous sequences include sequences found in other species, naturally occurring allelic variants and biologically active functional derivatives. A homologous sequence will have at least 80%, 85%, 88%, 90%, 93%, 95%, 97%, 98% and 99% identity to one of the sequences of SEQ ID NOs: 1, 3, 7, 9, 11, 13 and 15 when aligned using a sequence alignment program. For example, a homologue of a given sequence has greater than 80% sequence identity over a length of the given sequence *e.g*., the coding sequence for the Tf-E3 catalytic domain as described herein.

For a given heterologous cellulase fusion construct or components of the construct it is appreciated that as a result of the degeneracy of the genetic code, a number of coding sequences can be produced that encode a protein having the same amino acid sequence. For example, the triplet CGT encodes the amino acid arginine. Arginine is alternatively encoded by CGA, CGC, CGG, AGA, and AGG. Therefore it is appreciated that such substitutions in the coding region fall within the nucleic acid sequences covered by the present invention. Any and all of these sequences can be utilized in the same way as described herein as a CBH catalytic domain or a cellulase catalytic domain.

Exemplary computer programs which can be used to determine identity between two sequences include, but are not limited to, the suite of BLAST programs, *e.g*., BLASTN, BLASTX, and TBLASTX, BLASTP and TBLASTN, publicly available on the Internet at www.ncbi.nlm.nih.gov/BLAST/. See also, Altschul, *et al.,* 1990 and Altschul, *et al.,* 1997.

Sequence searches are typically carried out using the BLASTN program when evaluating a given nucleic acid sequence relative to nucleic acid sequences in the GenBank DNA Sequences and other public databases. The BLASTX program is preferred for searching nucleic acid sequences that have been translated in all reading frames against amino acid sequences in the GenBank Protein Sequences and other public databases. Both BLASTN and BLASTX are run using default parameters of an open gap penalty of 11.0, and an extended gap penalty of 1.0, and utilize the BLOSUM-62 matrix. (See, *e.g*., Altschul, *et al.,* 1997.)

A preferred alignment of selected sequences in order to determine "% identity" between two or more sequences, is performed using for example, the CLUSTAL-W program in MacVector version 6.5, operated with default parameters, including an open gap penalty of 10.0, an extended gap penalty of 0.1, and a BLOSUM 30 similarity matrix.

In one exemplary approach, sequence extension of a nucleic acid encoding a CBH or EG catalytic domain may be carried out using conventional primer extension procedures as described in Sambrook *et al., supra,* to detect CBH or bacterial EG precursors and processing intermediates of mRNA that may not have been reverse-transcribed into cDNA and/or to identify ORFs that encode the catalytic domain or full length protein.

In yet another aspect, the entire or partial nucleotide sequence of the nucleic acid sequence of the *T. reesei chb*1 or GH5a-E1 may be used as a probe. Such a probe may be used to identify and clone out homologous nucleic acid sequences from related organisms.

Screening of a cDNA or genomic library with the selected probe may be conducted using standard procedures, such as described in Sambrook *et al.,* (1989). Hybridization conditions, including moderate stringency and high stringency, are provided in Sambrook *et al., supra.*

**In addition, a**lignment of amino acid sequences to determine homology or identity between sequences is also preferably determined by using a "sequence comparison algorithm." Optimal alignment of sequences for comparison can be conducted, *e.g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), by visual inspection or MOE by Chemical Computing Group, Montreal Canada.

An example of an algorithm that is suitable for determining sequence similarity is the BLAST algorithm, which is described in Altschul, et al., J. Mol. Biol. 215:403-410 (1990) and reference is also made to Henikoff & Henikoff, Proc. Natl. Acad. Sci. USA 89:10915 (1989)). Software for performing BLAST analyses is publicly available through the National Center for Biotechnology Information (<www.ncbi.nlm.nih.gov>).

The heterologous cellulase fusion construct according to the invention may also include a terminator sequence. In some embodiments the terminator and the promoter are derived from the same source, for example a *Trichoderma* exo-cellobiohydrolase gene. In other embodiments the terminator and promoter are derived from different sources. In preferred embodiments the terminator is derived from a filamentous fungal source and particular a *Trichoderma.* Particularly suitable terminators include *cbh*1 derived from a strain of *Trichoderma* specifically *T. reesei* and the glucoamylase terminator derived from *Aspergillus niger* or A. *awamori.* (Nunberg et al., 1984 and Boel et al.,1984).

The heterologous fusion construct or a vector comprising the fusion construct may also include a selectable marker. The choice of the proper selectable marker will depend on the host cell, and appropriate markers for different hosts are well known in the art. Typical selectable marker genes include argB from A. *nidulans* or *T. reesei, amd*S from *A. nidulans, pyr*4 from *Neurospora crassa* or *T. reesei, pyr*G from *Aspergillus niger or A. nidulans.* Markers useful in vector systems for transformation of Trichoderma are described in Finkelstein, Chap. 6, in BIOTECHNOLOGY OF FILAMENTOUS FUNGI, Finkelstein et al eds. Butterworth-Heinemann, Boston, MA 1992. The *amd*S gene from *Aspergillus nidulans* encodes the enzyme acetamidase that allows transformant cells to grow on acetamide as a nitrogen source (Kelley et al., EMBO J. 4:475-479 (1985) and Penttila et al., Gene 61:155-164 (1987)). The selectable marker (e.g. *pyr*G*)* may restore the ability of an auxotrophic mutant strain to grow on a selective minimal medium and the selectable marker (e.g. *olic*31) may confer to transformants the ability to grow in the presence of an inhibitory drug or antibiotic

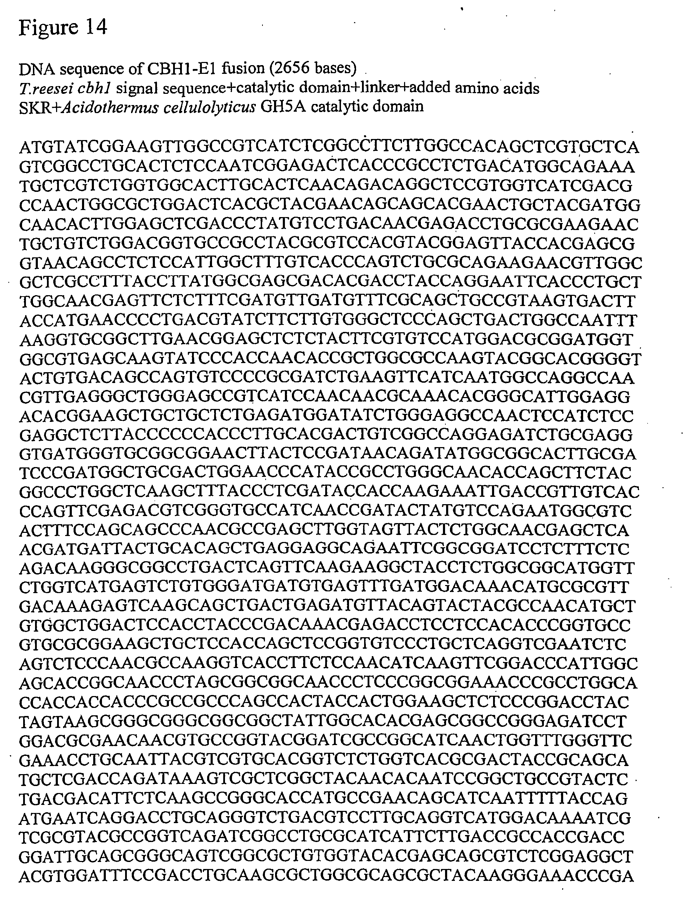

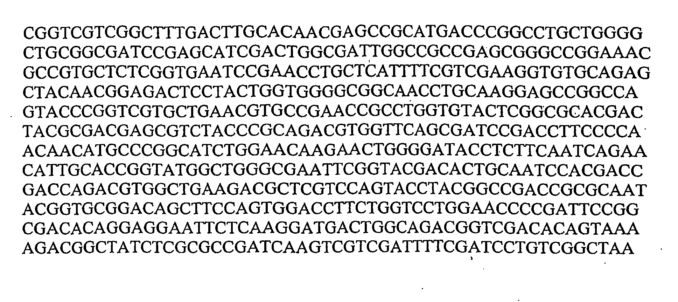

A typical heterologous cellulase fusion construct is depicted in Figures 1 and 14. Methods used to,ligate a heterologous cellulase nucleic acid construct encompassed by the invention and other heterologous nucleic acid sequences and to insert them into suitable vectors are well known in the art. Linking is generally accomplished by ligation at convenient restriction sites, and if such sites do not exist, synthetic oligonucleotide linkers are used in accordance with conventional practice. Additionally vectors can be constructed using known recombination techniques.

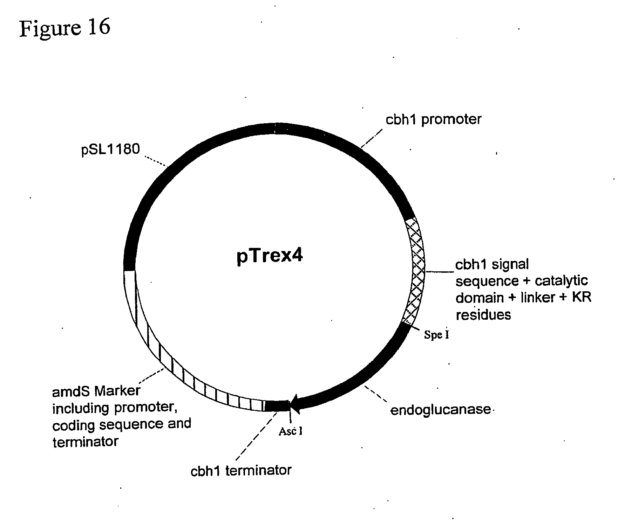

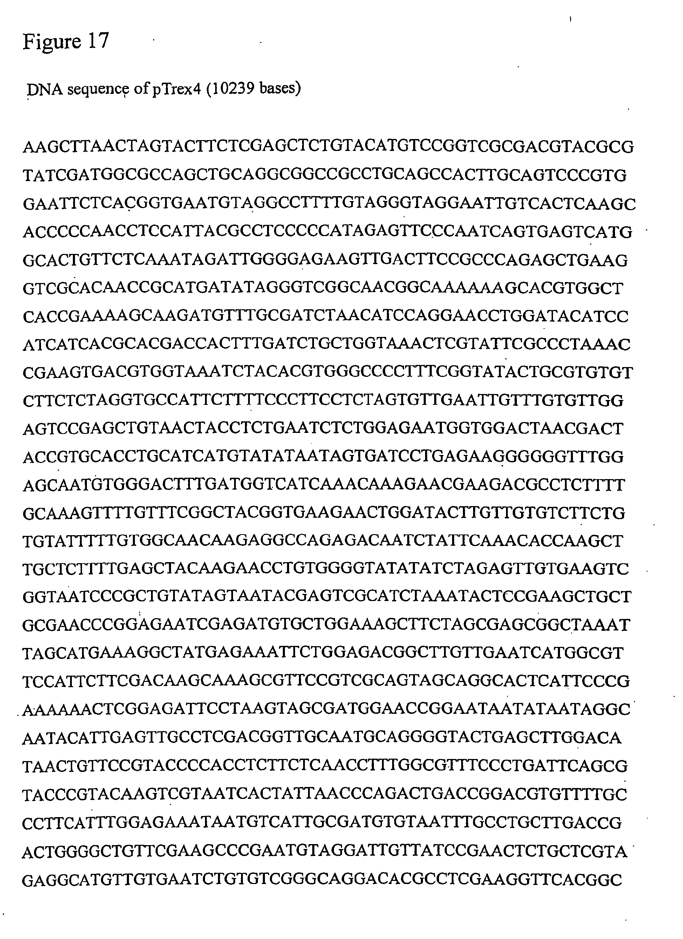

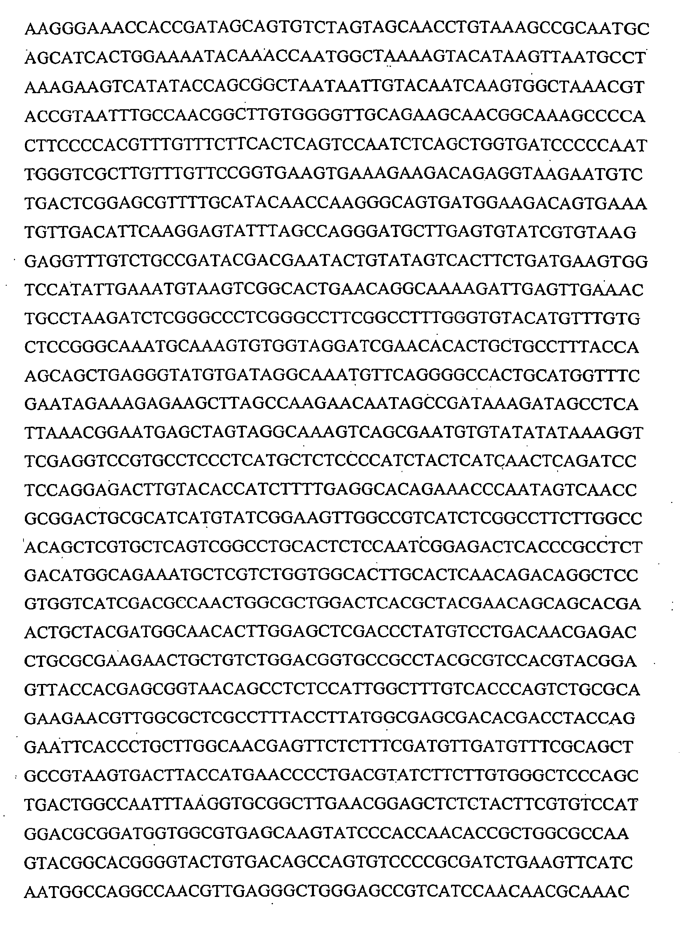

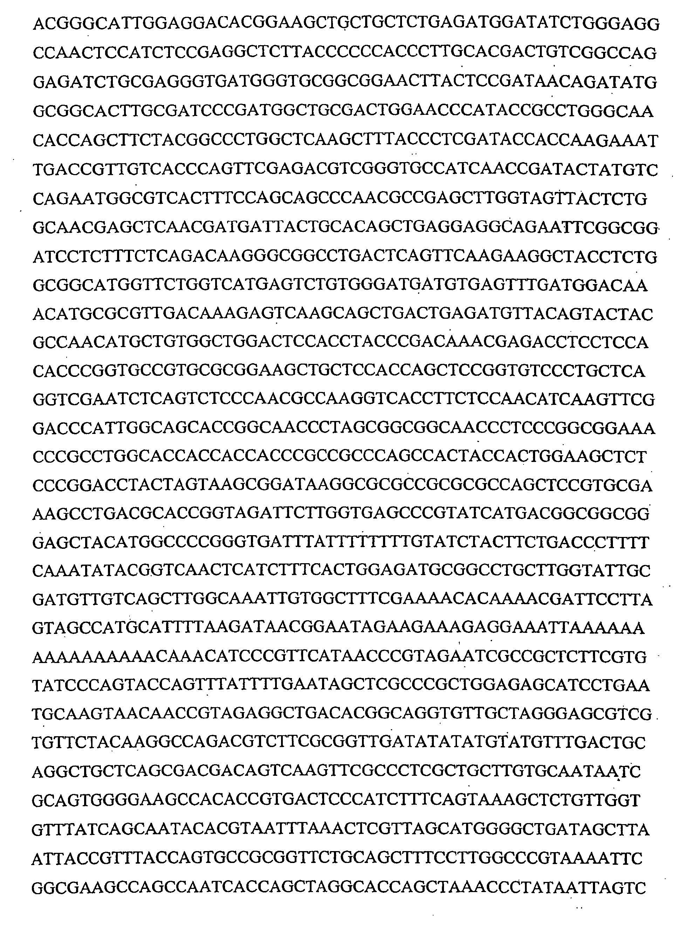

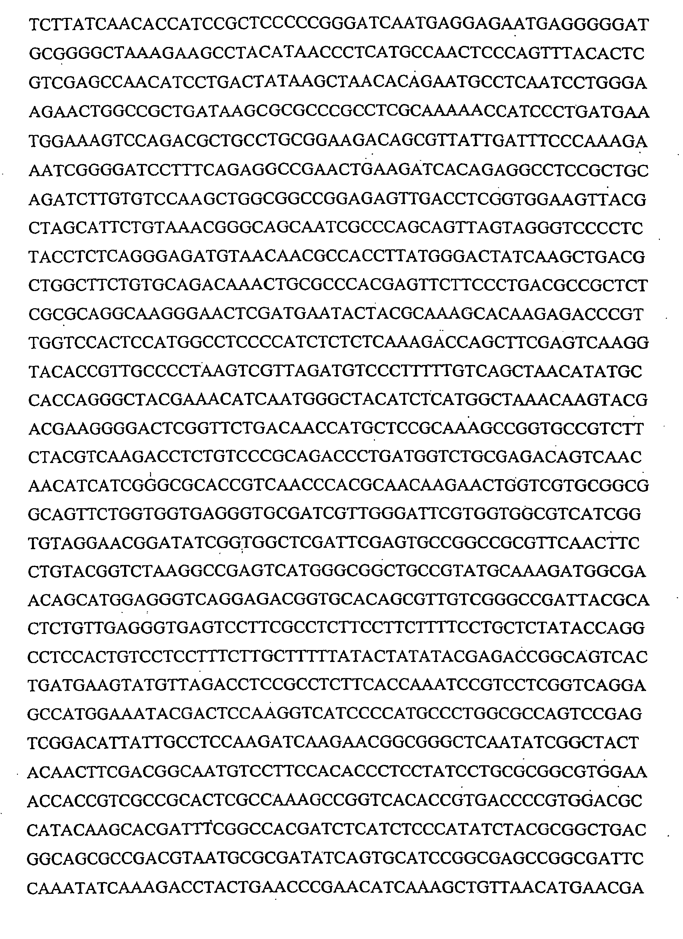

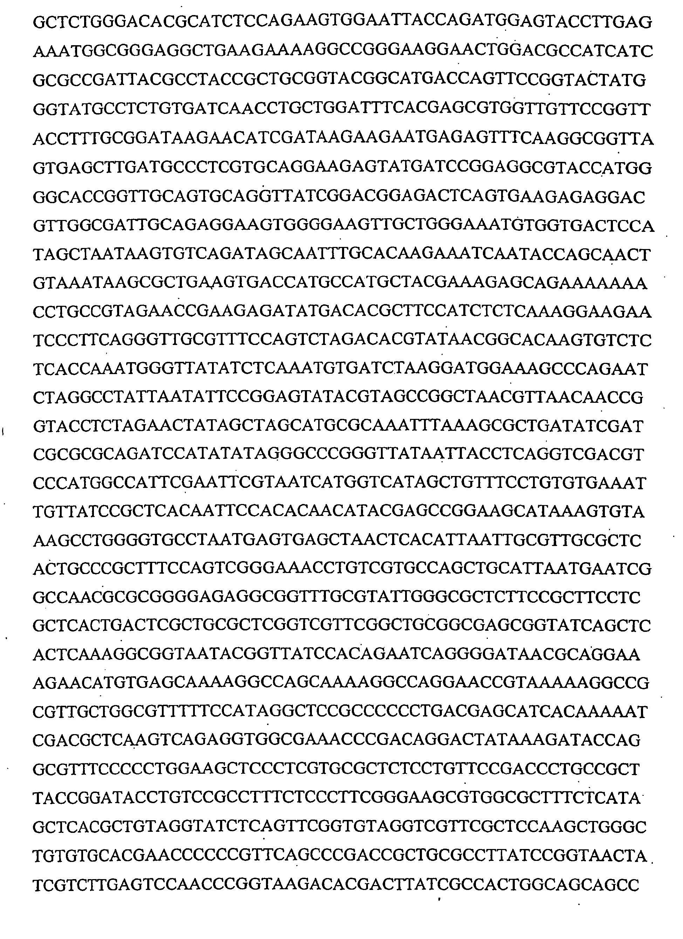

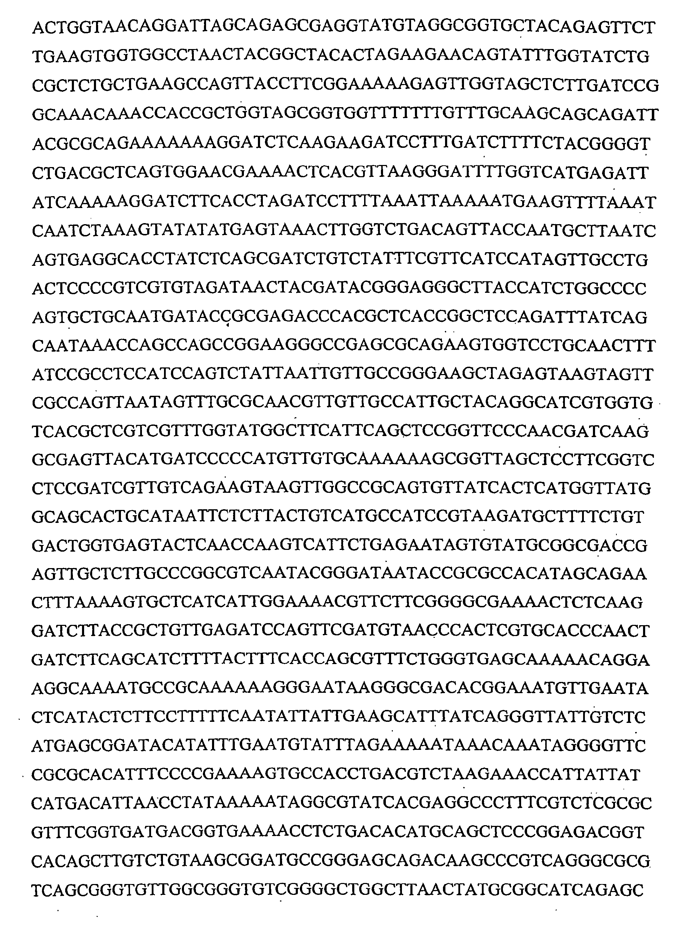

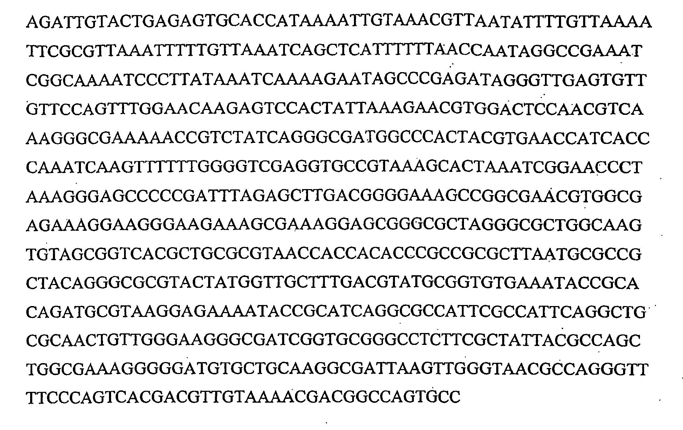

Any vector may be used as long as it is replicable and viable in the cells into which it is introduced. Large numbers of suitable cloning and expression vectors are described in Sambrook *et al.,* 1989, Ausubel FM *et al.,* 1993, and Strathern *et al.,* 1981, each of which is expressly incorporated by reference herein. Further appropriate expression vectors for fungi are described in van den Hondel, C.A.M.J.J. et al. (1991) In: Bennett, J.W. and Lasure, L.L. (eds.) More Gene Manipulations in Fungi. Academic Press, pp. 396-428. The appropriate DNA sequence may be inserted into a vector by a variety of procedures. In general, the DNA sequence is inserted into an appropriate restriction endonuclease site(s) by standard procedures. Such procedures and related sub-cloning procedures are deemed to be within the scope of knowledge of those skilled in the art. Exemplary useful plasmids include pUC18, pBR322, pUC100, pSL1180 (Pharmacia Inc., Piscataway, NJ) and pFB6. Other general purpose vectors such as in *Aspergillus,* pRAX and in *Trichoderma,* pTEX maybe also be used (Figures 16 and 17).

### B. Target Host Cells.

In one embodiment of the present invention, the filamentous fungal parent or host cell may be a cell of a species of, but not limited to, *Trichoderma sp., Penicillium sp., Humicola sp., Chrysosporium sp., Gliocladium sp., Aspergillus sp., Fusarium sp., Neurospora sp., Hypocrea sp., and Emericella sp.* As used herein, the term *"Trichoderma"* or *"Trichoderma sp."* refers to any fungal strains which have previously been classified as *Trichoderma* or are currently classified as *Trichoderma.* Some preferred species for *Trichoderma* fungal parent cells include *Trichoderma longibrachiatum (reesei), Trichoderma viride, Trichoderma koningii,* and *Trichoderma harzianum* cells. Particularly preferred host cells include cells from strains of *T. reesei,* such as RL-P37 (Sheir-Neiss, et al., Appl. Microbiol. Biotechnol. 20:46-53 (1984) and functionally equivalent and derivative strains, such as *Trichoderma reesei* strain RUT-C30 (ATCC No. 56765) and strain QM9414 (ATCC No. 26921). Also reference is made to ATCC No. 13631, ATCC No. 26921, ATCC No. 56764, ATCC No. 56767 and NRRL 1509.

Some preferred species for *Aspergillus* fungal parent cells include *Aspergillus niger, Aspergillus awamori, Aspergillus aculeatus,* and *Aspergillus nidulans* cells. In one embodiment, the strain comprises *Aspergillus niger,* for example A. *niger* var. *awamori* dgr246 (Goedegebuur et al, (2002) Curr. Genet 41: 89-98) and GCDAP3, GCDAP4 and GAP3-4 (Ward, M, et al., (1993), Appl. Microbiol. Biotechnol. 39:738-743).

In some instances it is desired to obtain a filamentous host cell strain such as a *Trichoderma* host cell strain which has had one or more cellulase genes deleted prior to introduction of a heterologous cellulase fusion construct encompassed by the invention. Such strains may be prepared by the method disclosed in U.S. Patent No. 5,246,853, U.S. Patent 5,861,271 and WO 92/06209, which disclosures are hereby incorporated by reference. By expressing a cellulase fusion protein or components thereof having cellulolytic activity in a host microorganism that is missing one or more cellulase genes, the identification and subsequent purification procedures are simplified. Any gene from *Trichoderma sp.* which has been cloned can be deleted, for example, the *cbh1, cbh2, egl1,* and *egl2* genes as well as those encoding EG3 and/or EG5 protein (*see e.g.,* U.S. Patent No. 5,475,101 and WO 94/28117, respectively). Gene deletion may be accomplished by inserting a form of the desired gene to be deleted or disrupted into a plasmid by methods known in the art.

Parental fungal cell lines are generally cultured under standard conditions with media containing physiological salts and nutrients, such as described by Pourquie, J. et al., BIOCHEMISTRY AND GENETICS OF CELLULOSE DEGRADATION, eds. Aubert J.P. et al., Academic Press pp. 71-86 (1988) and limen, M. et al., Appl. Environ. Microbiol. 63:1298 -1306 (1997). Also reference is made to common commercially prepared media such as yeast Malt Extract (YM) broth, Luria Bertani (LB) broth and Sabouraud Dextrose (SD) broth.

### C. Introduction of a Heterologous Cellulase Fusion Construct or Vector into Fungal Host Cells and Culture Conditions.

A host fungal cell may be genetically modified (*i.e*., transduced, transformed or transfected) with a heterologous cellulase fusion construct according to the invention, a cloning vector or an expression vector comprising a heterologous cellulase fusion construct. The methods of transformation of the present invention may result in the stable integration of all or part of the construct or vector into the genome of the filamentous fungus. However, transformation resulting in the maintenance of a self-replicating extra-chromosomal transformation vector is also contemplated.

Many standard transformation methods can be used to produce a filamentous fungal cell line such as a *Trichoderma* or *Aspergillus* cell line that express large quantities of a heterologous protein. Some of the published methods for the introduction of DNA constructs into cellulase-producing strains of *Trichoderma* include Lorito, Hayes, DiPietro and Harman (1993) Curr. Genet. 24: 349-356; Goldman, VanMontagu and Herrera-Estrella (1990) Curr. Genet. 17:169-174; Penttila, Nevalainen, Ratto, Salminen and Knowles (1987) Gene 61: 155-164, EP-A-0 244 234 and also Hazell B. et al., 2000; for *Aspergillus* include Yelton, Hamer and Timberlake (1984) Proc. Natl. Acad. Sci. USA 81: 1470-1474; for *Fusarium* include Bajar, Podila and Kolattukudy, (1991) Proc. Natl. Acad. Sci. USA 88: 8202-8212; for *Streptomyces* include Hopwood et al., (1985) Genetic manipulation of streptomyces: a laboratory manual, The John Innes Foundation, Norwich, UK; and for *Bacillus* include Brigidi, DeRossi, Bertarini, Riccardi and Matteuzzi, (1990), FEMS Microbiol. Lett. 55: 135-138.

Other methods for introducing a heterologous cellulase fusion construct or vector into filamentous fungi (*e.g., H. jecorina)* include, but are not limited to the use of a particle or gene gun (biolistics), permeabilization of filamentous fungi cells walls prior to the transformation process (*e.g*., by use of high concentrations of alkali, *e.g*., 0.05 M to 0.4 M CaCl₂ or lithium acetate), protoplast fusion, electroporation, or agrobacterium mediated transformation (US Patent 6,255,115).

An exemplary method for transformation of filamentous fungi by treatment of protoplasts or spheroplasts with polyethylene glycol and CaCl₂ is described in Campbell, et al., (1989) Curr. Genet. 16:53-56, 1989 and Penttila, M. et al., (1988) Gene, 63:11-22 and Penttila, M. et al., (1987) Gene, 61:155-164.

Any of the well-known procedures for introducing foreign nucleotide sequences into host cells may be used. It is only necessary that the particular genetic engineering procedure used be capable of successfully introducing at least one gene into the host cell capable of expressing the heterologous gene.

The invention includes the transformants of filamentous fungi especially *Trichoderma* cells comprising the coding sequences for the cellulase fusion protein. The invention further includes the filamentous fungi transformants for use in producing fungal cellulase compositions, which include the cellulase fusion protein or components thereof.

Following introduction of a heterologous cellulase fusion construct comprising the exoglucanase catalytic domain coding sequence and the endoglucanase catalytic domain coding sequence, the genetically modified cells can be cultured in conventional nutrient media as described above for growth of target host cells and modified as appropriate for activating promoters and selecting transformants. The culture conditions, such as temperature, pH and the like, are those previously used for the host cell selected for expression, and will be apparent to those skilled in the art. Also preferred culture conditions for a given filamentous fungus may be found in the scientific literature and/or from the source of the fungi such as the American Type Culture Collection (ATCC; "www.atcc.org/").

Stable transformants of filamentous fungi can generally be distinguished from unstable transformants by their faster growth rate and the formation of circular colonies with a smooth rather than ragged outline on solid culture medium. Additionally, in some cases, a further test of stability can be made by growing the transformants on solid non-selective medium, harvesting the spores from this culture medium and determining the percentage of these spores which will subsequently germinate and grow on selective medium.

The progeny of cells into which such heterologous cellulase fusion constructs, or vectors including the same, have been introduced are generally considered to comprise the fusion protein encoded by the nucleic acid sequence found in the heterologous cellulase fusion construct.

In one exemplary application of the invention encompassed herein a recombinant strain of filamentous fungi, *e.g., Trichoderma reesei,* comprising a heterologous cellulase fusion construct will produce not only a cellulase fusion protein but also will produce components of the cellulase fusion protein. In some embodiments the recombinant cells including the cellulase fusion construct will produce an increased amount of cellulolytic activity compared to a corresponding recombinant filamentous fungi strain grown under essentially the same conditions but genetically modified to include separate heterologous nucleic acid constructs encoding an exo-cellobiohydrolase catalytic domain and/or a cellulase catalytic domain.

In some embodiments the cellulase fusion protein and components thereof include the *Trichoderma reesei* CBH1 catalytic domain fused to the *Acidothermus cellulolyticus* E1 catalytic domain and the cleaved CBH1 and E1 products.

In some embodiments the cellulase fusion protein and components thereof include the *Trichoderma reesei* CBH1 catalytic domain fused to the *Acidothermus cellulolyticus* GH48 cellulase catalytic domain and the cleaved CBH1 and *Acidothermus cellulolyticus* GH48 products.

In other embodiments the cellulase fusion protein and components thereof include the *T. reesei* CBH1 catalytic domain fused to a *Acidothermus cellulolyticus* GH74 endoglucanase catalytic domain and the cleaved CBH1 and GH74 products.

In other embodiments the cellulase fusion protein and components thereof include the *T. reesei* CBH1 catalytic domain fused to a *Thermobifida fusca* E3 cellulase catalytic domain and the cleaved CBH1 and E3 products.

In other embodiments the cellulase fusion protein and components thereof include the *T. reesei* CBH1 catalytic domain fused to a *Thermobifida fusca* E5 endoglucanase catalytic domain and the cleaved CBH1 and E5 products.

### D. Analysis of Protein Expression

In order to evaluate the expression of a cellulase fusion protein of the invention by a cell line that has been transformed with a heterologous cellulase fusion construct, assays can be carried out at the protein level, the RNA level or by use of functional bioassays particular to exo-cellobiohydrolase activity or endoglucanase activity and/or production.

In general, the following assays can be used to determine integration of cellulase fusion protein expression constructs and vector sequences, Northern blotting, dot blotting (DNA or RNA analysis), RT-PCR (reverse transcriptase polymerase chain reaction), *in situ* hybridization, using an appropriately labeled probe (based on the nucleic acid coding sequence), conventional Southern blotting and autoradiography.

In addition, the production and/or expression of a cellulase enzyme may be measured in a sample directly, for example, by assays for cellobiohydrolase or endoglucanase activity, expression and/or production. Such assays are described, for example, in Becker et al., Biochem J. (2001) 356:19-30; Mitsuishi et al., FEBS (1990) 275:135-138. Shoemaker et al. 1978; and Schulein 1988) each of which is expressly incorporated by reference herein. The ability of CBH1 to hydrolyze isolated soluble and insoluble substrates can be measured using assays described in Srisodsuk et al., J. Biotech. (1997) 57:49-57 and Nidetzky and Claeyssens Biotech. Bioeng. (1994) 44:961-966. Substrates useful for assaying exo-cellobiohydrolase, endoglucanase or β-glucosidase activities include crystalline cellulose, filter paper, phosphoric acid swollen cellulose, cellooligosaccharides, methylumbelliferyl lactoside, methylumbelliferyl cellobioside, orthonitrophenyl lactoside, paranitrophenyl lactoside, orthonitrophenyl cellobioside, paranitrophenyl cellobioside.

In addition, protein expression, may be evaluated by immunological methods, such as immunohistochemical staining of cells, tissue sections or immunoassay of tissue culture medium, *e.g*., by Western blot or ELISA. Such immunoassays can be used to qualitatively and quantitatively evaluate expression of a cellulase, for example CBH. The details of such methods are known to those of skill in the art and many reagents for practicing such methods are commercially available.

In an embodiment of the invention, the cellulase fusion protein which is expressed by the recombinant host cell will be about 0.1 to 80% of the total expressed cellulase. In other embodiments, the amount of expressed fusion protein will be in the range of about 0.1 mg to 100g; about 0.1 mg to 50 g and 0.1 mg to 10g protein per liter of culture media.

### E. Recovery And Purification Of Cellulase Fusion Proteins and Components Thereof.

In general, a cellulase fusion protein or components of the cellulase fusion protein produced in cell culture are secreted into the medium and may be recovered and optionally purified, *e.g*., by removing unwanted components from the cell culture medium. However, in some cases, a cellulase fusion protein or components thereof may be produced in a cellular form necessitating recovery from a cell lysate. In such cases the protein is purified from the cells in which it was produced using techniques routinely employed by those of skill in the art. Examples include, but are not limited to, affinity chromatography (van Tilbeurgh et al., FEBS Lett. 16:215, 1984), ion-exchange chromatographic methods (Goyal et al., Bioresource Technol. 36:37-50, 1991; Fliess et al., Eur. J. Appl. Microbiol. Biotechnol. 17:314-318, 1983; Bhikhabhai et al., J. Appl. Biochem. 6:336-345, 1984; Ellouz et al., J. Chromatography 396:307-317, 1987), including ion-exchange using materials with high resolution power (Medve et al., J. Chromatography A 808:153-165, 1998), hydrophobic interaction chromatography (Tomaz and Queiroz, J. Chromatography A 865:123-128, 1999), and two-phase partitioning (Brumbauer, et al., Bioseparation 7:287-295, 1999).

Once expression of a given cellulase fusion protein or components thereof is achieved, the proteins thereby produced may be purified from the cells or cell culture by methods known in the art and reference is made to Deutscher, Methods in Enzymology, vol. 182, no. 57, pp. 779, 1990; and Scopes, Methods Enzymol. 90: 479-91, 1982. Exemplary procedures suitable for such purification include the following: antibody-affinity column chromatography, ion exchange chromatography; ethanol precipitation; reverse phase HPLC; chromatography on silica or on a cation-exchange resin such as DEAE; chromatofocusing; SDS-PAGE; ammonium sulfate precipitation; and gel filtration using, *e.g*., Sephadex G-75.

A purified form of a cellulase fusion protein or components thereof may be used to produce either monoclonal or polyclonal antibodies specific to the expressed protein for use in various immunoassays. (See, *e.g*., Hu et al., Mol Cell Biol. vol.11, no. 11, pp. 5792-5799, 1991). Exemplary assays include ELISA, competitive immunoassays, radioimmunoassays, Western blot, indirect immunofluorescent assays and the like.

### F. Utility of enzymatic compositions comprising the cellulase fusion proteins or components thereof.

**The cellulase fusion protein and** components comprising the catalytic domains of the cellulase fusion protein find utility in a wide variety applications, including use in detergent compositions, stonewashing compositions, in compositions for degrading wood pulp into sugars (*e.g*., for bio-ethanol production), and/or in feed compositions. In some embodiments, the cellulase fusion protein or components thereof may be used as cell free extracts. In other embodiments the fungal cells expressing a heterologous cellulase fusion construct are grown under batch or continuous fermentation conditions. A classical batch fermentation is a closed system, wherein the composition of the medium is set at the beginning of the fermentation and is not subject to artificial alterations during the fermentation. Thus, at the beginning of the fermentation the medium is inoculated with the desired organism(s). In this method, fermentation is permitted to occur without the addition of any components to the system. Typically, a batch fermentation qualifies as a "batch" with respect to the addition of the carbon source and attempts are often made at controlling factors such as pH and oxygen concentration. The metabolite and biomass compositions of the batch system change constantly up to the time the fermentation is stopped. Within batch cultures, cells progress through a static lag phase to a high growth log phase and finally to a stationary phase where growth rate is diminished or halted. If untreated, cells in the stationary phase eventually die. In general, cells in log phase are responsible for the bulk of production of end product.

A variation on the standard batch system is the "fed-batch fermentation" system, which also finds use with the present invention. In this variation of a typical batch system, the substrate is added in increments as the fermentation progresses. Fed-batch systems are useful when catabolite repression is apt to inhibit the production of products and where it is desirable to have limited amounts of substrate in the medium. Measurement of the actual substrate concentration in fed-batch systems is difficult and is therefore estimated on the basis of the changes of measurable factors such as pH, dissolved oxygen and the partial pressure of waste gases such as CO₂. Batch and fed-batch fermentations are common and well known in the art.

Continuous fermentation is an open system where a defined fermentation medium is added continuously to a bioreactor and an equal amount of conditioned medium is removed simultaneously for processing. Continuous fermentation generally maintains the cultures at a constant high density where cells are primarily in log phase growth.

Continuous fermentation allows for the modulation of one factor or any number of factors that affect cell growth and/or end product concentration. For example, in one embodiment, a limiting nutrient such as the carbon source or nitrogen source is maintained at a fixed rate an all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off must be balanced against the cell growth rate in the fermentation. Methods of modulating nutrients and growth factors for continuous fermentation processes as well as techniques for maximizing the rate of product formation are well known in the art of industrial microbiology.

In some applications, the cellulase fusion protein and components thereof find utility in detergent compositions, stonewashing compositions or in the treatment of fabrics to improve their feel and appearance. A detergent composition refers to a mixture which is intended for use in a wash medium for the laundering of soiled cellulose containing fabrics. A stonewashing composition refers to a formulation for use in stonewashing cellulose containing fabrics. Stonewashing compositions are used to modify cellulose containing fabrics prior to sale, i.e., during the manufacturing process. In contrast, detergent compositions are intended for the cleaning of soiled garments and are not used during the manufacturing process.

In the context of the present invention, such compositions may also include, in addition to cellulases, surfactants, additional hydrolytic enzymes, builders, bleaching agents, bleach activators, bluing agents and fluorescent dyes, caking inhibitors, masking agents, cellulase activators, antioxidants, and solubilizers.

Surfactants may comprise anionic, cationic and nonionic surfactants such as those commonly found in detergents. Anionic surfactants include linear or branched alkylbenzenesulfonates; alkyl or alkenyl ether sulfates having linear or branched alkyl groups or alkenyl groups; alkyl or alkenyl sulfates; olefinsulfonates; and alkanesulfonates. Ampholytic surfactants include quaternary ammonium salt sulfonates, and betaine-type ampholytic surfactants. Such ampholytic surfactants have both the positive and negative charged groups in the same molecule. Nonionic surfactants may comprise polyoxyalkylene ethers, as well as higher fatty acid alkanolamides or alkylene oxide adduct thereof, fatty acid glycerine monoesters, and the like.

Cellulose containing fabric may be any sewn or unsewn fabrics, yarns or fibers made of cotton or non-cotton containing cellulose or cotton or non-cotton containing cellulose blends including natural cellulosics and manmade cellulosics (such as jute, flax, ramie, rayon, and lyocell). Cotton-containing fabrics are sewn or unsewn fabrics, yarns or fibers made of pure cotton or cotton blends including cotton woven fabrics, cotton knits, cotton denims, cotton yarns, raw cotton and the like.

Preferably the cellulase compositions comprising the cellulase fusion protein or components thereof are employed from about 0.00005 weight percent to about 5 weight percent relative to the total detergent composition. More preferably, the cellulase compositions are employed from about 0.0002 weight percent to about 2 weight percent relative to the total detergent composition.

Since the rate of hydrolysis of cellulosic products may be increased by using a transformant having a heterologous cellulase fusion construct inserted into the genome, products that contain cellulose or heteroglycans can be degraded at a faster rate and to a greater extent. Products made from cellulose such as paper, cotton, cellulosic diapers and the like can be degraded more efficiently in a landfill. Thus, the fermentation product obtainable from the transformants or the transformants alone may be used in compositions to help degrade by liquefaction a variety of cellulose products that add to the overcrowded landfills.

Cellulose-based feedstocks are comprised of agricultural wastes, grasses and woods and other low-value biomass such as municipal waste (e.g., recycled paper, yard clippings, etc.). Ethanol may be produced from the fermentation of any of these cellulosic feedstocks. However, the cellulose must first be converted to sugars before there can be conversion to ethanol. A composition containing an enhanced amount of cellulolytic activity due to the inclusion of a cellulase fusion protein or components thereof may find utility in ethanol production

Ethanol can be produced via saccharification and fermentation processes from cellulosic biomass such as trees, herbaceous plants, municipal solid waste and agricultural and forestry residues. However, the ratio of individual cellulase enzymes within a naturally occurring cellulase mixture produced by a microbe may not be the most efficient for rapid conversion of cellulose in biomass to glucose. It is known that endoglucanases act to produce new cellulose chain ends which themselves are substrates for the action of cellobiohydrolases and thereby improve the efficiency of hydrolysis of the entire cellulase system. Therefore, the use of increased or optimized endoglucanase activity from a cellulase fusion protein or components thereof may greatly enhance the production of ethanol and sugar which can be converted by fermentation to other chemicals.

Thus, the inventive cellulase fusion protein and components thereof find use in the hydrolysis of cellulose to its sugar components. In one embodiment, the cellulase fusion protein or components thereof are added to the biomass prior to the addition of a fermentative organism. In another embodiment, the cellulase fusion protein or components thereof are added to the biomass at the same time as a fermentative organism. Optionally, there may be other cellulase components present in either embodiment.

### EXPERIMENTAL

The present invention is described in further detail in the following examples which are not in any way intended to limit the scope of the invention.

In the disclosure and experimental section, which follows, the following abbreviations apply:
CBH1-E1 (*T. reesei* CBH1 catalytic domain and linker fused to an *Acidothermus cellulolyticus* GH5A endoglucanase I catalytic domain);
CBH1-48E *(T. reesei* CBH1 catalytic domain and linker fused to an *Acidothermus cellulolyticus* GH48 cellulase catalytic domain);
CBH1-74E (*T. reesei* CBH1 catalytic domain and linker fused to an *Acidothermus cellulolyticus* GH74 endoglucanase catalytic domain);
CBH1-TfE3 (*T. reesei* CBH1 catalytic domain and linker fused to the CBD, linker and catalytic domain of *Thermobifida fusca* E3 cellulase; and
CBH1-TfE5 (*T. reesei* CBH1 catalytic domain and linker fused to the CBD, linker and catalytic domain of *Thermobifida fusca* E5 endoglucanase;
wt% (weight percent); °C (degrees Centigrade); rpm (revolutions per minute); H₂O (water); dH₂O (deionized water); aa (amino acid); bp (base pair); kb (kilobase pair); kD (kilodaltons); g (grams); µg (micrograms); mg (milligrams); µL (microliters); ml and mL (milliliters); mm (millimeters); µm (micrometer); M (molar); mM (millimolar); µM (micromolar); U (units); MW (molecular weight); sec (seconds); min(s) (minute/minutes); hr(s) (hour/hours); PAGE (polyacrylamide gel electrophoresis); phthalate buffer, (sodium phthalate in water, 20mN, pH 5.0); PBS (phosphate buffered saline [150 mM NaCl, 10 mM sodium phosphate buffer, pH 7.2]);SDS (sodium dodecyl sulfate); Tris (tris(hydroxymethyl)aminomethane); w/v (weight to volume); w/w (weight ' to weight); v/v (volume to volume); and Genencor (Genencor International, Inc., Palo Alto, CA).

### EXAMPLE 1

### Construction of a CBH1-E1 Fusion Vector

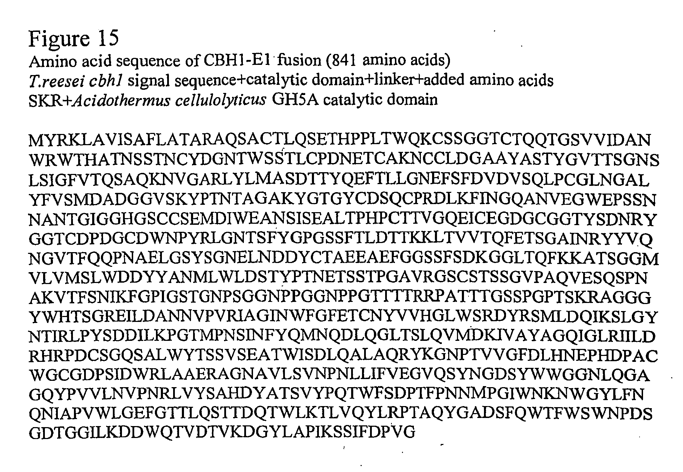

The CBH1-E1 fusion construct included the *T. reesei cbhl* promoter; the *T. reesei cbhl* gene sequence from the start codon to the end of the *cbh*l linker and an additional 12 bases of DNA 5' to the start of the endoglucanase coding sequence, a stop codon and the *T. reesei cbh1* terminator (see Figures 14 and 15). The additional 12 DNA bases (ACTAGTAAGCGG) (SEQ ID NO. 20) code for the restriction endonuclease Spel and the amino acids Ser, Lys, and Arg.

The plasmid E1-pUC19 which contained the open reading frame for the E1 gene locus was used as the DNA template in a PCR reaction. Equivalent plasmids are described in USP 5,536,655 which also describes the cloning of the E1 gene from the actinomycete *Acidothermus cellulolyticus,* ATCC 43068, Mohagheghi A. et al., 1986. Standard procedures for working with plamsid DNA and amplification of DNA using PCT were employed (Sambrook et al., 1989 and 2001).

The following two primers were used to amplify the coding region of the catalytic domain of the E1 endoglucanase.
Forward Primer 1 = EL-316 (containing a SpeI site):
GCTTATACTAGTAAGCGCGCGGGCGGCGGCTATTGGCACAC (SEQ ID NO: 21)
Reverse Primer 2 = EL-317 (containing an AscI site and **stop codon reverse compliment**):
GCTTATGGCGCGCCTTAGACAGGATCGAAAATCGACGAC (SEQ ID NO: 22).

The reaction conditions were as follow using materials from the PLATINUM Pfx DNA Polymerase kit (Invitrogen, Carlsbad, CA): 1 µl dNTP Master Mix (final concentration 0.2mM); 1 µl primer 1 (final conc 0.5 µM); 1 µl primer 2 (final conc 0.5 µM); 2µl DNA template (final conc 50 - 200 ng); 1 µl 50mM MgSO₄ (final conc 1mM); 5µl 10 x Pfx Amplification Buffer; 5µl 10 x PCRx Enhancer Solution; 1 µl Platinum Pfx DNA Polymerase (2.5U total); 33 µl water, for 50 µl total reaction volume.

Amplification parameters were: step 1 - 94°C for 2 min (1st cycle only to denature antibody bound polymerase); step 2 - 94°C for 45 sec; step 3 - 60°C for 30 sec; step 4 - 68°C for 2 min; step 5 - repeated step 2 for 24 cycles and step 6 - 68°C for 4 min.

The appropriately sized PCR product was cloned into the Zero Blunt TOPO vector and transformed into chemically competent Top10 *E. coli* cells (Invitrogen Corp., Carlsbad, CA) plated onto appropriate selection media (LA with 50 ppm kanamycin and grown overnight at 37°C. Several colonies were picked from the plate media and grown in 5 ml cultures at 37°C in selection media (LB with 50 ppm kanamycin) from which plasmid mini-preps were made.

Plasmid DNA from several clones was restriction digested to confirm the correct size insert. The correct sequence was confirmed by DNA sequencing. Following sequence verification, the E1 catalytic domain was excised from the TOPO vector by digesting with the restriction enzymes Spel and AscI. This fragment was ligated into the pTrex4 vector which had been digested with the restriction enzymes Spel and AscI (see Figures 16 and 17).

The ligation mixture was transformed into MM294 competent E. coli cells, plated onto appropriate selection media (LA with 50 ppm carbenicillin) and grown overnight at 37°C. Several colonies were picked from the plate media and grown overnight in 5 ml cultures at 37°C in selection media (LB with 50 ppm carbenicillin) from which plasmid mini-preps were made. Correctly ligated CBH1-E1 fusion vectors were confirmed by restriction digestion.

### EXAMPLE 2

### Transformation and Expression the CBH1-E1 Fusion Construct into a T. reesei host strain.

Various T. *reesei* strains were transformed with the CBH1-E1 fusion construct. The host strains included a derivative of T. *reesei* RL-P37 and a derivative of T. *reesei* wherein the native cellulase genes (*cbh*1, *cbh*2, *egl*1, and *egl*2) were deleted.

Approximately one-half swab (or 1 -2 cm²) of a plate of a sporulated T. *reesei* derivative of strain RL-P37 (Sheir-Neiss, *et al.,* 1984) mycelia (grown on a PDA plate for 7 days at 28°C) was inoculated into 50ml of YEG (5g/L yeast extract plus 20g/L glucose) broth in a 250 ml, 4-baffled shake flask and incubated at 30°C for 16-20 hours at 200 rpm.

The mycelia was recovered by transferring the liquid volume into 50ml conical tubes and spinning at 2500 rpm for 10 minutes. The supernatant was aspirated off. The mycelial pellet was transferred into a 250 ml, CA Corning bottle containing 40ml of B glucanase solution and incubated at 30°C, 200 rpm for 2 hrs to generate protoplasts for transformation. Protoplasts were harvested by filtration through sterile miracloth into a 50ml conical tube. They were pelleted by spinning at 2000 rpm for 5 minutes, and the supernatent was aspirated off. The protoplast pellet was washed once with 50ml of 1.2 M sorbitol, spun down, aspirated, and washed with 25ml of sorbitolCaCl₂. Protoplasts were counted and then pelleted again at 2000 rpm for 5 min, the supernatent was aspirated off, and the protoplast pellet was resuspended in a sufficient volume of sorbitol CaCl₂ to generate a protoplast concentration of 1.25 x 10⁸ protoplasts per ml constituting the protoplast solution.

Aliquots of up to 20 µg of expression vector DNA (in a volume no greater than 20 µl) were placed into 15ml conical tubes and the tubes were put on ice. Then 200µl of the protoplast solution was added, followed by 50µl PEG solution to each transformation aliquot. The tubes were mixed gently and incubated on ice for 20 min. Next an additional 2 ml of PEG solution was added to the transformation aliquot tubes, followed by gentle inversion and incubation at room temperature for 5 minutes. Next 4 ml of Sorbitol/CaCl₂ solution was added to the tubes (generating a total volume of 6.2 ml). This transformation mixture was divided into 3 aliquots each containing about 2ml. An overlay mixture was created by adding each of these three aliquots to three tubes containing 10 ml of melted acetamide/sorbitol top agar (kept molten by holding at 50°C) and this overlay mixture was poured onto a selection plate of acetamide/sorbitol agar. The transformation plates were then incubated at 30°C for four to seven days.

The transformation was performed with *amdS* selection. Acetamide/sorbitol plates and overlays were used for the transformation. For the selection plates, the same plates were used, but without sorbitol. Transformants were purified by transfer of isolated colonies to fresh selective media containing acetamide.

With reference to the examples the following solutions were made as follows.
1) 40 ml β-D-glucanase solution was made up in 1.2M sorbitol and included 600mg β-D-glucanase and 400mg MgSO₄ ·7H₂O (Catalog No. 0439-1, InterSpex Products Inc., San Mateo, CA).
2) 200 ml PEG solution contained 50g polyethylene glycol 4000 (BDH Laboratory Supplies Poole, England) and 1.47g CaCl₂ ·2H₂0 made up in dH₂O.
3) Sorbitol/ CaCl₂ contained 1.2M sorbitol and 50mM CaCl₂.
4) Acetamide/sorbitol agar:
   Part 1 - 0.6g acetamide (Aldrich, 99% sublime.), 1.68g CsCl, 20g glucose, 20g KH₂PO₄, 0.6g MgSO₄ ·7H₂O, 0.6g CaCl₂ ·2H₂O, 1 ml 1000 x salts (see below), adjusted to pH 5.5, brought to volume (300 mls) with dH₂O, filtered and sterilized.
   Part II - 20g Noble agar and 218g sorbitol brought to volume (700mls) with dH₂O and autoclaved.
   Part II was added to part I for a final volume of 1 L.
5) 1000 x Salts - 5g FeSO₄ ·7H₂O, 1.6g MnSO₄ ·H₂O, 1.4g ZnSO₄ ·7H₂O, 1g CoCl₂ ·6H₂O were combined and the volume was brought to 1 L with dH₂O. The solution was filtered and sterilized.
6) Acetamide/sorbitol top agar is prepared as is acetamide/sorbitol agar except that top agar is substituted for noble agar.
This procedure was similar to that described in Penttila et al., Gene 61: 155 -164, 1987. Individual fungal transformabts were grown up in shake flask culture to determine the level of fusion protein expression. The experiments were conducted essentially as described in example 1 of U. S. Patent 5,874,276 with the following modification: 16 g/L of alpha-lactose was substituted for cellulose in TSF medium. The highest level of cleaved E1 protein expression from a transformant in shake flasks was estimated to be greater than 3 g/L.

In general, the fermentation protocol as described in Foreman *et al.* (Foreman et al. (2003) J. Biol. Chem 278:31988-31997) was followed. Vogels minimal medium (Davis et al., (1970) Methods in Enzymology 17A, pg 79 - 143 and Davis, Rowland, NEUROSPORA, CONTRIBUTIONS OF A MODEL ORGANISM, Oxford University Press, (2000)) containing 5% glucose was inoculated with 1.5 ml frozen spore suspension. After 48 hours, each culture was transferred to 6.2L of the same medium in a 14L Biolafitte fermenter. The fermenter was run at 25°C, 750 RPM and 8 standard liters per minute airflow. One hour after the initial glucose was exhausted, a 25% (w/w) lactose feed was started and fed in a carbon limiting fashion to prevent lactose accumulation. The concentrations of glucose and lactose were monitored using a glucose oxidase assay kit or a glucose hexokinase assay kit with beta-galactosidase added to cleave lactose, respectively (Instrumentation Laboratory Co., Lexington, MA). Samples were obtained at regular intervals to monitor the progress of the fermentation. Collected samples were spun in a 50ml centrifuge tube at 3/4 speed in an International Equipment Company (Needham Heights, MA) clinical centrifuge.

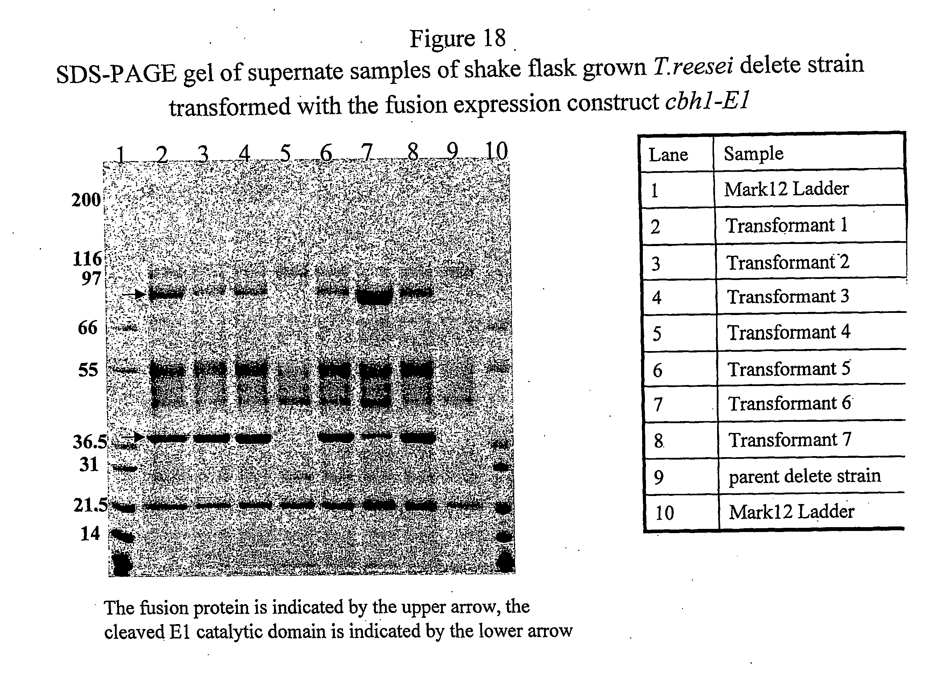

Shake flask grown supernatant samples were run on BIS-TRIS SDS -PAGE gels (Invitrogen), under reducing conditions with MOPS (morpholinepropanesulfonic acid) SDS running buffer and LDS sample buffer. The results are provided in Figure 18.

### EXAMPLE 3

### Assay of Cellulolytic Activity from Transformed Trichoderma reesei Clones

The following assays and substrates were used to determine the cellulolytic activity of the CBH1-E1 fusion protein.
Pretreated corn stover (PCS) - Corn stover was pretreated with 2% w/w H₂SO₄ as described in Schell, D. et al., J. Appl. Biochem. Biotechnol. 105:69 - 86 (2003) and followed by multiple washes with deionized water to obtain a pH of 4.5. Sodium acetate was added to make a final concentration of 50mM and this was titrated to pH 5.0.
Measurement of Total Protein - Protein concentration was measured using the bicinchoninic acid method with bovine serum albumin as a standard (Smith P. K. et al., Biochem. 150:76-85, 1985).
Cellulose conversion (Soluble sugar determinations) was evaluated by HPLC according to the methods described in Baker et al., Appl. Biochem. Biotechnol. 70-72:395 - 403 (1998).

A standard cellulosic conversion assay was used in the experiments. In this assay enzyme and buffered substrate were placed in containers and incubated at a temperature over time. The reaction was quenched with enough 100 mM Glycine, pH 11.0 to bring the pH of the reaction mixture to at least pH10. Once the reaction was quenched, an aliquot of the reaction mixture was filtered through a 0.2 micron membrane to remove solids. The filtered solution was then assayed for soluble sugars by HPLC as described above. The cellulose concentration in the reaction mixture was approximately 7%. The enzyme or enzyme mixtures were dosed anywhere from 1 to 60 mg of total protein per gram of cellulose.

In one set of experiments the percent conversion of 13.8% PCS (7.06% cellulose) at 55°C for 1 day was evaluated using 10 mg enzyme/g cellulose in 50 mM acetate buffer at 55°C. Samples were agitated at 700 rpm. Comparisons were made between supernatants from growth of 1) a T. reesei parent strain which included the native cellulase genes and 2) a corresponding T. *reesei* CBH1-E1 fusion strain transformed according to the examples herein. Samples were quenched at various times up to 24 hours.

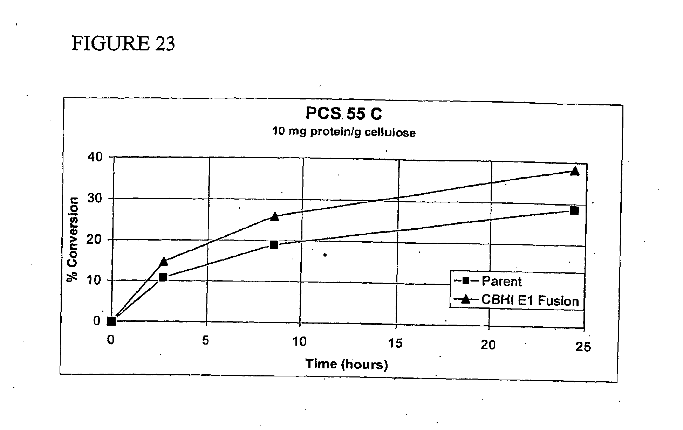

The results are presented in Figure 23 and it is observed that the CBH1-E1 fusion protein outperforms the parent. It took about 6 hours for the CBH1-E1 fusion protein to yield 20% cellulose conversion, while it requires 10 hours for the parent cellulase to reach 20% hydrolysis.

### EXAMPLE 4

### Transformation and Expression the CBH1-48E Fusion Construct into T. reesei

The CBH1-48E fusion construct was designed according to the procedures described below in example 1 with the following differences. The forward primer was designed to maintain the reading frame translation and include a Lys-Arg kexin cleavage site (underlined) at the end of the *cbh1* linker sequence. The reverse primer contained a stop codon at the end of the GH48E.

Primers were ordered with 5 prime phosphates to enable subsequent blunt cloning. The GH48 catalytic domain was amplified with the following forward and reverse primers:
GH48 forward primer bluntF4 -
   CTAAGAGAAACGACCCGTACATCCAGCGGTTCCTCACGATGTA (SEQ ID NO: 23)
GH48 reverse primer bluntR5 -
   TTACCCGGATGGGAAGAGCATGCCAAAATCGGCGTTCG (SEQ ID NO: 24).

Amplification was performed using Stratagene's Herculase High Fidelity Polymerase (Stratagene, La Jolla, CA). An annealing temperature of 65°C was used. A DNA plasmid encompassing the GH48 catalytic domain was used as the template for PCR (approximately 0.2 ug of DNA). An equivalent method for isolating the GH48 gene is described in U.S. Pat. Appln. No. 2003/0096342.

The amplification reaction set up:

| Component | in µl |
|---|---|
| 10X Herculase Buffer | 5 |
| 10 mM dNTPs | 1.5 |
| H₂O | 39.5 |
| Fwd primer (10 µM) | 1 |
| Rev primer (10 µM) | 1 |
| Template | 1 |
| Herculase Polymerase (5U) | 1 |
| Total reaction volume | 50 |

Cycling:

| Segment | No. of cycles | Temp °C | hr:min:sec |
|---|---|---|---|
| 1 | 1 | 95 | 00:03:00 |
| 2 | 10 | 95 | 00:00:40 |
| | | annealing Temp | 00:00:30 |
| | | 72 | 30 sec. per 500 bp |
| 3 | 20 | 95 | 00:00:40 |
| | | annealing Temp | 00:00:30 |
| | | 72 | 30 sec per 500 bp + 10 sec/cycle |
| 4 | 1 | 4 | hold |

All PCR products were gel purified and treated with Mung Bean Nuclease to produce blunt ends prior to ligation. The amplified, blunted fragment was ligated into pTrex4 that had been digested with the restriction enzymes Spel and AscI followed by nuclease digestion to remove the 3' overhangs and thereby creating blunt ends. The newly created vector was then transformed into E. coli. Plasmid DNA was isolated from the colonies of transformed E. coli. Since the amplified GH48 fragment could insert into pTrex4 in two different orientations, restriction digests were preformed to discern clones with correctly oriented inserts. Putative clones were confirmed by DNA sequencing.

Transformation of the fusion vector was performed using biolistic transformation according to the teaching of Hazell, B. W. et al., Lett. Appl. Microbiol. 30:282-286 (2000).

Expression of the CBH1-48E fusion protein was determined as described above for expression of the CBH1-EI fusion protein in Example 3. The highest level of CBH1-GH48 protein expression from a transformant in shakes was estimated to be about 0.1 g/L.

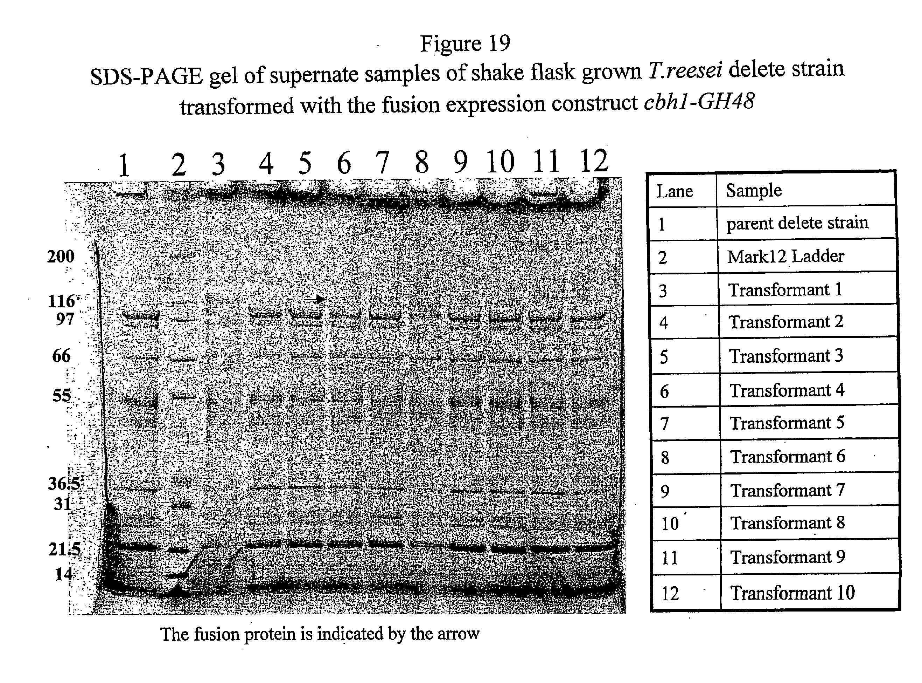

Shake flask grown supernatant samples were run on BIS-TRIS SDS -PAGE gels (Invitrogen), under reducing conditions with MOPS (morpholinepropanesulfonic acid) SDS running buffer and LDS sample buffer. The results are provided in Figure 19.

### EXAMPLE 5

### Transformation and Expression the CBH1-74E Fusion Construct into T. reesei

The CBH1-74E fusion construct was designed according to the procedures described above in example 1 with the following differences. The forward primer was designed to maintain the reading frame translation and include a Lys-Arg kexin cleavage site (underlined). The reverse primer encodes a stop codon (the reverse compliment - underlined) at the end of the catalytic domain.

Primers were ordered with 5 prime phosphates to enable subsequent blunt cloning. The GH74 catalytic domain was amplified with the following forward and reverse primers:
GH74 forward bluntF4 - CTAAGAGAGCGACGACTCAGCCGTACACCTGGAGCAACGTGGC (SEQ ID NO: 25) and
GH74 reverse bluntR4 - TTACGATCCGGACGGCGCACCACCAATGTCCCCGTATA (SEQ ID NO: 26).

The amplification conditions and subsequent cloning are as described in Example 4, but with an annealing temperature of 60°C,

An isolated fragment of DNA encompassing the GH74 catalytic domain was used as the template for PCR (approximately 0.2 ug of DNA). U.S. Pat. Appln. No. 2003/0108988 describes the cloning of GH74. (GH74 is referred to as AviIII in the published patent application.

After amplification, the amplified fragment was blunt end ligated into the pTrex4 vector that had been digested with Spel and AscI followed by nuclease digestion to remove the 3' overhang creating blunt ends. The subsequent vector with the fusion construct was confirmed by sequencing.

Transformation of the fusion vector into *T. reesei* was performed using biolistic transformation according to Hazell B. et al., Lett. Appl. Microbiol. 30:282 - 286 (2000).

Expression of the CBH1-74E fusion protein was determined as described above for expression of the CBH1-E1 fusion protein in Example 2. The highest level of cleaved GH74 protein expression from a transformant in shake flasks was estimated to be greater than 3 g/L.

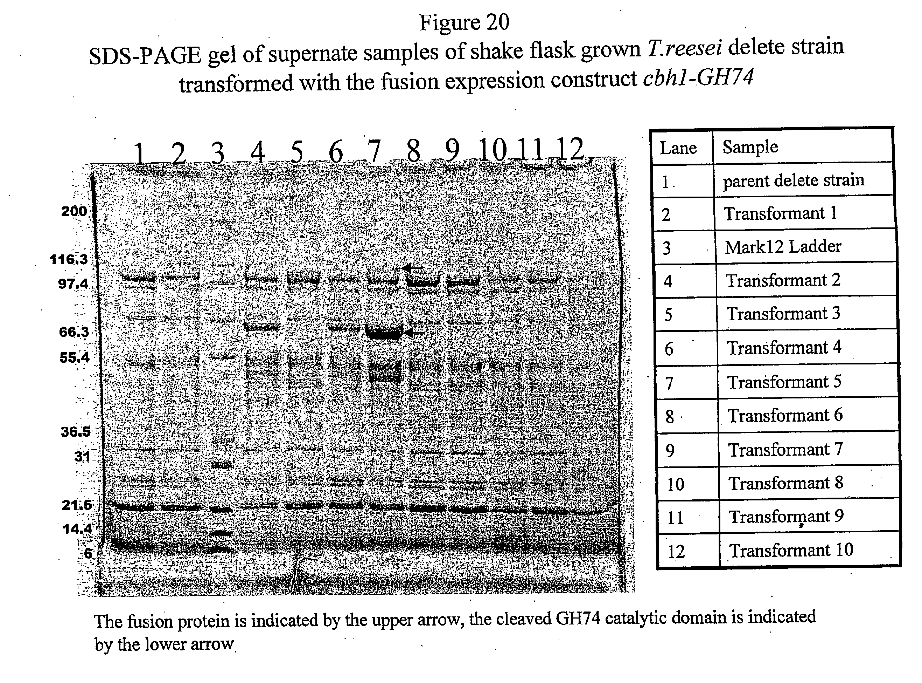

Shake flask grown supernatant samples were run on BIS-TRIS SDS -PAGE gels (Invitrogen), under reducing conditions with MOPS (morpholinepropanesulfonic acid) SDS running buffer and LDS sample buffer. The results are provided in Figure 20.

### EXAMPLE 6

### Transformation and Expression the CBH1-TfE3 Fusion Construct into T. reesei

The CBH1-TfE3 fusion construct was designed according to the procedures described above in Example 1 with the following differences.

The following primers were used to amplify the TfE3 cellulase - EL-310 forward (which contains a Spel site)
GCTTATACTAGTAAGCGCGCCGGCTGCTCGGTGGACTACACG (SEQ ID NO: 27)
and EL-311 reverse (which contains a AscI site) -
GCTTATGGCGCGCCTTACAGAGGCGGGTAGGCGTTGG (SEQ ID NO: 28).

The plasmid containing the TfE3 gene was used as the DNA template for amplification. An equivalent template DNA is described in Zhang, S. et al., Biochem. 34:3386 - 3395, 1995. The amplification conditions and subsequent cloning are as described in example 1. Vector construction and biolistic transformation of T. *reesei* proceeded as described above.

The highest level of expression in shake flasks was estimated to be greater than 0.4 g/L.

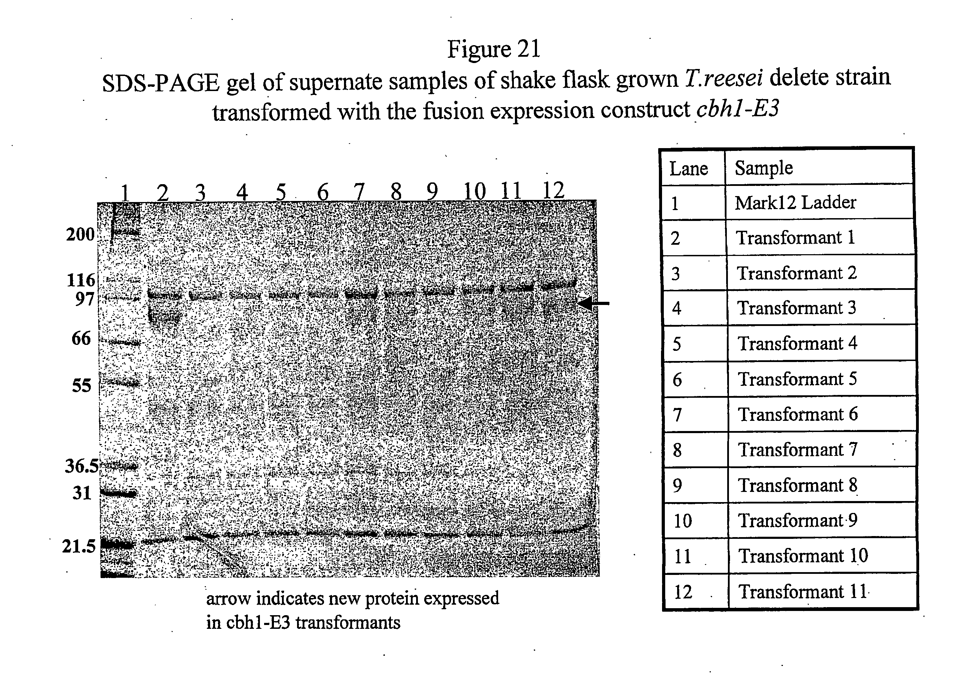

Shake flask grown supernatant samples were run on BIS-TRIS SDS -PAGE gels (Invitrogen), under reducing conditions with MOPS (morpholinepropanesulfonic acid) SDS running buffer and LDS sample buffer. The results are provided in Figure 21.

### EXAMPLE 7

### Transformation and Expression the CBH1-TfE5 Fusion Construct into T. reesei

The CBH1-TfE5 fusion construct was designed according to the procedures described above in Example 1 with the following differences. A plasmid equivalent to that described in Collmer & Wilson, Bio/Technol. 1:594 - 601 (1983) carrying the TfE5 gene was used as the DNA template to amplify the TfE5.

The following primers were used to amplify the TfE5 endoglucanase. EL-308 (which contains a SpeI site) - forward primer
GCTTATACTAGTAAGCGCGCCGGTCTCACCGCCACAGTCACC (SEQ ID NO: 29)
and EL-309 (which contains a AscI site) reverse primer -
GCTTATGGCGCGCCTCAGGACTGGAGCTTGCTCCGC (SEQ ID NO: 30).

Transformation was as described above. The highest level of cleaved TfE5 protein expression from a transformant in shake flasks was estimated to be greater than 2 g/L.

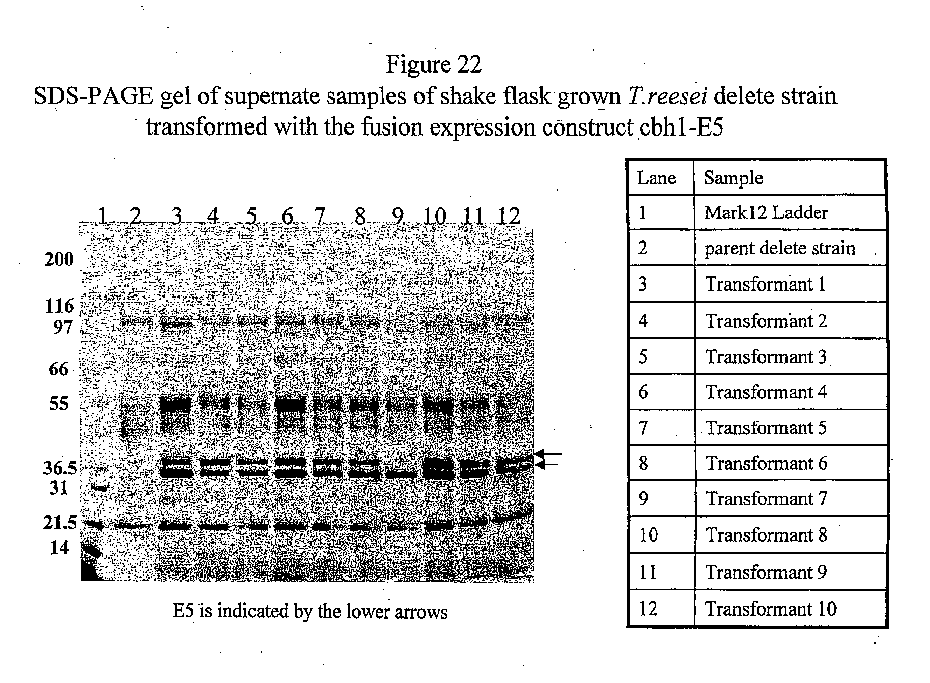

Shake flask grown supernatant samples were run on BIS-TRIS SDS -PAGE gels (Invitrogen), under reducing conditions with MOPS (morpholinepropanesulfonic acid) SDS running buffer and LDS sample buffer. The results are provided in Figure 22.

### REFERENCES

Altschul, S. F., et al., J. Mol. Biol. 215:403-410, 1990.
Altschul, S. F., et al., Nucleic Acids Res. 25:3389-3402, 1997.
Aro N, Saloheimo A, Ilmen M, Penttila M. ACEII, a novel transcriptional activator involved in regulation of cellulase and xylanase genes of Trichoderma reesei. J Biol Chem. 2001 Jun 29;276(26):24309-14. (Epub 2001 Apr 13.)
Aubert J.P. et al, p11 et seq., Biochemistry and Genetics of Cellulose Degradation, eds. Aubert, J. P., Beguin, P., Millet, J., Federation of European Microbiological Societies, Academic Press, 1988.
Ausubel G. M., et al. CURRENT PROTOCOLS IN MOLECULAR BIOLOGY, John Wiley & Sons, New York, N.Y., 1993.
Baker et al., Appl. Biochem. and Biotechnol. 45/46:245 - 256, 1994.
Bhikhabhai, R. et al., J. Appl. Biochem. 6:336, 1984.
Boel et al. EMBO J 3:1581-1585 1984.
Brumbauer, A. et al., Bioseparation 7:287-295, 1999.
Collmer A. and D. B. Wilson, Bio/Technol. 1: 594 - 601, 1983.
Deutscher, M.P., Methods Enzymol. 182:779-80, 1990.
Ellouz, S. et al., J. Chromatography 396:307, 1987.
Filho, et al. Can. J. Microbiol. 42:1-5, 1996.
Fliess, A., et al., Eur. J. Appl. Microbiol. Biotechnol. 17:314, 1983.
Goedegebuur et al., Curr. Genet. 41 :89 - 98, 2002.
Goyal, A. et al. Bioresource Technol. 36:37, 1991.
Hazell, B. W. et al., Lett. Appl. Microbiol. 30:282-286, 2000.
Herr et al., Appl. Microbiol. Biotechnol. 5:29-36, 1978.
Hu et al., Mol. Cell. Biol. 11:5792-9, 1991.
Jeeves et al., Biotechnol. Genet. Eng. Rev. 9:327 -369, 1991.
Kawaguchi, T et al., Gene 173(2):287-8, 1996.
Kelley et al. EMBO J. 4 :475-479, 1985.
Knowles, J. et al., TIBTECH 5, 255-261, 1987.
Krishna, S. et al., Bioresource Tech. 77:193-196, 2001.
Kuhls K. et al., Proc. Natl. Acad. Sci. USA 93(15): 7755 - 7760, 1996.
Kumar, A., et al., Textile Chemist and Colorist 29:37-42, 1997.
Medve, J. et al., J. Chromatography A 808:153, 1998.
Mohagheghi, A. et al., Int. J. Syst. Bacteriol. 36:435 - 443, 1986.
Nieves et al., Appl. Biochem. and Biotechnol. 51/52 211 - 223, 1995.
Nunberg et al. Mol. Cell Biol. 4:2306-2315 1984.
Ohmiya et al., Biotechnol. Gen. Engineer. Rev. 14:365-414, 1997.
Okada, M. et al., Appl. Environ. Microbiol., 64:555-563, 1988.
Ooi et al., Nucleic Acid Res. 18:5884, 1990
Penttila et al., Gene 45:253-263, 1986.
Penttila et al., Gene 61: 155 -164, 1987.
Penttila et al., Gene 63: 103-112, 1988.
Pere, J., et al., In Proc. Tappi Pulping Conf., Nashville, TN, 27-31, pp. 693-696, 1996.
Saarilahti et al., Gene 90:9-14, 1990.
Sakamoto et al., Curr. Genet. 27:435-439, 1995.
Saloheimo M, et al., Gene 63:11-22, 1988.
Saloheimo, A. et al., Molecular Microbiology, 13:219-228, 1994.
Saloheimo, M. et al., Eur. J. Biochem., 249:584-591, 1997.
Sambrook et al., MOLECULAR CLONING: A LABORATORY MANUAL (Second Edition), Cold Spring Harbor Press, Plainview, N.Y., 1989.
Schulein, Methods Enzymol., 160, 25, pages 234 et seq, 1988.
Scopes, Methods Enzymol. 90 Pt E:479-90, 1982.
Shoemaker et al., Biochem. Biophys. Acat. 523 :133-146 1978.
Shoemaker, S. et al., Bio/Technology, 1:691-696, 1983
Srisodsuk, M. et al. J. Biol. Chem. 268(28): 20756 - 20761, 1993.
Strathern et al., eds. (1981) The Molecular Biology of the Yeast Saccharomyces, Cold Spring Harbor Press, Plainview. N.Y.
Suurnakki, A. et al., Cellulose 7:189-209, 2000.
Teeri, T. et al., Gene, 51:43-52, 1987
Van Tilbeurgh, H. et al., FEBS Lett. 16:215, 1984.
Tomaz, C. and Queiroz, J., J. Chromatography A 865:123-128, 1999.
Tomme, P. et al., Eur. J. Biochem. 170:575-581, 1988.
Van Tilbeurgh, H. et al., FEBS Lett. 204:223-227, 1986.
Ward, M. et al., Appl. Microbiol. Biotechnol. 39:738 - 743, 1993.
Wood, Biochem. Soc. Trans., 13, pp. 407-410, 1985.
Wood et al., METHODS IN ENZYMOLOGY, 160, 25, p. 87 et seq., Academic Press, New York, 1988.

Some additional embodiments of the invention are described in the following paragraphs
1. A heterologous cellulase fusion construct encoding a cellulase fusion protein, in operable linkage from the 5' end of said construct, comprising:
   (a) a DNA molecule encoding a signal sequence;
   (b) a DNA molecule encoding a catalytic domain of an exo-cellobiohydrolase; and
   (c) a DNA molecule encoding an encoding a second catalytic domain
   wherein said second catalytic domain is the catalytic domain of a cellulase enzyme.
2. The heterologous cellulase fusion construct of para 1 further comprising a linker sequence located 3' of the first catalytic domain and 5' of the second catalytic domain.
3. The heterologous cellulase fusion construct of para 1 wherein the exo-cellobiohydrolase lacks a cellulose binding domain (CBD).
4. The heterologous cellulase fusion construct of para 2 further comprising a kexin site located after the linker sequence and before the second catalytic domain.
5. The heterologous cellulase fusion construct of para 1 further comprising a promoter of a filamentous fungus secretable protein, said promoter located in operable linkage 5' of the first catalytic domain.
6. The heterologous cellulase fusion construct of para 5 wherein the promoter is a *cbh* promoter.
7. The heterologous cellulase fusion construct of para 6 wherein the promoter is a *cbh*1 promoter derived from *T.* reesei.
8. The heterologous cellulase fusion construct of para 1 wherein the first catalytic domain is derived from a CBH1 exo-cellobiohydrolase.
9. The heterologous cellulase fusion construct of para 8 wherein the first catalytic domain is derived from a CBH1 having an amino acid sequence of at least 90% sequence identity with the sequence set forth in SEQ ID NO.: 6.
10. The heterologous cellulase fusion construct of para 1 wherein the second catalytic domain is an endoglucanase catalytic domain.
11. The heterologous cellulase fusion construct of para 10 wherein the second catalytic domain is an exo-cellbiohydrolase catalytic domain.
12. The heterologous cellulase fusion construct of para 10 wherein the second catalytic domain is derived from a bacterial cellulase.
13. The heterologous cellulase fusion construct of para 10 wherein the second catalytic domain is selected from the group consisting of an *Acidothermus cellulolyticus* GH5A endoglucanase I (E1) catalytic domain; an *Acidothermus cellulolyticus* GH48 (GH48) cellulase catalytic domain; an *Acidothermus cellulolyticus* GH74 endoglucanase (GH74-EG) catalytic domain: a *Thermobifida fusca* E3 (Tf-E3) cellulase catalytic domain; and a *Thermobifida fusca* E5 endoglucanase (Tf-E5) catalytic domain.
14. The heterologous cellulase fusion construct of para 1 wherein the heterologous cellulase fusion construct lacks the cellulose binding domain of the exo-cellbiohydrolase of the first catalytic domain and the cellulose binding domain of the cellulase of the second catalytic domain.
15. The heterologous cellulase fusion construct of para 13 wherein the second catalytic domain is an *Acidothermus cellulolyticus* GH5A E1 catalytic domain.
16. The heterologous cellulase fusion construct of para 15 wherein the second catalytic domain is an *Acidothermus cellulolyticus* GH5A E1 catalytic domain having an amino acid sequence of at least 90% sequence identity with the sequence set forth in SEQ ID NO. 8.
17. The heterologous cellulase fusion construct of para 1 further comprising a terminator sequence located 3' to the second catalytic domain.
18. The heterologous cellulase fusion construct of para 1 further comprising a selectable marker.
19. A vector comprising in operable linkage from the 5' end;
   (a) a promoter of a filamentous fungus secretable protein;
   (b) a DNA molecule encoding a signal sequence;
   (c) a DNA molecule encoding a first catalytic domain, wherein said first catalytic domain is derived from a fungal exo-cellobiohydrolase;
   (d) a DNA molecule encoding a second catalytic domain, wherein said second catalytic domain is the catalytic domain of a cellulase; and
   (e) a terminator.
20. The vector according to para 19 further comprising a selectable marker.
21. The vector according to para 19 further comprising a linker located 3' of the first catalytic domain and 5' of the second catalytic domain.
22. The vector according to para 19 wherein the vector will lack the DNA sequence encoding cellulose binding domain of the first catalytic domain.
23. The vector according to para 19 further comprising a kexin site.
24. The vector according to para 19 wherein the second catalytic domain is derived from a bacterial cellulase.
25. The vector according to Claim 19 wherein the vector lacks the DNA sequence encoding cellulose binding domain of the cellulase of the second catalytic domain.
26. A fungal host cell transformed with a heterologous cellulase fusion construct according to para 1.
27. A fungal host cell transformed with a vector comprising a heterologous cellulase fusion construct according to para 19.
28. A recombinant fungal cell comprising a DNA sequence selected from the group consisting of a heterologous cellulase fusion construct and a vector comprising the heterologous cellulase fusion construct.
29. A fungal host cell according to para 26 wherein said fungal host cell is a *Trichoderma* host cell.
30. A fungal host cell according to para 29 wherein said *Trichoderma* host cell is a strain of *T. reesei.*
31. A fungal host cell according to para 29 wherein one or more of the native cellulase genes has been deleted from the fungal host cell.
32. A fungal host cell according to para 31 wherein the native cellulase genes are selected from the group consisting of *cbh1, cbh2, egl1* and *egl2.*
33. An isolated cellulase fusion protein having cellulolytic activity comprising
   (a) a first catalytic domain, wherein said first catalytic domain is an exo-cellobiohydrolase catalytic domain and
   (b) a second catalytic domain, wherein said second catalytic domain is derived from a cellulase.
34. An isolated cellulase fusion protein according to para 33 wherein the exo-cellobiohydrolase is a CBH1.
35. An isolated cellulase fusion protein according to para 33 wherein the second catalytic domain is derived from a bacterial cellulase.
36. An isolated cellulase fusion protein according to para 35 wherein the bacterial cellulase is an endoglucanase.
37. An isolated cellulase fusion protein according to para 35 wherein the bacterial cellulase is an exo-cellobiohydrolase.
38. An isolated cellulase fusion protein according to para 35 wherein the bacterial cellulase is derived from a strain of *Acidothermus cellulolyticus.*
39. A cellulolytic composition comprising the isolated cellulase fusion protein according to para 33.
40. A method of producing an enzyme having cellulolytic activity comprising:
   (a) stably transforming a filamentous fungal host cell with either a heterologous cellulase fusion construct according to para 1 or vector according to para 19;
   (b) cultivating the transformed fungal host cell under conditions suitable for said fungal host cell to produce an enzyme having cellulolytic activity; and
   (c) recovering said enzyme.
41. The method of producing an enzyme having cellulolytic activity according to para 40 wherein the filamentous fungal host cell is a *Trichoderma* cell.
42. The method of producing an enzyme having cellulolytic activity according to para 40 wherein the filamentous fungal host cell is a T. *reesei* host cell.
43. The method of producing an enzyme having cellulolytic activity according to para 40 wherein the exo-cellobiohydrolase is a CBH1 and the cellulase is selected from the group consisting of an *Acidothermus cellulolyticus* cellulase and a *Thermobifida fusca* cellulase.
44. The method of producing an enzyme having cellulolytic activity according to para 40 wherein the recovered enzyme is selected from the group consisting of a cellulase fusion protein, components of the cellulase fusion protein, a combination of the cellulase fusion protein and the components thereof.
45. The method of producing an enzyme having cellulolytic activity according to para 44 wherein the recovered enzyme(s) is purified
46. A *Trichoderma* host cell which expresses a cellulase fusion protein, wherein said fusion protein comprises a first catalytic domain, wherein the catalytic domain is derived from an exo-cellobiohydrolase and a second catalytic domain, wherein the second catalytic domain is derived from a cellulase enzyme.
47. The *Trichoderma* host cell according to para 46 wherein the *Trichoderma* host cell is a *T. reesei* cell.
48. The *Trichoderma* host cell according to para 46 wherein the exo-cellobiohydrolase is a CBH1 and the cellulase is a bacterial cellulase.
49. The *Trichoderma* host cell according to para 48 wherein the bacterial cellulase is derived from an *Acidothermus cellulolyticus* cellulase.
50. The *Trichoderma* host cell according to para 49 wherein the bacterial cellulase is selected from the group consisting of an *Acidothermus cellulolyticus* E1, GH48 and GH74 cellulase.
51. The *Trichoderma* host cell according to para 46 wherein the fusion protein will lack the CBD of the cellulase.
52. The *Trichoderma* host cell according to para 46 wherein one or more of the native cellulase genes has been deleted from said Trichoderma host cell.
53. A fungal cellulase composition comprising a cellulase fusion protein or components thereof, wherein the fusion protein or components thereof is the product of a recombinant *Trichoderma spp.*

## Claims

1. A heterologous exo-endo cellulase fusion construct comprising in operable linkage from the 5' end of said construct
(a) a DNA molecule encoding a signal sequence;
(b) a DNA molecule encoding a catalytic domain of an exo-cellobiohydrolase; and
(c) a DNA molecule encoding a *Thermobifida fusca* E3 (Tf-E3) cellulase catalytic domain;
wherein the fusion construct optionally lacks a cellulose binding domain of the exo-cellobiohydrolase and/or a cellulose binding domain of the Tf-E3 cellulase.

2. The heterologous exo-endo cellulase fusion construct according to claim 1 further comprising a linker sequence located 3' of the catalytic domain of the exo-cellobiohydrolase and 5' of the catalytic domain of the endoglucanase, and optionally further comprising a kexin site located after the linker sequence and before the coding region of the endoglucanase catalytic domain.

3. The heterologous exo-endo fusion construct according to claim 1 further comprising a promoter of a filamentous fungus secretable protein, said promoter located in operable linkage 5' of the coding region of the exo-cellobiohydrolase catalytic domain, e.g. wherein the promoter is a *cbh* promoter, such as a *cbh1* promoter derived from *T.* reesei.

4. The heterologous exo-endo fusion construct according to claim 1 wherein the exo-cellobiohydrolase is a CBH1, e.g. wherein the CBH1 is derived from T. *reesei,* and optionally comprises an amino acid sequence of at least 90% sequence identity with the sequence set forth in SEQ ID NO.:6.

5. The heterologous exo-endo fusion construct according to claim 1 wherein the Tf-E3 cellulase catalytic domain has an amino acid sequence encoded by a nucleic acid sequence having at least 90% sequence identity with the sequence set forth in SEQ ID NO. 13.

6. A vector comprising in operable linkage a promoter of a filamentous fungus secretable protein, a heterologous exo-endo cellulase fusion construct according to any one of claims 1 to 5, a terminator, and optionally a selectable marker.

7. A recombinant fungal cell comprising the heterologous exo-endo cellulase fusion construct according to any one of claims 1 to 5 or a vector according to claim 6, e.g. wherein the fungal host cell is a *Trichoderma* host cell, such as a strain of T. *reesei,* and wherein optionally at least one gene selected from the group consisting of the *cbh1, cbh2, egl1* and *egl2* has been deleted from the fungal cell.

8. An isolated cellulase fusion protein having cellulolytic activity which comprises an exo-cellobiohydrolase catalytic domain, and a Tf-E3 cellulase catalytic domain, which optionally lacks a cellulose binding domain of the exo-cellobiohydrolase and/or a cellulose binding domain of the Tf-E3 cellulase.

9. The isolated cellulase fusion protein according to claim 8 further comprising a linker sequence located after the catalytic domain of the exo-cellobiohydrolase and before the catalytic domain of the endoglucanase, and optionally a kexin site located after the linker sequence and before the endoglucanase catalytic domain.

10. The isolated cellulase fusion protein according to claim 8 wherein the exo-cellobiohydrolase is a CBH1, e.g. a CBH1 derived from *T. reesei,* which optionally comprises an amino acid sequence of at least 90% sequence identity with the sequence set forth in SEQ ID NO.:6.

11. The isolated cellulase fusion protein according to claim 8 wherein the Tf-E3 cellulase catalytic domain has an amino acid sequence encoded by a nucleic acid sequence having at least 90% sequence identity with the sequence set forth in SEQ ID NO. 13.

12. A cellulolytic composition comprising the isolated cellulase fusion protein according to any one of claims 8 to 11.

13. A method of producing an enzyme having cellulolytic activity comprising,
(a) cultivating a fungal host cell according to claim 7 under conditions suitable for said fungal host cell to produce an enzyme having cellulolytic activity;
(b) recovering said enzyme; and optionally
(c) purifying said enzyme.

14. A method according to claim 13 wherein the recovered enzyme is selected from the group consisting of a cellulase fusion protein, components of the cellulase fusion protein, and a combination of the cellulase fusion protein and the components thereof.


SEQUENCE LISTING

<110>  Genencor International, Inc.

<120>  Cellulase Fusion Protein and Heterologous Cellulase Fusion
       Construct Encoding the Same

<130>  GRF/FP6718183

<140>  EP 10010133.6
<141>  2005-03-25

<150>  05744703.9
<151>  2005-03-25

<150>  PCT/US2005/010242
<151>  2005-03-25

<150>  US 60/556,711
<151>  2004-03-25

<160>  30

<170>  PatentIn version 3.2

<210>  1
<211>  1570
<212>  DNA
<213>  Trichoderma reesei

<400>  1
atgtatcgga agttggccgt catctcggcc ttcttggcca cagctcgtgc tcagtcggcc      60
tgcactctcc aatcggagac tcacccgcct ctgacatggc agaaatgctc gtctggtggc     120
acttgcactc aacagacagg ctccgtggtc atcgacgcca actggcgctg gactcacgct     180
acgaacagca gcacgaactg ctacgatggc aacacttgga gctcgaccct atgtcctgac     240
aacgagacct gcgcgaagaa ctgctgtctg gacggtgccg cctacgcgtc cacgtacgga     300
gttaccacga gcggtaacag cctctccatt ggctttgtca cccagtctgc gcagaagaac     360
gttggcgctc gcctttacct tatggcgagc gacacgacct accaggaatt caccctgctt     420
ggcaacgagt tctctttcga tgttgatgtt tcgcagctgc cgtaagtgac ttaccatgaa     480
cccctgacgt atcttcttgt gggctcccag ctgactggcc aatttaaggt gcggcttgaa     540
cggagctctc tacttcgtgt ccatggacgc ggatggtggc gtgagcaagt atcccaccaa     600
caccgctggc gccaagtacg gcacggggta ctgtgacagc cagtgtcccc gcgatctgaa     660
gttcatcaat ggccaggcca acgttgaggg ctgggagccg tcatccaaca acgcaaacac     720
gggcattgga ggacacggaa gctgctgctc tgagatggat atctgggagg ccaactccat     780
ctccgaggct cttacccccc acccttgcac gactgtcggc caggagatct gcgagggtga     840
tgggtgcggc ggaacttact ccgataacag atatggcggc acttgcgatc ccgatggctg     900
cgactggaac ccataccgcc tgggcaacac cagcttctac ggccctggct caagctttac     960
cctcgatacc accaagaaat tgaccgttgt cacccagttc gagacgtcgg gtgccatcaa    1020
ccgatactat gtccagaatg gcgtcacttt ccagcagccc aacgccgagc ttggtagtta    1080
ctctggcaac gagctcaacg atgattactg cacagctgag gaggcagaat tcggcggatc    1140
ctctttctca gacaagggcg gcctgactca gttcaagaag gctacctctg gcggcatggt    1200
tctggtcatg agtctgtggg atgatgtgag tttgatggac aaacatgcgc gttgacaaag    1260
agtcaagcag ctgactgaga tgttacagta ctacgccaac atgctgtggc tggactccac    1320
ctacccgaca aacgagacct cctccacacc cggtgccgtg cgcggaagct gctccaccag    1380
ctccggtgtc cctgctcagg tcgaatctca gtctcccaac gccaaggtca ccttctccaa    1440
catcaagttc ggacccattg gcagcaccgg caaccctagc ggcggcaacc ctcccggcgg    1500
aaacccgcct ggcaccacca ccacccgccg cccagccact accactggaa gctctcccgg    1560

acctactagt                                                          1570

<210>  2

<211>  51
<212>  DNA
<213>  Trichoderma reesei

<400>  2
atgtatcgga agttggccgt catctcggcc ttcttggcca cagctcgtgc t              51

<210>  3
<211>  1438
<212>  DNA
<213>  Trichoderma reesei

<400>  3
cagtcggcct gcactctcca atcggagact cacccgcctc tgacatggca gaaatgctcg      60
tctggtggca cttgcactca acagacaggc tccgtggtca tcgacgccaa ctggcgctgg     120
actcacgcta cgaacagcag cacgaactgc tacgatggca acacttggag ctcgacccta     180
tgtcctgaca acgagacctg cgcgaagaac tgctgtctgg acggtgccgc ctacgcgtcc     240
acgtacggag ttaccacgag cggtaacagc ctctccattg gctttgtcac ccagtctgcg     300
cagaagaacg ttggcgctcg cctttacctt atggcgagcg acacgaccta ccaggaattc     360
accctgcttg gcaacgagtt ctctttcgat gttgatgttt cgcagctgcc gtaagtgact     420
taccatgaac ccctgacgta tcttcttgtg ggctcccagc tgactggcca atttaaggtg     480
cggcttgaac ggagctctct acttcgtgtc catggacgcg gatggtggcg tgagcaagta     540
tcccaccaac accgctggcg ccaagtacgg cacggggtac tgtgacagcc agtgtccccg     600
cgatctgaag ttcatcaatg gccaggccaa cgttgagggc tgggagccgt catccaacaa     660
cgcaaacacg ggcattggag gacacggaag ctgctgctct gagatggata tctgggaggc     720
caactccatc tccgaggctc ttacccccca cccttgcacg actgtcggcc aggagatctg     780
cgagggtgat gggtgcggcg gaacttactc cgataacaga tatggcggca cttgcgatcc     840
cgatggctgc gactggaacc cataccgcct gggcaacacc agcttctacg gccctggctc     900
aagctttacc ctcgatacca ccaagaaatt gaccgttgtc acccagttcg agacgtcggg     960
tgccatcaac cgatactatg tccagaatgg cgtcactttc cagcagccca acgccgagct    1020
tggtagttac tctggcaacg agctcaacga tgattactgc acagctgagg aggcagaatt    1080
cggcggatcc tctttctcag acaagggcgg cctgactcag ttcaagaagg ctacctctgg    1140
cggcatggtt ctggtcatga gtctgtggga tgatgtgagt ttgatggaca aacatgcgcg    1200
ttgacaaaga gtcaagcagc tgactgagat gttacagtac tacgccaaca tgctgtggct    1260
ggactccacc tacccgacaa acgagacctc ctccacaccc ggtgccgtgc gcggaagctg    1320
ctccaccagc tccggtgtcc ctgctcaggt cgaatctcag tctcccaacg ccaaggtcac    1380
cttctccaac atcaagttcg gacccattgg cagcaccggc aaccctagcg gcggcaac      1438

<210>  4
<211>  81
<212>  DNA
<213>  Trichoderma reesei

<400>  4
cctcccggcg gaaacccgcc tggcaccacc accacccgcc gcccagccac taccactgga      60
agctctcccg gacctactag t                                                81

<210>  5
<211>  480
<212>  PRT
<213>  Trichoderma reesei

<400>  5

Met Tyr Arg Lys Leu Ala Val Ile Ser Ala Phe Leu Ala Thr Ala Arg
1               5                   10                  15
Ala Gln Ser Ala Cys Thr Leu Gln Ser Glu Thr His Pro Pro Leu Thr
            20                  25                  30
Trp Gln Lys Cys Ser Ser Gly Gly Thr Cys Thr Gln Gln Thr Gly Ser
        35                  40                  45
Val Val Ile Asp Ala Asn Trp Arg Trp Thr His Ala Thr Asn Ser Ser
    50                  55                  60
Thr Asn Cys Tyr Asp Gly Asn Thr Trp Ser Ser Thr Leu Cys Pro Asp
65                  70                  75                  80
Asn Glu Thr Cys Ala Lys Asn Cys Cys Leu Asp Gly Ala Ala Tyr Ala
                85                  90                  95
Ser Thr Tyr Gly Val Thr Thr Ser Gly Asn Ser Leu Ser Ile Gly Phe
            100                 105                 110
Val Thr Gln Ser Ala Gln Lys Asn Val Gly Ala Arg Leu Tyr Leu Met
        115                 120                 125
Ala Ser Asp Thr Thr Tyr Gln Glu Phe Thr Leu Leu Gly Asn Glu Phe
    130                 135                 140
Ser Phe Asp Val Asp Val Ser Gln Leu Pro Cys Gly Leu Asn Gly Ala
145                 150                 155                 160
Leu Tyr Phe Val Ser Met Asp Ala Asp Gly Gly Val Ser Lys Tyr Pro
                165                 170                 175
Thr Asn Thr Ala Gly Ala Lys Tyr Gly Thr Gly Tyr Cys Asp Ser Gln
            180                 185                 190
Cys Pro Arg Asp Leu Lys Phe Ile Asn Gly Gln Ala Asn Val Glu Gly
        195                 200                 205
Trp Glu Pro Ser Ser Asn Asn Ala Asn Thr Gly Ile Gly Gly His Gly
    210                 215                 220
Ser Cys Cys Ser Glu Met Asp Ile Trp Glu Ala Asn Ser Ile Ser Glu
225                 230                 235                 240
Ala Leu Thr Pro His Pro Cys Thr Thr Val Gly Gln Glu Ile Cys Glu
                245                 250                 255
Gly Asp Gly Cys Gly Gly Thr Tyr Ser Asp Asn Arg Tyr Gly Gly Thr
            260                 265                 270
Cys Asp Pro Asp Gly Cys Asp Trp Asn Pro Tyr Arg Leu Gly Asn Thr
        275                 280                 285
Ser Phe Tyr Gly Pro Gly Ser Ser Phe Thr Leu Asp Thr Thr Lys Lys
    290                 295                 300
Leu Thr Val Val Thr Gln Phe Glu Thr Ser Gly Ala Ile Asn Arg Tyr
305                 310                 315                 320
Tyr Val Gln Asn Gly Val Thr Phe Gln Gln Pro Asn Ala Glu Leu Gly
                325                 330                 335
Ser Tyr Ser Gly Asn Glu Leu Asn Asp Asp Tyr Cys Thr Ala Glu Glu
            340                 345                 350
Ala Glu Phe Gly Gly Ser Ser Phe Ser Asp Lys Gly Gly Leu Thr Gln
        355                 360                 365
Phe Lys Lys Ala Thr Ser Gly Gly Met Val Leu Val Met Ser Leu Trp
    370                 375                 380
Asp Asp Tyr Tyr Ala Asn Met Leu Trp Leu Asp Ser Thr Tyr Pro Thr
385                 390                 395                 400
Asn Glu Thr Ser Ser Thr Pro Gly Ala Val Arg Gly Ser Cys Ser Thr
                405                 410                 415
Ser Ser Gly Val Pro Ala Gln Val Glu Ser Gln Ser Pro Asn Ala Lys
            420                 425                 430
Val Thr Phe Ser Asn Ile Lys Phe Gly Pro Ile Gly Ser Thr Gly Asn
        435                 440                 445
Pro Ser Gly Gly Asn Pro Pro Gly Gly Asn Pro Pro Gly Thr Thr Thr

    450                 455                 460
Thr Arg Arg Pro Ala Thr Thr Thr Gly Ser Ser Pro Gly Pro Thr Ser
465                 470                 475                 480

<210>  6
<211>  431
<212>  PRT
<213>  Trichoderma reesei

<400>  6
Gln Ser Ala Cys Thr Leu Gln Ser Glu Thr His Pro Pro Leu Thr Trp
1               5                   10                  15
Gln Lys Cys Ser Ser Gly Gly Thr Cys Thr Gln Gln Thr Gly Ser Val
            20                  25                  30
Val Ile Asp Ala Asn Trp Arg Trp Thr His Ala Thr Asn Ser Ser Thr
        35                  40                  45
Asn Cys Tyr Asp Gly Asn Thr Trp Ser Ser Thr Leu Cys Pro Asp Asn
    50                  55                  60
Glu Thr Cys Ala Lys Asn Cys Cys Leu Asp Gly Ala Ala Tyr Ala Ser
65                  70                  75                  80
Thr Tyr Gly Val Thr Thr Ser Gly Asn Ser Leu Ser Ile Gly Phe Val
                85                  90                  95
Thr Gln Ser Ala Gln Lys Asn Val Gly Ala Arg Leu Tyr Leu Met Ala
            100                 105                 110
Ser Asp Thr Thr Tyr Gln Glu Phe Thr Leu Leu Gly Asn Glu Phe Ser
        115                 120                 125
Phe Asp Val Asp Val Ser Gln Leu Pro Cys Gly Leu Asn Gly Ala Leu
    130                 135                 140
Tyr Phe Val Ser Met Asp Ala Asp Gly Gly Val Ser Lys Tyr Pro Thr
145                 150                 155                 160
Asn Thr Ala Gly Ala Lys Tyr Gly Thr Gly Tyr Cys Asp Ser Gln Cys
                165                 170                 175
Pro Arg Asp Leu Lys Phe Ile Asn Gly Gln Ala Asn Val Glu Gly Trp
            180                 185                 190
Glu Pro Ser Ser Asn Asn Ala Asn Thr Gly Ile Gly Gly His Gly Ser
        195                 200                 205
Cys Cys Ser Glu Met Asp Ile Trp Glu Ala Asn Ser Ile Ser Glu Ala
    210                 215                 220
Leu Thr Pro His Pro Cys Thr Thr Val Gly Gln Glu Ile Cys Glu Gly
225                 230                 235                 240
Asp Gly Cys Gly Gly Thr Tyr Ser Asp Asn Arg Tyr Gly Gly Thr Cys
                245                 250                 255
Asp Pro Asp Gly Cys Asp Trp Asn Pro Tyr Arg Leu Gly Asn Thr Ser
            260                 265                 270
Phe Tyr Gly Pro Gly Ser Ser Phe Thr Leu Asp Thr Thr Lys Lys Leu
        275                 280                 285
Thr Val Val Thr Gln Phe Glu Thr Ser Gly Ala Ile Asn Arg Tyr Tyr
    290                 295                 300
Val Gln Asn Gly Val Thr Phe Gln Gln Pro Asn Ala Glu Leu Gly Ser
305                 310                 315                 320
Tyr Ser Gly Asn Glu Leu Asn Asp Asp Tyr Cys Thr Ala Glu Glu Ala
                325                 330                 335
Glu Phe Gly Gly Ser Ser Phe Ser Asp Lys Gly Gly Leu Thr Gln Phe
            340                 345                 350
Lys Lys Ala Thr Ser Gly Gly Met Val Leu Val Met Ser Leu Trp Asp
        355                 360                 365
Asp Tyr Tyr Ala Asn Met Leu Trp Leu Asp Ser Thr Tyr Pro Thr Asn

370                 375                 380
Glu Thr Ser Ser Thr Pro Gly Ala Val Arg Gly Ser Cys Ser Thr Ser
385                 390                 395                 400
Ser Gly Val Pro Ala Gln Val Glu Ser Gln Ser Pro Asn Ala Lys Val
                405                 410                 415
Thr Phe Ser Asn Ile Lys Phe Gly Pro Ile Gly Ser Thr Gly Asn
            420                 425                 430

<210>  7
<211>  1077
<212>  DNA
<213>  Acidothermus cellulolyticus

<400>  7
gcgggcggcg gctattggca cacgagcggc cgggagatcc tggacgcgaa caacgtgccg      60
gtacggatcg ccggcatcaa ctggtttggg ttcgaaacct gcaattacgt cgtgcacggt     120
ctctggtcac gcgactaccg cagcatgctc gaccagataa agtcgctcgg ctacaacaca     180
atccggctgc cgtactctga cgacattctc aagccgggca ccatgccgaa cagcatcaat     240
ttttaccaga tgaatcagga cctgcagggt ctgacgtcct tgcaggtcat ggacaaaatc     300
gtcgcgtacg ccggtcagat cggcctgcgc atcattcttg accgccaccg accggattgc     360
agcgggcagt cggcgctgtg gtacacgagc agcgtctcgg aggctacgtg gatttccgac     420
ctgcaagcgc tggcgcagcg ctacaaggga aacccgacgg tcgtcggctt tgacttgcac     480
aacgagccgc atgacccggc ctgctggggc tgcggcgatc cgagcatcga ctggcgattg     540
gccgccgagc gggccggaaa cgccgtgctc tcggtgaatc cgaacctgct cattttcgtc     600
gaaggtgtgc agagctacaa cggagactcc tactggtggg gcggcaacct gcaaggagcc     660
ggccagtacc cggtcgtgct gaacgtgccg aaccgcctgg tgtactcggc gcacgactac     720
gcgacgagcg tctacccgca gacgtggttc agcgatccga ccttccccaa caacatgccc     780
ggcatctgga acaagaactg gggatacctc ttcaatcaga acattgcacc ggtatggctg     840
ggcgaattcg gtacgacact gcaatccacg accgaccaga cgtggctgaa gacgctcgtc     900
cagtacctac ggccgaccgc gcaatacggt gcggacagct tccagtggac cttctggtcc     960
tggaaccccg attccggcga cacaggagga attctcaagg atgactggca gacggtcgac    1020
acagtaaaag acggctatct cgcgccgatc aagtcgtcga ttttcgatcc tgtcggc       1077

<210>  8
<211>  359
<212>  PRT
<213>  Acidothermus cellulolyticus

<400>  8
Ala Gly Gly Gly Tyr Trp His Thr Ser Gly Arg Glu Ile Leu Asp Ala
1               5                   10                  15
Asn Asn Val Pro Val Arg Ile Ala Gly Ile Asn Trp Phe Gly Phe Glu
            20                  25                  30
Thr Cys Asn Tyr Val Val His Gly Leu Trp Ser Arg Asp Tyr Arg Ser
        35                  40                  45
Met Leu Asp Gln Ile Lys Ser Leu Gly Tyr Asn Thr Ile Arg Leu Pro
    50                  55                  60
Tyr Ser Asp Asp Ile Leu Lys Pro Gly Thr Met Pro Asn Ser Ile Asn
65                  70                  75                  80
Phe Tyr Gln Met Asn Gln Asp Leu Gln Gly Leu Thr Ser Leu Gln Val
                85                  90                  95
Met Asp Lys Ile Val Ala Tyr Ala Gly Gln Ile Gly Leu Arg Ile Ile
            100                 105                 110
Leu Asp Arg His Arg Pro Asp Cys Ser Gly Gln Ser Ala Leu Trp Tyr
        115                 120                 125
Thr Ser Ser Val Ser Glu Ala Thr Trp Ile Ser Asp Leu Gln Ala Leu
    130                 135                 140

Ala Gln Arg Tyr Lys Gly Asn Pro Thr Val Val Gly Phe Asp Leu His
145                 150                 155                 160
Asn Glu Pro His Asp Pro Ala Cys Trp Gly Cys Gly Asp Pro Ser Ile
                165                 170                 175
Asp Trp Arg Leu Ala Ala Glu Arg Ala Gly Asn Ala Val Leu Ser Val
            180                 185                 190
Asn Pro Asn Leu Leu Ile Phe Val Glu Gly Val Gln Ser Tyr Asn Gly
        195                 200                 205
Asp Ser Tyr Trp Trp Gly Gly Asn Leu Gln Gly Ala Gly Gln Tyr Pro
    210                 215                 220
Val Val Leu Asn Val Pro Asn Arg Leu Val Tyr Ser Ala His Asp Tyr
225                 230                 235                 240
Ala Thr Ser Val Tyr Pro Gln Thr Trp Phe Ser Asp Pro Thr Phe Pro
                245                 250                 255
Asn Asn Met Pro Gly Ile Trp Asn Lys Asn Trp Gly Tyr Leu Phe Asn
            260                 265                 270
Gln Asn Ile Ala Pro Val Trp Leu Gly Glu Phe Gly Thr Thr Leu Gln
        275                 280                 285
Ser Thr Thr Asp Gln Thr Trp Leu Lys Thr Leu Val Gln Tyr Leu Arg
    290                 295                 300
Pro Thr Ala Gln Tyr Gly Ala Asp Ser Phe Gln Trp Thr Phe Trp Ser
305                 310                 315                 320
Trp Asn Pro Asp Ser Gly Asp Thr Gly Gly Ile Leu Lys Asp Asp Trp
                325                 330                 335
Gln Thr Val Asp Thr Val Lys Asp Gly Tyr Leu Ala Pro Ile Lys Ser
            340                 345                 350
Ser Ile Phe Asp Pro Val Gly
        355

<210>  9
<211>  1914
<212>  DNA
<213>  Acidothermus cellulolyticus

<400>  9
aacgacccgt acatccagcg gttcctcacg atgtacaaca agattcacga cccagcgaac      60
ggctacttca gcccgcaggg aattccctac cactcggtag aaacgctcat cgttgaggca     120
ccggactacg ggcacgagac aacttcggag gcgtacagct tctggctctg gctcgaagcg     180
acgtacggcg cagtgaccgg caactggacg ccgttcaaca acgcctggac gacgatggaa     240
acgtacatga tcccgcagca cgcggaccag ccgaacaacg cgtcgtacaa ccccaacagc     300
ccggcgtcgt acgctccgga agagccgctg cccagcatgt acccggttgc catcgacagc     360
agcgtgccgg ttgggcacga cccgctcgcc gccgaattgc agtcgacgta cggcactccg     420
gacatttacg gcatgcactg gctggccgac gttgacaaca tctacggata cggcgacagc     480
cccggcggtg gttgcgaact cggtccttcc gctaagggcg tctcctacat caacacattc     540
cagcgcggct cgcaggagtc cgtctgggag acggtcaccc agccgacgtg cgacaacggc     600
aagtacggtg ggcgcacggg ctacgtcgac ctgttcatcc agggttcgac gccgccgcag     660
tggaagtaca ccgatgcccc ggacgccgac gcccgtgccg tccaggctgc gtactgggcc     720
tacacctggg catcggcgca gggcaaggca agcgcgattg ccccgacgat cgccaaggcg     780
gccaaactcg gcgactacct gcggtactcg ctctttgaca agtacttcaa gcaggtcggc     840
aactgctacc cggccagctc ctgccctgga gcaaccggac gccagagcga gacctacctg     900
atcggctggt actacgcctg gggcggctca agccaaggct gggcctggcg cattggtgac     960
ggcgccgcgc acttcggcta ccagaatccg cttgccgcgt gggcgatgtc gaacgtgaca    1020
ccgctcattc cgctctcgcc cacggcaaag agcgactggg cggcgagctt gcagcgccag    1080
ctggagttct accagtggtt gcaatccgcg gaaggagcca ttgcgggcgg cgccaccaac    1140
agctggaacg gcaattacgg gaccccgccg gccggagact cgaccttcta cggcatggcg    1200
tacgactggg agccggtcta ccacgacccg ccgagcaaca actggttcgg cttccaggcg    1260
tggtccatgg aacgggttgc cgagtactac tacgtcaccg gcgacccgaa ggccaaggcg    1320

ctgctcgaca agtgggtcgc atgggtgaag ccgaatgtca ccaccggtgc ctcatggtcg 1380
attccgtcga atttgtcctg gagcggccaa ccggatacct ggaatccgag caacccagga 1440
acgaatgcca acctgcacgt gaccatcacg tcgtccgggc aggacgtcgg tgttgccgcg 1500
gcgctcgcga agacactcga gtactacgcg gcaaaatccg gcgatacggc ctcgcgcgac 1560
ctcgcgaagg gattgctcga ctccatgtgg aacaacgacc aggacagcct cggtgtgagc 1620
acaccggaga cgcggaccga ctactctcgg ttcactcagg tgtacgaccc gacgactggt 1680
gacggcctct acatcccgtc gggttggacg gggaccatgc ccaacggtga ccaaatcaag 1740
ccgggtgcga ccttcctgag catccggtcc tggtacacca aggatccgca gtggtcgaag 1800
gtgcaggcgt acctcaacgg cgggcctgct ccgacgttca actaccaccg gttctgggcg 1860
gagtccgact tcgcgatggc gaacgccgat tttggcatgc tcttcccatc cggg 1914

<210> 10
<211> 638
<212> PRT
<213> Acidothermus cellulolyticus

<400> 10
Asn Asp Pro Tyr Ile Gln Arg Phe Leu Thr Met Tyr Asn Lys Ile His
1               5                   10                  15
Asp Pro Ala Asn Gly Tyr Phe Ser Pro Gln Gly Ile Pro Tyr His Ser
            20                  25                  30
Val Glu Thr Leu Ile Val Glu Ala Pro Asp Tyr Gly His Glu Thr Thr
        35                  40                  45
Ser Glu Ala Tyr Ser Phe Trp Leu Trp Leu Glu Ala Thr Tyr Gly Ala
    50                  55                  60
Val Thr Gly Asn Trp Thr Pro Phe Asn Asn Ala Trp Thr Thr Met Glu
65                  70                  75                  80
Thr Tyr Met Ile Pro Gln His Ala Asp Gln Pro Asn Asn Ala Ser Tyr
                85                  90                  95
Asn Pro Asn Ser Pro Ala Ser Tyr Ala Pro Glu Glu Pro Leu Pro Ser
            100                 105                 110
Met Tyr Pro Val Ala Ile Asp Ser Ser Val Pro Val Gly His Asp Pro
        115                 120                 125
Leu Ala Ala Glu Leu Gln Ser Thr Tyr Gly Thr Pro Asp Ile Tyr Gly
    130                 135                 140
Met His Trp Leu Ala Asp Val Asp Asn Ile Tyr Gly Tyr Gly Asp Ser
145                 150                 155                 160
Pro Gly Gly Gly Cys Glu Leu Gly Pro Ser Ala Lys Gly Val Ser Tyr
                165                 170                 175
Ile Asn Thr Phe Gln Arg Gly Ser Gln Glu Ser Val Trp Glu Thr Val
            180                 185                 190
Thr Gln Pro Thr Cys Asp Asn Gly Lys Tyr Gly Gly Ala His Gly Tyr
        195                 200                 205
Val Asp Leu Phe Ile Gln Gly Ser Thr Pro Pro Gln Trp Lys Tyr Thr
    210                 215                 220
Asp Ala Pro Asp Ala Asp Ala Arg Ala Val Gln Ala Ala Tyr Trp Ala
225                 230                 235                 240
Tyr Thr Trp Ala Ser Ala Gln Gly Lys Ala Ser Ala Ile Ala Pro Thr
                245                 250                 255
Ile Ala Lys Ala Ala Lys Leu Gly Asp Tyr Leu Arg Tyr Ser Leu Phe
            260                 265                 270
Asp Lys Tyr Phe Lys Gln Val Gly Asn Cys Tyr Pro Ala Ser Ser Cys
        275                 280                 285
Pro Gly Ala Thr Gly Arg Gln Ser Glu Thr Tyr Leu Ile Gly Trp Tyr
    290                 295                 300
Tyr Ala Trp Gly Gly Ser Ser Gln Gly Trp Ala Trp Arg Ile Gly Asp
305                 310                 315                 320

Gly Ala Ala His Phe Gly Tyr Gln Asn Pro Leu Ala Ala Trp Ala Met
                325                 330                 335
Ser Asn Val Thr Pro Leu Ile Pro Leu Ser Pro Thr Ala Lys Ser Asp
            340                 345                 350
Trp Ala Ala Ser Leu Gln Arg Gln Leu Glu Phe Tyr Gln Trp Leu Gln
        355                 360                 365
Ser Ala Glu Gly Ala Ile Ala Gly Gly Ala Thr Asn Ser Trp Asn Gly
    370                 375                 380
Asn Tyr Gly Thr Pro Pro Ala Gly Asp Ser Thr Phe Tyr Gly Met Ala
385                 390                 395                 400
Tyr Asp Trp Glu Pro Val Tyr His Asp Pro Pro Ser Asn Asn Trp Phe
                405                 410                 415
Gly Phe Gln Ala Trp Ser Met Glu Arg Val Ala Glu Tyr Tyr Tyr Val
            420                 425                 430
Thr Gly Asp Pro Lys Ala Lys Ala Leu Leu Asp Lys Trp Val Ala Trp
        435                 440                 445
Val Lys Pro Asn Val Thr Thr Gly Ala Ser Trp Ser Ile Pro Ser Asn
    450                 455                 460
Leu Ser Trp Ser Gly Gln Pro Asp Thr Trp Asn Pro Ser Asn Pro Gly
465                 470                 475                 480
Thr Asn Ala Asn Leu His Val Thr Ile Thr Ser Ser Gly Gln Asp Val
                485                 490                 495
Gly Val Ala Ala Ala Leu Ala Lys Thr Leu Glu Tyr Tyr Ala Ala Lys
            500                 505                 510
Ser Gly Asp Thr Ala Ser Arg Asp Leu Ala Lys Gly Leu Leu Asp Ser
        515                 520                 525
Met Trp Asn Asn Asp Gln Asp Ser Leu Gly Val Ser Thr Pro Glu Thr
    530                 535                 540
Arg Thr Asp Tyr Ser Arg Phe Thr Gln Val Tyr Asp Pro Thr Thr Gly
545                 550                 555                 560
Asp Gly Leu Tyr Ile Pro Ser Gly Trp Thr Gly Thr Met Pro Asn Gly
                565                 570                 575
Asp Gln Ile Lys Pro Gly Ala Thr Phe Leu Ser Ile Arg Ser Trp Tyr
            580                 585                 590
Thr Lys Asp Pro Gln Trp Ser Lys Val Gln Ala Tyr Leu Asn Gly Gly
        595                 600                 605
Pro Ala Pro Thr Phe Asn Tyr His Arg Phe Trp Ala Glu Ser Asp Phe
    610                 615                 620
Ala Met Ala Asn Ala Asp Phe Gly Met Leu Phe Pro Ser Gly
625                 630                 635

<210>  11
<211>  2223
<212>  DNA
<213>  Acidothermus cellulolyticus

<400>  11
gcgacgactc agccgtacac ctggagcaac gtggcgatcg ggggcggcgg ctttgtcgac      60
gggatcgtct tcaatgaagg tgcaccggga attctgtacg tgcggacgga catcgggggg     120
atgtatcgat gggatgccgc caacgggcgg tggatccctc ttctggattg ggtgggatgg     180
aacaattggg ggtacaacgg cgtcgtcagc attgcggcag acccgatcaa tactaacaag     240
gtatgggccg ccgtcggaat gtacaccaac agctgggacc caaacgacgg agcgattctc     300
cgctcgtctg atcagggcgc aacgtggcaa ataacgcccc tgccgttcaa gcttggcggc     360
aacatgcccg ggcgtggaat gggcgagcgg cttgcggtgg atccaaacaa tgacaacatt     420
ctgtatttcg gcgccccgag cggcaaaggg ctctggagaa gcacagattc cggcgcgacc     480
tggtcccaga tgacgaactt tccggacgta ggcacgtaca ttgcaaatcc cactgacacg     540
accggctatc agagcgatat tcaaggcgtc gtctgggtcg ctttcgacaa gtcttcgtca     600

tcgctcgggc aagcgagtaa gaccattttt gtgggcgtgg cggatcccaa taatccggtc 660
ttctggagca gagacggcgg cgcgacgtgg caggcggtgc cgggtgcgcc gaccggcttc 720
atcccgcaca agggcgtctt tgacccggtc aaccacgtgc tctatattgc caccagcaat 780
acgggtggtc cgtatgacgg gagctccggc gacgtctgga aattctcggt gacctccggg 840
acatggacgc gaatcagccc ggtaccttcg acggacacgg ccaacgacta ctttggttac 900
agcggcctca ctatcgaccg ccagcacccg aacacgataa tggtggcaac ccagatatcg 960
tggtggccgg acaccataat ctttcggagc accgacggcg gtgcgacgtg gacgcggatc 1020
tgggattgga cgagttatcc caatcgaagc ttgcgatatg tgcttgacat ttcggcggag 1080
ccttggctga ccttcggcgt acagccgaat cctcccgtac cgagtccgaa gctcggctgg 1140
atggatgaag cgatggcaat cgatccgttc aactctgatc ggatgctcta cggaacaggc 1200
gcgacgttgt acgcaacaaa tgatctcacg aagtgggact ccggcggcca gattcatatc 1260
gcgccgatgg tcaaaggatt ggaggagacg gcggtaaacg atctcatcag cccgccgtct 1320
ggcgccccgc tcatcagcgc tctcggagac ctcggcggct tcacccacgc cgacgttact 1380
gccgtgccat cgacgatctt cacgtcaccg gtgttcacga ccggcaccag cgtcgactat 1440
gcggaattga atccgtcgat catcgttcgc gctggaagtt tcgatccatc gagccaaccg 1500
aacgacaggc acgtcgcgtt ctcgacagac ggcggcaaga actggttcca aggcagcgaa 1560
cctggcgggg tgacgacggg cggcaccgtc gccgcatcgg ccgacggctc tcgtttcgtc 1620
tgggctcccg gcgatcccgg tcagcctgtg gtgtacgcag tcggatttgg caactcctgg 1680
gctgcttcgc aaggtgttcc cgccaatgcc cagatccgct cagaccgggt gaatccaaag 1740
actttctatg ccctatccaa tggaaccttc tatcgaagca cggacggcgg cgtgacattc 1800
caaccggtcg cggccggtct tccgagcagc ggtgccgtcg gtgtcatgtt ccacgcggtg 1860
cctggaaaag aaggcgatct gtggctcgct gcatcgagcg ggctttacca ctcaaccaat 1920
ggcggcagca gttggtctgc aatcaccggc gtatcctccg cggtgaacgt gggatttggt 1980
aagtctgcgc ccgggtcgtc atacccagcc gtctttgtcg tcggcacgat cggaggcgtt 2040
acgggggcgt accgctccga cgacggtggg acgacctggg tacggatcaa tgatgaccag 2100
caccaatacg gaaattgggg acaagcaatc accggtgacc cgcgaattta cgggcgggtg 2160
tacataggca cgaacggccg tggaattgtc tacggggaca ttggtggtgc gccgtccgga 2220
tcg 2223

<210> 12
<211> 741
<212> PRT
<213> Acidothermus cellulolyticus

<400> 12
Ala Thr Thr Gln Pro Tyr Thr Trp Ser Asn Val Ala Ile Gly Gly Gly
1               5                   10                  15
Gly Phe Val Asp Gly Ile Val Phe Asn Glu Gly Ala Pro Gly Ile Leu
            20                  25                  30
Tyr Val Arg Thr Asp Ile Gly Gly Met Tyr Arg Trp Asp Ala Ala Asn
        35                  40                  45
Gly Arg Trp Ile Pro Leu Leu Asp Trp Val Gly Trp Asn Asn Trp Gly
    50                  55                  60
Tyr Asn Gly Val Val Ser Ile Ala Ala Asp Pro Ile Asn Thr Asn Lys
65                  70                  75                  80
Val Trp Ala Ala Val Gly Met Tyr Thr Asn Ser Trp Asp Pro Asn Asp
                85                  90                  95
Gly Ala Ile Leu Arg Ser Ser Asp Gln Gly Ala Thr Trp Gln Ile Thr
            100                 105                 110
Pro Leu Pro Phe Lys Leu Gly Gly Asn Met Pro Gly Arg Gly Met Gly
        115                 120                 125
Glu Arg Leu Ala Val Asp Pro Asn Asn Asp Asn Ile Leu Tyr Phe Gly
    130                 135                 140
Ala Pro Ser Gly Lys Gly Leu Trp Arg Ser Thr Asp Ser Gly Ala Thr
145                 150                 155                 160
Trp Ser Gln Met Thr Asn Phe Pro Asp Val Gly Thr Tyr Ile Ala Asn
                165                 170                 175

Pro Thr Asp Thr Thr Gly Tyr Gln Ser Asp Ile Gln Gly Val Val Trp
            180                 185                 190
Val Ala Phe Asp Lys Ser Ser Ser Ser Leu Gly Gln Ala Ser Lys Thr
        195                 200                 205
Ile Phe Val Gly Val Ala Asp Pro Asn Asn Pro Val Phe Trp Ser Arg
    210                 215                 220
Asp Gly Gly Ala Thr Trp Gln Ala Val Pro Gly Ala Pro Thr Gly Phe
225                 230                 235                 240
Ile Pro His Lys Gly Val Phe Asp Pro Val Asn His Val Leu Tyr Ile
                245                 250                 255
Ala Thr Ser Asn Thr Gly Gly Pro Tyr Asp Gly Ser Ser Gly Asp Val
            260                 265                 270
Trp Lys Phe Ser Val Thr Ser Gly Thr Trp Thr Arg Ile Ser Pro Val
        275                 280                 285
Pro Ser Thr Asp Thr Ala Asn Asp Tyr Phe Gly Tyr Ser Gly Leu Thr
    290                 295                 300
Ile Asp Arg Gln His Pro Asn Thr Ile Met Val Ala Thr Gln Ile Ser
305                 310                 315                 320
Trp Trp Pro Asp Thr Ile Ile Phe Arg Ser Thr Asp Gly Gly Ala Thr
                325                 330                 335
Trp Thr Arg Ile Trp Asp Trp Thr Ser Tyr Pro Asn Arg Ser Leu Arg
            340                 345                 350
Tyr Val Leu Asp Ile Ser Ala Glu Pro Trp Leu Thr Phe Gly Val Gln
        355                 360                 365
Pro Asn Pro Pro Val Pro Ser Pro Lys Leu Gly Trp Met Asp Glu Ala
    370                 375                 380
Met Ala Ile Asp Pro Phe Asn Ser Asp Arg Met Leu Tyr Gly Thr Gly
385                 390                 395                 400
Ala Thr Leu Tyr Ala Thr Asn Asp Leu Thr Lys Trp Asp Ser Gly Gly
                405                 410                 415
Gln Ile His Ile Ala Pro Met Val Lys Gly Leu Glu Glu Thr Ala Val
            420                 425                 430
Asn Asp Leu Ile Ser Pro Pro Ser Gly Ala Pro Leu Ile Ser Ala Leu
        435                 440                 445
Gly Asp Leu Gly Gly Phe Thr His Ala Asp Val Thr Ala Val Pro Ser
    450                 455                 460
Thr Ile Phe Thr Ser Pro Val Phe Thr Thr Gly Thr Ser Val Asp Tyr
465                 470                 475                 480
Ala Glu Leu Asn Pro Ser Ile Ile Val Arg Ala Gly Ser Phe Asp Pro
                485                 490                 495
Ser Ser Gln Pro Asn Asp Arg His Val Ala Phe Ser Thr Asp Gly Gly
            500                 505                 510
Lys Asn Trp Phe Gln Gly Ser Glu Pro Gly Gly Val Thr Thr Gly Gly
        515                 520                 525
Thr Val Ala Ala Ser Ala Asp Gly Ser Arg Phe Val Trp Ala Pro Gly
    530                 535                 540
Asp Pro Gly Gln Pro Val Val Tyr Ala Val Gly Phe Gly Asn Ser Trp
545                 550                 555                 560
Ala Ala Ser Gln Gly Val Pro Ala Asn Ala Gln Ile Arg Ser Asp Arg
                565                 570                 575
Val Asn Pro Lys Thr Phe Tyr Ala Leu Ser Asn Gly Thr Phe Tyr Arg
            580                 585                 590
Ser Thr Asp Gly Gly Val Thr Phe Gln Pro Val Ala Ala Gly Leu Pro
        595                 600                 605
Ser Ser Gly Ala Val Gly Val Met Phe His Ala Val Pro Gly Lys Glu
    610                 615                 620
Gly Asp Leu Trp Leu Ala Ala Ser Ser Gly Leu Tyr His Ser Thr Asn

625                 630                 635                 640
Gly Gly Ser Ser Trp Ser Ala Ile Thr Gly Val Ser Ser Ala Val Asn
                645                 650                 655
Val Gly Phe Gly Lys Ser Ala Pro Gly Ser Ser Tyr Pro Ala Val Phe
            660                 665                 670
Val Val Gly Thr Ile Gly Gly Val Thr Gly Ala Tyr Arg Ser Asp Asp
        675                 680                 685
Gly Gly Thr Thr Trp Val Arg Ile Asn Asp Asp Gln His Gln Tyr Gly
    690                 695                 700
Asn Trp Gly Gln Ala Ile Thr Gly Asp Pro Arg Ile Tyr Gly Arg Val
705                 710                 715                 720
Tyr Ile Gly Thr Asn Gly Arg Gly Ile Val Tyr Gly Asp Ile Gly Gly
                725                 730                 735
Ala Pro Ser Gly Ser
            740

<210>  13
<211>  1677
<212>  DNA
<213>  Artificial Sequence

<220>
<223>  construct based on Thermobifida fusca

<400>  13
gccggctgct cggtggacta cacggtcaac tcctggggta ccgggttcac cgccaacgtc      60
accatcacca acctcggcag tgcgatcaac ggctggaccc tggagtggga cttccccggc     120
aaccagcagg tgaccaacct gtggaacggg acctacaccc agtccgggca gcacgtgtcg     180
gtcagcaacg ccccgtacaa cgcctccatc ccggccaacg gaacggttga gttcgggttc     240
aacggctcct actcgggcag caacgacatc ccctcctcct tcaagctgaa cggggttacc     300
tgcgacggct cggacgaccc cgaccccgag cccagcccct cccccagccc ttcccccagc     360
cccacagacc cggatgagcc gggcggcccg accaacccgc ccaccaaccc cggcgagaag     420
gtcgacaacc cgttcgaggg cgccaagctg tacgtgaacc cggtctggtc ggccaaggcc     480
gccgctgagc cgggcggttc cgcggtcgcc aacgagtcca ccgctgtctg gctggaccgt     540
atcggcgcca tcgagggcaa cgacagcccg accaccggct ccatgggtct gcgcgaccac     600
ctggaggagg ccgtccgcca gtccggtggc gacccgctga ccatccaggt cgtcatctac     660
aacctgcccg gccgcgactg cgccgcgctg gcctccaacg gtgagctggg tcccgatgaa     720
ctcgaccgct acaagagcga gtacatcgac ccgatcgccg acatcatgtg ggacttcgca     780
gactacgaga acctgcggat cgtcgccatc atcgagatcg actccctgcc caacctcgtc     840
accaacgtgg gcgggaacgg cggcaccgag ctctgcgcct acatgaagca gaacggcggc     900
tacgtcaacg gtgtcggcta cgccctccgc aagctgggcg agatcccgaa cgtctacaac     960
tacatcgacg ccgcccacca cggctggatc ggctgggact ccaacttcgg cccctcggtg    1020
gacatcttct acgaggccgc caacgcctcc ggctccaccg tggactacgt gcacggcttc    1080
atctccaaca cggccaacta ctcggccact gtggagccgt acctggacgt caacggcacc    1140
gttaacggcc agctcatccg ccagtccaag tgggttgact ggaaccagta cgtcgacgag    1200
ctctccttcg tccaggacct gcgtcaggcc ctgatcgcca agggcttccg gtccgacatc    1260
ggtatgctca tcgacacctc ccgcaacggc tggggtggcc cgaaccgtcc gaccggaccg    1320
agctcctcca ccgacctcaa cacctacgtt gacgagagcc gtatcgaccg ccgtatccac    1380
cccggtaact ggtgcaacca ggccggtgcg ggcctcggcg agcggcccac ggtcaacccg    1440
gctcccggtg ttgacgccta cgtctgggtg aagcccccgg gtgagtccga cggcgccagc    1500
gaggagatcc cgaacgacga gggcaagggc ttcgaccgca tgtgcgaccc gacctaccag    1560
ggcaacgccc gcaacggcaa caacccctcg ggtgcgctgc ccaacgcccc catctccggc    1620
cactggttct ctgcccagtt ccgcgagctg ctggccaacg cctacccgcc tctgtaa       1677

<210>  14
<211>  558
<212>  PRT

<213>  Artificial Sequence

<220>
<223>  construct based on Thermobifida fusca

<400>  14
Ala Gly Cys Ser Val Asp Tyr Thr Val Asn Ser Trp Gly Thr Gly Phe
1               5                   10                  15
Thr Ala Asn Val Thr Ile Thr Asn Leu Gly Ser Ala Ile Asn Gly Trp
            20                  25                  30
Thr Leu Glu Trp Asp Phe Pro Gly Asn Gln Gln Val Thr Asn Leu Trp
        35                  40                  45
Asn Gly Thr Tyr Thr Gln Ser Gly Gln His Val Ser Val Ser Asn Ala
    50                  55                  60
Pro Tyr Asn Ala Ser Ile Pro Ala Asn Gly Thr Val Glu Phe Gly Phe
65                  70                  75                  80
Asn Gly Ser Tyr Ser Gly Ser Asn Asp Ile Pro Ser Ser Phe Lys Leu
                85                  90                  95
Asn Gly Val Thr Cys Asp Gly Ser Asp Asp Pro Asp Pro Glu Pro Ser
            100                 105                 110
Pro Ser Pro Ser Pro Ser Pro Ser Pro Thr Asp Pro Asp Glu Pro Gly
        115                 120                 125
Gly Pro Thr Asn Pro Pro Thr Asn Pro Gly Glu Lys Val Asp Asn Pro
    130                 135                 140
Phe Glu Gly Ala Lys Leu Tyr Val Asn Pro Val Trp Ser Ala Lys Ala
145                 150                 155                 160
Ala Ala Glu Pro Gly Gly Ser Ala Val Ala Asn Glu Ser Thr Ala Val
                165                 170                 175
Trp Leu Asp Arg Ile Gly Ala Ile Glu Gly Asn Asp Ser Pro Thr Thr
            180                 185                 190
Gly Ser Met Gly Leu Arg Asp His Leu Glu Glu Ala Val Arg Gln Ser
        195                 200                 205
Gly Gly Asp Pro Leu Thr Ile Gln Val Val Ile Tyr Asn Leu Pro Gly
    210                 215                 220
Arg Asp Cys Ala Ala Leu Ala Ser Asn Gly Glu Leu Gly Pro Asp Glu
225                 230                 235                 240
Leu Asp Arg Tyr Lys Ser Glu Tyr Ile Asp Pro Ile Ala Asp Ile Met
                245                 250                 255
Trp Asp Phe Ala Asp Tyr Glu Asn Leu Arg Ile Val Ala Ile Ile Glu
            260                 265                 270
Ile Asp Ser Leu Pro Asn Leu Val Thr Asn Val Gly Gly Asn Gly Gly
        275                 280                 285
Thr Glu Leu Cys Ala Tyr Met Lys Gln Asn Gly Gly Tyr Val Asn Gly
    290                 295                 300
Val Gly Tyr Ala Leu Arg Lys Leu Gly Glu Ile Pro Asn Val Tyr Asn
305                 310                 315                 320
Tyr Ile Asp Ala Ala His His Gly Trp Ile Gly Trp Asp Ser Asn Phe
                325                 330                 335
Gly Pro Ser Val Asp Ile Phe Tyr Glu Ala Ala Asn Ala Ser Gly Ser
            340                 345                 350
Thr Val Asp Tyr Val His Gly Phe Ile Ser Asn Thr Ala Asn Tyr Ser
        355                 360                 365
Ala Thr Val Glu Pro Tyr Leu Asp Val Asn Gly Thr Val Asn Gly Gln
    370                 375                 380
Leu Ile Arg Gln Ser Lys Trp Val Asp Trp Asn Gln Tyr Val Asp Glu
385                 390                 395                 400
Leu Ser Phe Val Gln Asp Leu Arg Gln Ala Leu Ile Ala Lys Gly Phe

                    405                 410                 415
Arg Ser Asp Ile Gly Met Leu Ile Asp Thr Ser Arg Asn Gly Trp Gly
            420                 425                 430
Gly Pro Asn Arg Pro Thr Gly Pro Ser Ser Ser Thr Asp Leu Asn Thr
        435                 440                 445
Tyr Val Asp Glu Ser Arg Ile Asp Arg Arg Ile His Pro Gly Asn Trp
    450                 455                 460
Cys Asn Gln Ala Gly Ala Gly Leu Gly Glu Arg Pro Thr Val Asn Pro
465                 470                 475                 480
Ala Pro Gly Val Asp Ala Tyr Val Trp Val Lys Pro Pro Gly Glu Ser
                485                 490                 495
Asp Gly Ala Ser Glu Glu Ile Pro Asn Asp Glu Gly Lys Gly Phe Asp
            500                 505                 510
Arg Met Cys Asp Pro Thr Tyr Gln Gly Asn Ala Arg Asn Gly Asn Asn
        515                 520                 525
Pro Ser Gly Ala Leu Pro Asn Ala Pro Ile Ser Gly His Trp Phe Ser
    530                 535                 540
Ala Gln Phe Arg Glu Leu Leu Ala Asn Ala Tyr Pro Pro Leu
545                 550                 555

<210>  15
<211>  1293
<212>  DNA
<213>  Artificial Sequence

<220>
<223>  construct based on Thermobifida fusca

<400>  15
gccggtctca ccgccacagt caccaaagaa tcctcgtggg acaacggcta ctccgcgtcc      60
gtcaccgtcc gcaacgacac ctcgagcacc gtctcccagt gggaggtcgt cctcaccctg     120
cccggcggca ctacagtggc ccaggtgtgg aacgcccagc acaccagcag cggcaactcc     180
cacaccttca ccggggtttc ctggaacagc accatcccgc ccggaggcac cgcctcttcc     240
ggcttcatcg cttccggcag cggcgaaccc acccactgca ccatcaacgg cgccccctgc     300
gacgaaggct ccgagccggg cggccccggc ggtcccggaa ccccctcccc cgaccccggc     360
acgcagcccg gcaccggcac cccggtcgag cggtacggca aagtccaggt ctgcggcacc     420
cagctctgcg acgagcacgg caacccggtc caactgcgcg gcatgagcac ccacggcatc     480
cagtggttcg accactgcct gaccgacagc tcgctggacg ccctggccta cgactggaag     540
gccgacatca tccgcctgtc catgtacatc caggaagacg gctacgagac caacccgcgc     600
ggcttcaccg accggatgca ccagctcatc gacatggcca cggcgcgcgg cctgtacgtg     660
atcgtggact ggcacatcct caccccgggc gatccccact acaacctgga ccgggccaag     720
accttcttcg cggaaatcgc ccagcgccac gccagcaaga ccaacgtgct ctacgagatc     780
gccaacgaac ccaacggagt gagctgggcc tccatcaaga gctacgccga agaggtcatc     840
ccggtgatcc gccagcgcga ccccgactcg gtgatcatcg tgggcacccg cggctggtcg     900
tcgctcggcg tctccgaagg ctccggcccc gccgagatcg cggccaaccc ggtcaacgcc     960
tccaacatca tgtacgcctt ccacttctac gcggcctcgc accgcgacaa ctacctcaac    1020
gcgctgcgtg aggcctccga gctgttcccg gtcttcgtca ccgagttcgg caccgagacc    1080
tacaccggtg acggcgccaa cgacttccag atggccgacc gctacatcga cctgatggcg    1140
gaacggaaga tcgggtggac caagtggaac tactcggacg acttccgttc cggcgcggtc    1200
ttccagccgg gcacctgcgc gtccggcggc ccgtggagcg gttcgtcgct gaaggcgtcc    1260
ggacagtggg tgcggagcaa gctccagtcc tga                                 1293

<210>  16
<211>  430
<212>  PRT
<213>  Artificial Sequence

<220>
<223>  construct based on Thermobifida fusca

<400>  16
Ala Gly Leu Thr Ala Thr Val Thr Lys Glu Ser Ser Trp Asp Asn Gly
1               5                   10                  15
Tyr Ser Ala Ser Val Thr Val Arg Asn Asp Thr Ser Ser Thr Val Ser
            20                  25                  30
Gln Trp Glu Val Val Leu Thr Leu Pro Gly Gly Thr Thr Val Ala Gln
        35                  40                  45
Val Trp Asn Ala Gln His Thr Ser Ser Gly Asn Ser His Thr Phe Thr
    50                  55                  60
Gly Val Ser Trp Asn Ser Thr Ile Pro Pro Gly Gly Thr Ala Ser Ser
65                  70                  75                  80
Gly Phe Ile Ala Ser Gly Ser Gly Glu Pro Thr His Cys Thr Ile Asn
                85                  90                  95
Gly Ala Pro Cys Asp Glu Gly Ser Glu Pro Gly Gly Pro Gly Gly Pro
            100                 105                 110
Gly Thr Pro Ser Pro Asp Pro Gly Thr Gln Pro Gly Thr Gly Thr Pro
        115                 120                 125
Val Glu Arg Tyr Gly Lys Val Gln Val Cys Gly Thr Gln Leu Cys Asp
    130                 135                 140
Glu His Gly Asn Pro Val Gln Leu Arg Gly Met Ser Thr His Gly Ile
145                 150                 155                 160
Gln Trp Phe Asp His Cys Leu Thr Asp Ser Ser Leu Asp Ala Leu Ala
                165                 170                 175
Tyr Asp Trp Lys Ala Asp Ile Ile Arg Leu Ser Met Tyr Ile Gln Glu
            180                 185                 190
Asp Gly Tyr Glu Thr Asn Pro Arg Gly Phe Thr Asp Arg Met His Gln
        195                 200                 205
Leu Ile Asp Met Ala Thr Ala Arg Gly Leu Tyr Val Ile Val Asp Trp
    210                 215                 220
His Ile Leu Thr Pro Gly Asp Pro His Tyr Asn Leu Asp Arg Ala Lys
225                 230                 235                 240
Thr Phe Phe Ala Glu Ile Ala Gln Arg His Ala Ser Lys Thr Asn Val
                245                 250                 255
Leu Tyr Glu Ile Ala Asn Glu Pro Asn Gly Val Ser Trp Ala Ser Ile
            260                 265                 270
Lys Ser Tyr Ala Glu Glu Val Ile Pro Val Ile Arg Gln Arg Asp Pro
        275                 280                 285
Asp Ser Val Ile Ile Val Gly Thr Arg Gly Trp Ser Ser Leu Gly Val
    290                 295                 300
Ser Glu Gly Ser Gly Pro Ala Glu Ile Ala Ala Asn Pro Val Asn Ala
305                 310                 315                 320
Ser Asn Ile Met Tyr Ala Phe His Phe Tyr Ala Ala Ser His Arg Asp
                325                 330                 335
Asn Tyr Leu Asn Ala Leu Arg Glu Ala Ser Glu Leu Phe Pro Val Phe
            340                 345                 350
Val Thr Glu Phe Gly Thr Glu Thr Tyr Thr Gly Asp Gly Ala Asn Asp
        355                 360                 365
Phe Gln Met Ala Asp Arg Tyr Ile Asp Leu Met Ala Glu Arg Lys Ile
    370                 375                 380
Gly Trp Thr Lys Trp Asn Tyr Ser Asp Asp Phe Arg Ser Gly Ala Val
385                 390                 395                 400
Phe Gln Pro Gly Thr Cys Ala Ser Gly Gly Pro Trp Ser Gly Ser Ser
                405                 410                 415
Leu Lys Ala Ser Gly Gln Trp Val Arg Ser Lys Leu Gln Ser

            420                 425                 430

<210> 17
<211> 2656
<212> DNA
<213> Artificial Sequence

<220>
<223> fusion construct

<400> 17
atgtatcgga agttggccgt catctcggcc ttcttggcca cagctcgtgc tcagtcggcc 60
tgcactctcc aatcggagac tcacccgcct ctgacatggc agaaatgctc gtctggtggc 120
acttgcactc aacagacagg ctccgtggtc atcgacgcca actggcgctg gactcacgct 180
acgaacagca gcacgaactg ctacgatggc aacacttgga gctcgaccct atgtcctgac 240
aacgagacct gcgcgaagaa ctgctgtctg gacggtgccg cctacgcgtc cacgtacgga 300
gttaccacga gcggtaacag cctctccatt ggctttgtca cccagtctgc gcagaagaac 360
gttggcgctc gcctttacct tatggcgagc gacacgacct accaggaatt caccctgctt 420
ggcaacgagt tctctttcga tgttgatgtt tcgcagctgc cgtaagtgac ttaccatgaa 480
cccctgacgt atcttcttgt gggctcccag ctgactggcc aatttaaggt gcggcttgaa 540
cggagctctc tacttcgtgt ccatggacgc ggatggtggc gtgagcaagt atcccaccaa 600
caccgctggc gccaagtacg gcacggggta ctgtgacagc cagtgtcccc gcgatctgaa 660
gttcatcaat ggccaggcca acgttgaggg ctgggagccg tcatccaaca acgcaaacac 720
gggcattgga ggacacggaa gctgctgctc tgagatggat atctgggagg ccaactccat 780
ctccgaggct cttacccccc acccttgcac gactgtcggc caggagatct gcgagggtga 840
tgggtgcggc ggaacttact ccgataacag atatggcggc acttgcgatc ccgatggctg 900
cgactggaac ccataccgcc tgggcaacac cagcttctac ggccctggct caagctttac 960
cctcgatacc accaagaaat tgaccgttgt cacccagttc gagacgtcgg gtgccatcaa 1020
ccgatactat gtccagaatg gcgtcacttt ccagcagccc aacgccgagc ttggtagtta 1080
ctctggcaac gagctcaacg atgattactg cacagctgag gaggcagaat tcggcggatc 1140
ctctttctca gacaagggcg gcctgactca gttcaagaag gctacctctg gcggcatggt 1200
tctggtcatg agtctgtggg atgatgtgag tttgatggac aaacatgcgc gttgacaaag 1260
agtcaagcag ctgactgaga tgttacagta ctacgccaac atgctgtggc tggactccac 1320
ctacccgaca aacgagacct cctccacacc cggtgccgtg cgcggaagct gctccaccag 1380
ctccggtgtc cctgctcagg tcgaatctca gtctcccaac gccaaggtca ccttctccaa 1440
catcaagttc ggacccattg gcagcaccgg caaccctagc ggcggcaacc ctcccggcgg 1500
aaacccgcct ggcaccacca ccacccgccg cccagccact accactggaa gctctcccgg 1560
acctactagt aagcgggcgg gcggcggcta ttggcacacg agcggccggg agatcctgga 1620
cgcgaacaac gtgccggtac ggatcgccgg catcaactgg tttgggttcg aaacctgcaa 1680
ttacgtcgtg cacggtctct ggtcacgcga ctaccgcagc atgctcgacc agataaagtc 1740
gctcggctac aacacaatcc ggctgccgta ctctgacgac attctcaagc cgggcaccat 1800
gccgaacagc atcaattttt accagatgaa tcaggacctg cagggtctga cgtccttgca 1860
ggtcatggac aaaatcgtcg cgtacgccgg tcagatcggc ctgcgcatca ttcttgaccg 1920
ccaccgaccg gattgcagcg ggcagtcggc gctgtggtac acgagcagcg tctcggaggc 1980
tacgtggatt tccgacctgc aagcgctggc gcagcgctac aagggaaacc cgacggtcgt 2040
cggctttgac ttgcacaacg agccgcatga cccggcctgc tggggctgcg gcgatccgag 2100
catcgactgg cgattggccg ccgagcgggc cggaaacgcc gtgctctcgg tgaatccgaa 2160
cctgctcatt ttcgtcgaag gtgtgcagag ctacaacgga gactcctact ggtggggcgg 2220
caacctgcaa ggagccggcc agtacccggt cgtgctgaac gtgccgaacc gcctggtgta 2280
ctcggcgcac gactacgcga cgagcgtcta cccgcagacg tggttcagcg atccgacctt 2340
ccccaacaac atgcccggca tctggaacaa gaactgggga tacctcttca atcagaacat 2400
tgcaccggta tggctgggcg aattcggtac gacactgcaa tccacgaccg accagacgtg 2460
gctgaagacg ctcgtccagt acctacggcc gaccgcgcaa tacggtgcgg acagcttcca 2520
gtggaccttc tggtcctgga accccgattc cggcgacaca ggaggaattc tcaaggatga 2580
ctggcagacg gtcgacacag taaaagacgg ctatctcgcg ccgatcaagt cgtcgatttt 2640
cgatcctgtc ggctaa 2656

<210> 18
<211> 841
<212> PRT
<213> Artificial Sequence

<220>
<223> fusion construct

<400> 18
Met Tyr Arg Lys Leu Ala Val Ile Ser Ala Phe Leu Ala Thr Ala Arg
1               5                   10                  15
Ala Gln Ser Ala Cys Thr Leu Gln Ser Glu Thr His Pro Pro Leu Thr
            20                  25                  30
Trp Gln Lys Cys Ser Ser Gly Gly Thr Cys Thr Gln Gln Thr Gly Ser
        35                  40                  45
Val Val Ile Asp Ala Asn Trp Arg Trp Thr His Ala Thr Asn Ser Ser
    50                  55                  60
Thr Asn Cys Tyr Asp Gly Asn Thr Trp Ser Ser Thr Leu Cys Pro Asp
65                  70                  75                  80
Asn Glu Thr Cys Ala Lys Asn Cys Cys Leu Asp Gly Ala Ala Tyr Ala
                85                  90                  95
Ser Thr Tyr Gly Val Thr Thr Ser Gly Asn Ser Leu Ser Ile Gly Phe
            100                 105                 110
Val Thr Gln Ser Ala Gln Lys Asn Val Gly Ala Arg Leu Tyr Leu Met
        115                 120                 125
Ala Ser Asp Thr Thr Tyr Gln Glu Phe Thr Leu Leu Gly Asn Glu Phe
    130                 135                 140
Ser Phe Asp Val Asp Val Ser Gln Leu Pro Cys Gly Leu Asn Gly Ala
145                 150                 155                 160
Leu Tyr Phe Val Ser Met Asp Ala Asp Gly Gly Val Ser Lys Tyr Pro
                165                 170                 175
Thr Asn Thr Ala Gly Ala Lys Tyr Gly Thr Gly Tyr Cys Asp Ser Gln
            180                 185                 190
Cys Pro Arg Asp Leu Lys Phe Ile Asn Gly Gln Ala Asn Val Glu Gly
        195                 200                 205
Trp Glu Pro Ser Ser Asn Asn Ala Asn Thr Gly Ile Gly Gly His Gly
    210                 215                 220
Ser Cys Cys Ser Glu Met Asp Ile Trp Glu Ala Asn Ser Ile Ser Glu
225                 230                 235                 240
Ala Leu Thr Pro His Pro Cys Thr Thr Val Gly Gln Glu Ile Cys Glu
                245                 250                 255
Gly Asp Gly Cys Gly Gly Thr Tyr Ser Asp Asn Arg Tyr Gly Gly Thr
            260                 265                 270
Cys Asp Pro Asp Gly Cys Asp Trp Asn Pro Tyr Arg Leu Gly Asn Thr
        275                 280                 285
Ser Phe Tyr Gly Pro Gly Ser Ser Phe Thr Leu Asp Thr Thr Lys Lys
    290                 295                 300
Leu Thr Val Val Thr Gln Phe Glu Thr Ser Gly Ala Ile Asn Arg Tyr
305                 310                 315                 320
Tyr Val Gln Asn Gly Val Thr Phe Gln Gln Pro Asn Ala Glu Leu Gly
                325                 330                 335
Ser Tyr Ser Gly Asn Glu Leu Asn Asp Asp Tyr Cys Thr Ala Glu Glu
            340                 345                 350
Ala Glu Phe Gly Gly Ser Ser Phe Ser Asp Lys Gly Gly Leu Thr Gln
        355                 360                 365
Phe Lys Lys Ala Thr Ser Gly Gly Met Val Leu Val Met Ser Leu Trp
    370                 375                 380

Asp Asp Tyr Tyr Ala Asn Met Leu Trp Leu Asp Ser Thr Tyr Pro Thr
385                 390                 395                 400
Asn Glu Thr Ser Ser Thr Pro Gly Ala Val Arg Gly Ser Cys Ser Thr
                405                 410                 415
Ser Ser Gly Val Pro Ala Gln Val Glu Ser Gln Ser Pro Asn Ala Lys
            420                 425                 430
Val Thr Phe Ser Asn Ile Lys Phe Gly Pro Ile Gly Ser Thr Gly Asn
        435                 440                 445
Pro Ser Gly Gly Asn Pro Pro Gly Gly Asn Pro Pro Gly Thr Thr Thr
    450                 455                 460
Thr Arg Arg Pro Ala Thr Thr Thr Gly Ser Ser Pro Gly Pro Thr Ser
465                 470                 475                 480
Lys Arg Ala Gly Gly Gly Tyr Trp His Thr Ser Gly Arg Glu Ile Leu
                485                 490                 495
Asp Ala Asn Asn Val Pro Val Arg Ile Ala Gly Ile Asn Trp Phe Gly
            500                 505                 510
Phe Glu Thr Cys Asn Tyr Val Val His Gly Leu Trp Ser Arg Asp Tyr
        515                 520                 525
Arg Ser Met Leu Asp Gln Ile Lys Ser Leu Gly Tyr Asn Thr Ile Arg
    530                 535                 540
Leu Pro Tyr Ser Asp Asp Ile Leu Lys Pro Gly Thr Met Pro Asn Ser
545                 550                 555                 560
Ile Asn Phe Tyr Gln Met Asn Gln Asp Leu Gln Gly Leu Thr Ser Leu
                565                 570                 575
Gln Val Met Asp Lys Ile Val Ala Tyr Ala Gly Gln Ile Gly Leu Arg
            580                 585                 590
Ile Ile Leu Asp Arg His Arg Pro Asp Cys Ser Gly Gln Ser Ala Leu
        595                 600                 605
Trp Tyr Thr Ser Ser Val Ser Glu Ala Thr Trp Ile Ser Asp Leu Gln
    610                 615                 620
Ala Leu Ala Gln Arg Tyr Lys Gly Asn Pro Thr Val Val Gly Phe Asp
625                 630                 635                 640
Leu His Asn Glu Pro His Asp Pro Ala Cys Trp Gly Cys Gly Asp Pro
                645                 650                 655
Ser Ile Asp Trp Arg Leu Ala Ala Glu Arg Ala Gly Asn Ala Val Leu
            660                 665                 670
Ser Val Asn Pro Asn Leu Leu Ile Phe Val Glu Gly Val Gln Ser Tyr
        675                 680                 685
Asn Gly Asp Ser Tyr Trp Trp Gly Gly Asn Leu Gln Gly Ala Gly Gln
    690                 695                 700
Tyr Pro Val Val Leu Asn Val Pro Asn Arg Leu Val Tyr Ser Ala His
705                 710                 715                 720
Asp Tyr Ala Thr Ser Val Tyr Pro Gln Thr Trp Phe Ser Asp Pro Thr
                725                 730                 735
Phe Pro Asn Asn Met Pro Gly Ile Trp Asn Lys Asn Trp Gly Tyr Leu
            740                 745                 750
Phe Asn Gln Asn Ile Ala Pro Val Trp Leu Gly Glu Phe Gly Thr Thr
        755                 760                 765
Leu Gln Ser Thr Thr Asp Gln Thr Trp Leu Lys Thr Leu Val Gln Tyr
    770                 775                 780
Leu Arg Pro Thr Ala Gln Tyr Gly Ala Asp Ser Phe Gln Trp Thr Phe
785                 790                 795                 800
Trp Ser Trp Asn Pro Asp Ser Gly Asp Thr Gly Gly Ile Leu Lys Asp
                805                 810                 815
Asp Trp Gln Thr Val Asp Thr Val Lys Asp Gly Tyr Leu Ala Pro Ile
            820                 825                 830
Lys Ser Ser Ile Phe Asp Pro Val Gly

        835                 840

<210> 19
<211> 10239
<212> DNA
<213> Artificial Sequence

<220>
<223> pTrex plasmid

<400> 19
aagcttaact agtacttctc gagctctgta catgtccggt cgcgacgtac gcgtatcgat 60
ggcgccagct gcaggcggcc gcctgcagcc acttgcagtc ccgtggaatt ctcacggtga 120
atgtaggcct tttgtagggt aggaattgtc actcaagcac ccccaacctc cattacgcct 180
cccccataga gttcccaatc agtgagtcat ggcactgttc tcaaatagat tggggagaag 240
ttgacttccg cccagagctg aaggtcgcac aaccgcatga tatagggtcg gcaacggcaa 300
aaaagcacgt ggctcaccga aaagcaagat gtttgcgatc taacatccag gaacctggat 360
acatccatca tcacgcacga ccactttgat ctgctggtaa actcgtattc gccctaaacc 420
gaagtgacgt ggtaaatcta cacgtgggcc cctttcggta tactgcgtgt gtcttctcta 480
ggtgccattc ttttcccttc ctctagtgtt gaattgtttg tgttggagtc cgagctgtaa 540
ctacctctga atctctggag aatggtggac taacgactac cgtgcacctg catcatgtat 600
ataatagtga tcctgagaag gggggtttgg agcaatgtgg gactttgatg gtcatcaaac 660
aaagaacgaa gacgcctctt ttgcaaagtt ttgtttcggc tacggtgaag aactggatac 720
ttgttgtgtc ttctgtgtat ttttgtggca acaagaggcc agagacaatc tattcaaaca 780
ccaagcttgc tcttttgagc tacaagaacc tgtggggtat atatctagag ttgtgaagtc 840
ggtaatcccg ctgtatagta atacgagtcg catctaaata ctccgaagct gctgcgaacc 900
cggagaatcg agatgtgctg gaaagcttct agcgagcggc taaattagca tgaaaggcta 960
tgagaaattc tggagacggc ttgttgaatc atggcgttcc attcttcgac aagcaaagcg 1020
ttccgtcgca gtagcaggca ctcattcccg aaaaaactcg gagattccta agtagcgatg 1080
gaaccggaat aatataatag gcaatacatt gagttgcctc gacggttgca atgcaggggt 1140
actgagcttg gacataactg ttccgtaccc cacctcttct caacctttgg cgtttccctg 1200
attcagcgta cccgtacaag tcgtaatcac tattaaccca gactgaccgg acgtgttttg 1260
cccttcattt ggagaaataa tgtcattgcg atgtgtaatt tgcctgcttg accgactggg 1320
gctgttcgaa gcccgaatgt aggattgtta tccgaactct gctcgtagag gcatgttgtg 1380
aatctgtgtc gggcaggaca cgcctcgaag gttcacggca agggaaacca ccgatagcag 1440
tgtctagtag caacctgtaa agccgcaatg cagcatcact ggaaaataca aaccaatggc 1500
taaaagtaca taagttaatg cctaaagaag tcatatacca gcggctaata attgtacaat 1560
caagtggcta aacgtaccgt aatttgccaa cggcttgtgg ggttgcagaa gcaacggcaa 1620
agccccactt ccccacgttt gtttcttcac tcagtccaat ctcagctggt gatcccccaa 1680
ttgggtcgct tgtttgttcc ggtgaagtga aagaagacag aggtaagaat gtctgactcg 1740
gagcgttttg catacaacca agggcagtga tggaagacag tgaaatgttg acattcaagg 1800
agtatttagc cagggatgct tgagtgtatc gtgtaaggag gtttgtctgc cgatacgacg 1860
aatactgtat agtcacttct gatgaagtgg tccatattga aatgtaagtc ggcactgaac 1920
aggcaaaaga ttgagttgaa actgcctaag atctcgggcc ctcgggcctt cggcctttgg 1980
gtgtacatgt ttgtgctccg ggcaaatgca aagtgtggta ggatcgaaca cactgctgcc 2040
tttaccaagc agctgagggt atgtgatagg caaatgttca ggggccactg catggtttcg 2100
aatagaaaga gaagcttagc caagaacaat agccgataaa gatagcctca ttaaacggaa 2160
tgagctagta ggcaaagtca gcgaatgtgt atatataaag gttcgaggtc cgtgcctccc 2220
tcatgctctc cccatctact catcaactca gatcctccag gagacttgta caccatcttt 2280
tgaggcacag aaacccaata gtcaaccgcg gactgcgcat catgtatcgg aagttggccg 2340
tcatctcggc cttcttggcc acagctcgtg ctcagtcggc ctgcactctc caatcggaga 2400
ctcacccgcc tctgacatgg cagaaatgct cgtctggtgg cacttgcact caacagacag 2460
gctccgtggt catcgacgcc aactggcgct ggactcacgc tacgaacagc agcacgaact 2520
gctacgatgg caacacttgg agctcgaccc tatgtcctga caacgagacc tgcgcgaaga 2580
actgctgtct ggacggtgcc gcctacgcgt ccacgtacgg agttaccacg agcggtaaca 2640
gcctctccat tggctttgtc acccagtctg cgcagaagaa cgttggcgct cgcctttacc 2700
ttatggcgag cgacacgacc taccaggaat tcaccctgct tggcaacgag ttctctttcg 2760

atgttgatgt ttcgcagctg ccgtaagtga cttaccatca acccctgacg tatcttcttg 2820
tgggctccca gctgactggc caatttaagg tgcggcttga acggagctct ctacttcgtg 2880
tccatggacg cggatggtgg cgtgagcaag tatcccacca acaccgctgg cgccaagtac 2940
ggcacggggt actgtgacag ccagtgtccc cgcgatctga agttcatcaa tggccaggcc 3000
aacgttgagg gctgggagcc gtcatccaac aacgcaaaca cgggcattgg aggacacgga 3060
agctgctgct ctgagatgga tatctgggag gccaactcca tctccgaggc tcttaccccc 3120
cacccttgca cgactgtcgg ccaggagatc tgcgagggtg atgggtgcgg cggaacttac 3180
tccgataaca gatatggcgg cacttgcgat cccgatggct gcgactggaa cccataccgc 3240
ctgggcaaca ccagcttcta cggccctggc tcaagcttta ccctcgatac caccaagaaa 3300
ttgaccgttg tcacccagtt cgagacgtcg ggtgccatca accgatacta tgtccagaat 3360
ggcgtcactt tccagcagcc caacgccgag cttggtagtt actctggcaa cgagctcaac 3420
gatgattact gcacagctga ggaggcagaa ttcggcggat cctctttctc agacaagggc 3480
ggcctgactc agttcaagaa ggctacctct ggcggcatgg ttctggtcat gagtctgtgg 3540
gatgatgtga gtttgatgga caaacatgcg cgttgacaaa gagtcaagca gctgactgag 3600
atgttacagt actacgccaa catgctgtgg ctggactcca cctacccgac aaacgagacc 3660
tcctccacac ccggtgccgt gcgcggaagc tgctccacca gctccggtgt ccctgctcag 3720
gtcgaatctc agtctcccaa cgccaaggtc accttctcca acatcaagtt cggacccatt 3780
ggcagcaccg gcaaccctag cggcggcaac cctcccggcg gaaacccgcc tggcaccacc 3840
accacccgcc gcccagccac taccactgga agctctcccg gacctactag taagcggata 3900
aggcgcgccg cgcgccagct ccgtgcgaaa gcctgacgca ccggtagatt cttggtgagc 3960
ccgtatcatg acggcggcgg gagctacatg gccccgggtg atttattttt tttgtatcta 4020
cttctgaccc ttttcaaata tacggtcaac tcatctttca ctggagatgc ggcctgcttg 4080
gtattgcgat gttgtcagct tggcaaattg tggctttcga aaacacaaaa cgattcctta 4140
gtagccatgc attttaagat aacggaatag aagaaagagg aaattaaaaa aaaaaaaaaa 4200
acaaacatcc cgttcataac ccgtagaatc gccgctcttc gtgtatccca gtaccagttt 4260
attttgaata gctcgcccgc tggagagcat cctgaatgca agtaacaacc gtagaggctg 4320
acacggcagg tgttgctagg gagcgtcgtg ttctacaagg ccagacgtct tcgcggttga 4380
tatatatgta tgtttgactg caggctgctc agcgacgaca gtcaagttcg ccctcgctgc 4440
ttgtgcaata atcgcagtgg ggaagccaca ccgtgactcc catctttcag taaagctctg 4500
ttggtgttta tcagcaatac acgtaattta aactcgttag catggggctg atagcttaat 4560
taccgtttac cagtgccgcg gttctgcagc tttccttggc ccgtaaaatt cggcgaagcc 4620
agccaatcac cagctaggca ccagctaaac cctataatta gtctcttatc aacaccatcc 4680
gctcccccgg gatcaatgag gagaatgagg gggatgcggg gctaaacaag cctacataac 4740
cctcatgcca actcccagtt tacactcgtc gagccaacat cctgactata agctaacaca 4800
gaatgcctca atcctgggaa gaactggccg ctgataagcg cgcccgcctc gcaaaaacca 4860
tccctgatga atggaaagtc cagacgctgc ctgcggaaga cagcgttatt gatttcccaa 4920
agaaatcggg gatcctttca gaggccgaac tgaagatcac agaggcctcc gctgcagatc 4980
ttgtgtccaa gctggcggcc ggagagttga cctcggtgga agttacgcta gcattctgta 5040
aacgggcagc aatcgcccag cagttagtag ggtcccctct acctctcagg gagatgtaac 5100
aacgccacct tatgggacta tcaagctgac gctggcttct gtgcagacaa actgcgccca 5160
cgagttcttc cctgacgccg ctctcgcgca ggcaagggaa ctcgatgaat actacgcaaa 5220
gcacaagaga cccgttggtc cactccatgg cctccccatc tctctcaaag accagcttcg 5280
agtcaaggta caccgttgcc cctaagtcgt tagatgtccc tttttgtcag ctaacatatg 5340
ccaccagggc tacgaaacat caatgggcta catctcatgg ctaaacaagt acgacgaagg 5400
ggactcggtt ctgacaacca tgctccgcaa agccggtgcc gtcttctacg tcaagacctc 5460
tgtcccgcag accctgatgg tctgcgagac agtcaacaac atcatcgggc gcaccgtcaa 5520
cccacgcaac aagaactggt cgtgcggcgg cagttctggt ggtgagggtg cgatcgttgg 5580
gattcgtggt ggcgtcatcg gtgtaggaac ggatatcggt ggctcgattc gagtgccggc 5640
cgcgttcaac ttcctgtacg gtctaaggcc gagtcatggg cggctgccgt atgcaaagat 5700
ggcgaacagc atggagggtc aggagacggt gcacagcgtt gtcgggccga ttacgcactc 5760
tgttgagggt gagtccttcg cctcttcctt cttttcctgc tctataccag gcctccactg 5820
tcctcctttc ttgcttttta tactatatac gagaccggca gtcactgatg aagtatgtta 5880
gacctccgcc tcttcaccaa atccgtcctc ggtcaggagc catggaaata cgactccaag 5940
gtcatcccca tgccctggcg ccagtccgag tcggacatta ttgcctccaa gatcaagaac 6000
ggcgggctca atatcggcta ctacaacttc gacggcaatg tccttccaca ccctcctatc 6060
ctgcgcggcg tggaaaccac cgtcgccgca ctcgccaaag ccggtcacac cgtgaccccg 6120
tggacgccat acaagcacga tttcggccac gatctcatct cccatatcta cgcggctgac 6180

ggcagcgccg acgtaatgcg cgatatcagt gcatccggcg agccggcgat tccaaatatc 6240
aaagacctac tgaacccgaa catcaaagct gttaacatga acgagctctg ggacacgcat 6300
ctccagaagt ggaattacca gatggagtac cttgagaaat ggcgggaggc tgaagaaaag 6360
gccgggaagg aactggacgc catcatcgcg ccgattacgc ctaccgctgc ggtacggcat 6420
gaccagttcc ggtactatgg gtatgcctct gtgatcaacc tgctggattt cacgagcgtg 6480
gttgttccgg ttacctttgc ggataagaac atcgataaga agaatgagag tttcaaggcg 6540
gttagtgagc ttgatgccct cgtgcaggaa gagtatgatc cggaggcgta ccatggggca 6600
ccggttgcag tgcaggttat cggacggaga ctcagtgaag agaggacgtt ggcgattgca 6660
gaggaagtgg ggaagttgct gggaaatgtg gtgactccat agctaataag tgtcagatag 6720
caatttgcac aagaaatcaa taccagcaac tgtaaataag cgctgaagtg accatgccat 6780
gctacgaaag agcagaaaaa aacctgccgt agaaccgaag agatatgaca cgcttccatc 6840
tctcaaagga agaatccctt cagggttgcg tttccagtct agacacgtat aacggcacaa 6900
gtgtctctca ccaaatgggt tatatctcaa atgtgatcta aggatggaaa gcccagaatc 6960
taggcctatt aatattccgg agtatacgta gccggctaac gttaacaacc ggtacctcta 7020
gaactatagc tagcatgcgc aaatttaaag cgctgatatc gatcgcgcgc agatccatat 7080
atagggcccg ggttataatt acctcaggtc gacgtcccat ggccattcga attcgtaatc 7140
atggtcatag ctgtttcctg tgtgaaattg ttatccgctc acaattccac acaacatacg 7200
agccggaagc ataaagtgta aagcctgggg tgcctaatga gtgagctaac tcacattaat 7260
tgcgttgcgc tcactgcccg ctttccagtc gggaaacctg tcgtgccagc tgcattaatg 7320
aatcggccaa cgcgcgggga gaggcggttt gcgtattggg cgctcttccg cttcctcgct 7380
cactgactcg ctgcgctcgg tcgttcggct gcggcgagcg gtatcagctc actcaaaggc 7440
ggtaatacgg ttatccacag aatcagggga taacgcagga aagaacatgt gagcaaaagg 7500
ccagcaaaag gccaggaacc gtaaaaaggc cgcgttgctg gcgtttttcc ataggctccg 7560
cccccctgac gagcatcaca aaaatcgacg ctcaagtcag aggtggcgaa acccgacagg 7620
actataaaga taccaggcgt ttccccctgg aagctccctc gtgcgctctc ctgttccgac 7680
cctgccgctt accggatacc tgtccgcctt tctcccttcg ggaagcgtgg cgctttctca 7740
tagctcacgc tgtaggtatc tcagttcggt gtaggtcgtt cgctccaagc tgggctgtgt 7800
gcacgaaccc cccgttcagc ccgaccgctg cgccttatcc ggtaactatc gtcttgagtc 7860
caacccggta agacacgact tatcgccact ggcagcagcc actggtaaca ggattagcag 7920
agcgaggtat gtaggcggtg ctacagagtt cttgaagtgg tggcctaact acggctacac 7980
tagaagaaca gtatttggta tctgcgctct gctgaagcca gttaccttcg gaaaaagagt 8040
tggtagctct tgatccggca aacaaaccac cgctggtagc ggtggttttt ttgtttgcaa 8100
gcagcagatt acgcgcagaa aaaaaggatc tcaagaagat cctttgatct tttctacggg 8160
gtctgacgct cagtggaacg aaaactcacg ttaagggatt ttggtcatga gattatcaaa 8220
aaggatcttc acctagatcc ttttaaatta aaaatgaagt tttaaatcaa tctaaagtat 8280
atatgagtaa acttggtctg acagttacca atgcttaatc agtgaggcac ctatctcagc 8340
gatctgtcta tttcgttcat ccatagttgc ctgactcccc gtcgtgtaga taactacgat 8400
acgggagggc ttaccatctg gccccagtgc tgcaatgata ccgcgagacc cacgctcacc 8460
ggctccagat ttatcagcaa taaaccagcc agccggaagg gccgagcgca gaagtggtcc 8520
tgcaacttta tccgcctcca tccagtctat taattgttgc cgggaagcta gagtaagtag 8580
ttcgccagtt aatagtttgc gcaacgttgt tgccattgct acaggcatcg tggtgtcacg 8640
ctcgtcgttt ggtatggctt cattcagctc cggttcccaa cgatcaaggc gagttacatg 8700
atcccccatg ttgtgcaaaa aagcggttag ctccttcggt cctccgatcg ttgtcagaag 8760
taagttggcc gcagtgttat cactcatggt tatggcagca ctgcataatt ctcttactgt 8820
catgccatcc gtaagatgct tttctgtgac tggtgagtac tcaaccaagt cattctgaga 8880
atagtgtatg cggcgaccga gttgctcttg cccggcgtca atacgggata ataccgcgcc 8940
acatagcaga actttaaaag tgctcatcat tggaaaacgt tcttcggggc gaaaactctc 9000
aaggatctta ccgctgttga gatccagttc gatgtaaccc actcgtgcac ccaactgatc 9060
ttcagcatct tttactttca ccagcgtttc tgggtgagca aaaacaggaa ggcaaaatgc 9120
cgcaaaaaag ggaataaggg cgacacggaa atgttgaata ctcatactct tcctttttca 9180
atattattga agcatttatc agggttattg tctcatgagc ggatacatat ttgaatgtat 9240
ttagaaaaat aaacaaatag gggttccgcg cacatttccc cgaaaagtgc cacctgacgt 9300
ctaagaaacc attattatca tgacattaac ctataaaaat aggcgtatca cgaggccctt 9360
tcgtctcgcg cgtttcggtg atgacggtga aaacctctga cacatgcagc tcccggagac 9420
ggtcacagct tgtctgtaag cggatgccgg gagcagacaa gcccgtcagg gcgcgtcagc 9480
gggtgttggc gggtgtcggg gctggcttaa ctatgcggca tcagagcaga ttgtactgag 9540
agtgcaccat aaaattgtaa acgttaatat tttgttaaaa ttcgcgttaa atttttgtta 9600

aatcagctca ttttttaacc aataggccga aatcggcaaa atcccttata aatcaaaaga 9660
atagcccgag atagggttga gtgttgttcc agtttggaac aagagtccac tattaaagaa 9720
cgtggactcc aacgtcaaag ggcgaaaaac cgtctatcag ggcgatggcc cactacgtga 9780
accatcaccc aaatcaagtt ttttggggtc gaggtgccgt aaagcactaa atcggaaccc 9840
taaagggagc ccccgattta gagcttgacg gggaaagccg gcgaacgtgg cgagaaagga 9900
agggaagaaa gcgaaaggag cgggcgctag ggcgctggca agtgtagcgg tcacgctgcg 9960
cgtaaccacc acacccgccg cgcttaatgc gccgctacag ggcgcgtact atggttgctt 10020
tgacgtatgc ggtgtgaaat accgcacaga tgcgtaagga gaaaataccg catcaggcgc 10080
cattcgccat tcaggctgcg caactgttgg gaagggcgat cggtgcgggc ctcttcgcta 10140
ttacgccagc tggcgaaagg gggatgtgct gcaaggcgat taagttgggt aacgccaggg 10200
ttttcccagt cacgacgttg taaaacgacg gccagtgcc 10239

<210> 20
<211> 12
<212> DNA
<213> Trichoderma reesei

<400> 20
actagtaagc gg 12

<210> 21
<211> 41
<212> DNA
<213> Artificial Sequence

<220>
<223> primer

<400> 21
gcttatacta gtaagcgcgc gggcggcggc tattggcaca c 41

<210> 22
<211> 39
<212> DNA
<213> Artificial Sequence

<220>
<223> primer

<400> 22
gcttatggcg cgccttagac aggatcgaaa atcgacgac 39

<210> 23
<211> 43
<212> DNA
<213> Artificial Sequence

<220>
<223> primer

<400> 23
ctaagagaaa cgacccgtac atccagcggt tcctcacgat gta 43

<210> 24
<211> 38
<212> DNA
<213> Artificial Sequence

<220>
<223>  primer

<400>  24
ttacccggat gggaagagca tgccaaaatc ggcgttcg                              38

<210>  25
<211>  43
<212>  DNA
<213>  Artificial Sequence

<220>
<223>  primer

<400>  25
ctaagagagc gacgactcag ccgtacacct ggagcaacgt ggc                        43

<210>  26
<211>  38
<212>  DNA
<213>  Artificial Sequence

<220>
<223>  primer

<400>  26
ttacgatccg gacggcgcac caccaatgtc cccgtata                              38

<210>  27
<211>  42
<212>  DNA
<213>  Artificial Sequence

<220>
<223>  primer

<400>  27
gcttatacta gtaagcgcgc cggctgctcg gtggactaca cg                         42

<210>  28
<211>  37
<212>  DNA
<213>  Artificial Sequence

<220>
<223>  primer

<400>  28
gcttatggcg cgccttacag aggcgggtag gcgttgg                               37

<210>  29
<211>  42
<212>  DNA
<213>  Artificial Sequence

<220>

<223>  primer

<400>  29
gcttatacta gtaagcgcgc cggtctcacc gccacagtca cc                          42

<210>  30
<211>  36
<212>  DNA
<213>  Artificial Sequence

<220>
<223>  primer

<400>  30
gcttatggcg cgcctcagga ctggagcttg ctccgc                                 36n
21